# EUROPEAN PATENT APPLICATION

(11) **EP 4 029 516 A1**
(43) Date of publication of application: **20.07.2022**
(21) Application number: 21152072.1
(22) Date of filing: 18.01.2021
(51) Int. Cl.: A61K 38/17, G01N 33/50, A61P 31/14

(54) **MUCINS AND ISOFORMS THEREOF IN DISEASES CHARACTERIZED BY BARRIER DYSFUNCTION**

(71) Applicant: Universiteit Antwerpen, 2000 Antwerpen (BE)
(72) Inventor: The designation of the inventor has not yet been filed
(74) Representative: Arnold & Siedsma

(57) **Abstract**

The present invention relates to the field of mucins and isoforms thereof, more in particular for use in the diagnosis, monitoring, prevention and/or treatment of a disease characterized by barrier dysfunction, such as but not limited to a gastrointestinal disorder (e.g. Inflammatory Bowel Disease (IBD), Irritable Bowel Syndrome (IBS), cancer, gastro-intestinal infections, obesitas, non-alcoholic fatty liver disease (NAFLD)), neurodegenerative disorders, respiratory infections,... more in particular coronaviral infections In a specific embodiment, said mucins and/or isoforms thereof are selected from the list comprising: MUC16, MUC21, MUC2, MUC4, MUC5AC, MUC5B, MUC13, and MUC1 and isoforms thereof

## Description

### FIELD OF THE INVENTION

The present invention relates to the field of mucins and mRNA isoforms thereof, more in particular for use in the diagnosis, monitoring, prevention and/or treatment of a disease characterized by barrier dysfunction, such as but not limited to a gastrointestinal disorder (e.g. Inflammatory Bowel Disease (IBD), Irritable Bowel Syndrome (IBS), cancer, gastrointestinal infections, obesitas, non-alcoholic fatty liver disease (NAFLD)), neurodegenerative disorders, respiratory infections,... more in particular coronaviral infections. In a specific embodiment, said mucins and/or mRNA isoforms thereof are selected from the list comprising: MUC13, MUC16, MUC21, MUC2, MUC4, MUC5AC, MUC5B and MUC1 and mRNA isoforms thereof.

### BACKGROUND TO THE INVENTION

All epithelial tissues in the human body are covered by a mucus layer consisting of secreted and membrane-bound mucins that are a family of large molecular weight glycoproteins. Besides providing a protective function to the underlying epithelium by the formation of a physical barrier, transmembrane mucins also participate in the intracellular signal transduction. Mucins contain multiple exonic regions that encode for various functional domains. More specifically, they possess a large extracellular domain (ECD) consisting of variable number of tandem repeat (VNTR) regions rich in proline, threonine and serine (i.e. PTS domains) and heavily glycosylated. In addition, transmembrane mucins also contain extracellular epidermal growth factor (EGF)-like domains, a transmembrane region (TMD) and a shorter cytoplasmic tail (CT) that contains multiple phosphorylation sites. Binding of the ECD to the TMD is mediated by a sea urchin sperm protein, enterokinase and agrin (SEA) domain that is present in all transmembrane mucins except for MUC4. This SEA domain is autoproteolytically cleaved in the endoplasmic reticulum resulting in the noncovalent binding of the α-chain (ECD) and β-chain (TMD and CT).

Aberrant expression of transmembrane mucins has been observed during chronic inflammation and cancer. Of particular interest are MUC1 and MUC13. These transmembrane mucins are upregulated in the inflamed colonic mucosa from patients with inflammatory bowel disease (IBD) and in the tumor tissue of patients with gastric and colorectal cancer. Furthermore, emerging evidence suggests that their aberrant expression upon inflammation is associated with loss of mucosal epithelial barrier integrity.

Due to their polymorphic nature, the presence of genetic differences (i.e. single nucleotide polymorphisms (SNPs)) in mucin genes can result in different mRNA isoforms or splice variants due to alternative splicing. While most mRNA isoforms encode similar biological functions, others have the potential to alter the protein function resulting in progression toward disease. Although still poorly understood, differential expression of mucin mRNA isoforms could be involved in the pathophysiology of inflammatory diseases and cancer involving loss of barrier integrity.

### SUMMARY OF THE INVENTION

In a first aspect, the present invention provides a mucin or mucin mRNA isoform thereof for use in the diagnosis, monitoring, prevention and/or treatment of a disease characterized by barrier dysfunction, in particular a coronaviral infection or coronaviral infectious disease, wherein the mucin or mRNA isoform thereof is selected from the list comprising: MUC16, MUC21, MUC2, MUC4, MUC5AC, MUC5B, MUC13, MUC1, MUC16 mRNA isoforms, MUC 21 mRNA isoforms, MUC2 mRNA isoforms, MUC4 mRNA isoforms, MUC5AC mRNA isoforms, MUC5B mRNA isoforms, MUC13 mRNA isoforms, or MUC1 mRNA isoforms,

In a particular embodiment, the present invention provides an in vitro method for diagnosing and/or determining the severity of a coronaviral infection or coronaviral infectious disease and/or associated co-infections, said method comprising:
a) providing a biological sample from a subject suspected of having a coronaviral infection or coronaviral infectious disease and/or associated co-infections, and
b) determining the presence and/or quantity of one or more mucins selected from the list comprising: MUC16, MUC21, MUC2, MUC4, MUC5AC, MUC5B, MUC13, optionally in combination with MUC1;
wherein the presence and/or quantity of the one or more mucins is indicative for the presence and/or severity of a coronaviral infection or coronaviral infectious disease.

In a specific embodiment, said method comprises determining the presence and/or quantity of MUC13 and MUC21; and wherein high levels of MUC13 and MUC21 are indicative of a coronaviral infection or coronaviral infectious disease.

In another specific embodiment, said method comprises determining the presence and/or quantity of MUC1 and MUC16; and wherein high levels of MUC1 and low levels of MUC16 are indicative of a more severe coronaviral infection or coronaviral infectious disease.

In another particular embodiment, the present invention provides an in vitro method for diagnosing and/or determining the severity of a coronaviral infection or coronaviral infectious disease and/or associated co-infections, said method comprising:
a) providing a biological sample from a subject suspected of having a coronaviral infection or coronaviral infectious disease and/or associated co-infections, and
b) determining the presence and/or quantity of one or more of mucin isoforms; selected from the group comprising MUC16 mRNA isoforms, MUC21 mRNA isoforms, MUC2 mRNA isoforms, MUC4 mRNA isoforms, MUC5AC mRNA isoforms, MUC5B mRNA isoforms, MUC13 mRNA isoforms, or MUC1 mRNA isoforms ;
wherein the presence and/or quantity of the one or more mucin mRNA isoforms is indicative for the presence and/or severity of a coronaviral infection or coronaviral infectious disease.

In a specific embodiment, said one or more mucin mRNA isoforms are selected from the group comprising MUC1 mRNA isoforms, MUC13 mRNA isoforms, MUC16 mRNA isoforms, or MUC21 mRNA isoforms.

In yet a further embodiment at least 2, preferably at least 3 mucin mRNA isoforms are determined and/or quantified.

In a further embodiment, of the present invention, the presence and/or quantity of MUC1 mRNA isoforms, MUC13 mRNA isoforms, MUC16 mRNA isoforms and MUC21 mRNA isoforms are determined and wherein the presence and/or quantity of said mucin mRNA isoforms is indicative for the presence and/or severity of a coronaviral infection or coronaviral infectious disease.

In yet a further embodiment of the present invention, the presence and/or quantity of MUC13 mRNA isoforms and/or MUC21 mRNA isoforms is determined and wherein the presence and/or quantity of MUC13 mRNA isoforms and/or MUC21 mRNA isoforms is indicative for the diagnosis of a coronaviral infection or coronaviral infectious disease.

In another embodiment of the present invention, the presence and/or quantity of MUC1 mRNA isoforms and/or MUC16 mRNA isoforms is determined and wherein the presence and/or quantity of MUC1 mRNA isoforms and/or MUC16 mRNA isoforms is indicative for the severity of the coronaviral infection or coronaviral infectious disease.

The present invention further provides one or more mucin mRNA isoforms for use in the diagnosis and/or monitoring of a coronaviral infection or coronaviral infectious disease, wherein the one or more mucin mRNA isoforms are selected from the list comprising: MUC1 mRNA isoforms, MUC2 mRNA isoforms, MUC4 mRNA isoforms, MUC5AC mRNA isoforms, MUC5B mRNA isoforms, MUC13 isoforms, MUC16 mRNA isoforms, and MUC 21 mRNA isoforms.

In a particular embodiment, said mucin mRNA isoform is a transmembrane mucin.

In a specific embodiment, the one or more mucin mRNA isoforms for use in the present invention are selected from the list comprising: MUC1 mRNA isoforms, MUC13 isoforms, MUC16 mRNA isoforms, or MUC21 mRNA isoforms; optionally in combination with one or more of MUC2 mRNA isoforms, MUC4 mRNA isoforms, MUC5AC mRNA isoforms or MUC5B mRNA isoforms.

The present invention also provides a combination of MUC1 mRNA isoforms, MUC13 mRNA isoforms, MUC16 mRNA isoforms and MUC21 mRNA isoforms for use in the diagnosis and/or monitoring of a coronaviral infection or coronaviral infectious disease.

Furthermore, the present invention provides a MUC1 mRNA isoform and MUC16 mRNA isoform for use in determining the severity of a coronaviral infection or coronaviral infectious disease.

The present invention also provides a combination of MUC13 mRNA isoforms and MUC21 mRNA isoforms for use in the diagnosis of a coronaviral infection or coronaviral disease.

In another embodiment, the coronaviral infection or coronaviral disease is a SARS-CoV-2 infection or SARS-CoV-2 associated disease.
The invention also provides a diagnostic kit for performing the in vitro method according to the present invention, said kit comprising agents for detecting the presence of one or more mucins and/or mucin mRNA isoforms as defined herein.

In another particular embodiment, the present invention provides a mucin mRNA isoform as defined herein, for use as a biomarker for diagnosis and disease surveillance or monitoring.

In another particular embodiment, the present invention provides a mucin mRNA isoform as defined herein, for use as a new therapeutic target. In particular, said mucin mRNA isoform may be specifically targeted by monoclonal antibodies, small molecules or antisense technology.

In a specific embodiment of the present invention, said disease characterized by barrier dysfunction is a gastrointestinal disorder such as selected from the list comprising: Inflammatory Bowel Disease (IBD), Irritable Bowel Syndrome (IBS), cancer, gastro-intestinal infections, obesitas, non-alcoholic fatty liver disease (NAFLD); a neurodegenerative disorder; or a respiratory infection.

In another particular embodiment of the present invention, said cancer may be selected from the list comprising: esophageal cancer, gastric cancer, colorectal cancer, pancreas cancer, liver cancer, kidney cancer, lung cancer, ovarian cancer, colon cancer and prostate cancer.

In a further embodiment of the present invention, said gastro-intestinal infection may be selected from the list comprising: *Helicobacter* infection, *Campylobacter* infection, *Clostridioides difficile* infection and *Salmonella* infection.

In yet a further embodiment of the present invention, said neurodegenerative disorder may be selected from the list comprising: Parkinson's disease, Alzheimer's disease, Multiple Sclerosis (MS) and Autism.

In another embodiment of the present invention, said Inflammatory Bowel Disease may be selected from the list comprising: Crohn's disease and ulcerative colitis.

In yet a further embodiment, said respiratory infection may be selected from the list comprising: respiratory syncytial viral infections, influenza viral infections, rhinoviral infections, metapneumoviral infections, Pseudomonas aeruginosa viral infections and coronaviral infections. Said coronaviral infection for example being a SARS-CoV-2 infection.

### BRIEF DESCRIPTION OF THE DRAWINGS

With specific reference now to the figures, it is stressed that the particulars shown are by way of example and for purposes of illustrative discussion of the different embodiments of the present invention only. They are presented in the cause of providing what is believed to be the most useful and readily description of the principles and conceptual aspects of the invention. In this regard no attempt is made to show structural details of the invention in more detail than is necessary for a fundamental understanding of the invention. The description taken with the drawings making apparent to those skilled in the art how the several forms of the invention may be embodied in practice.

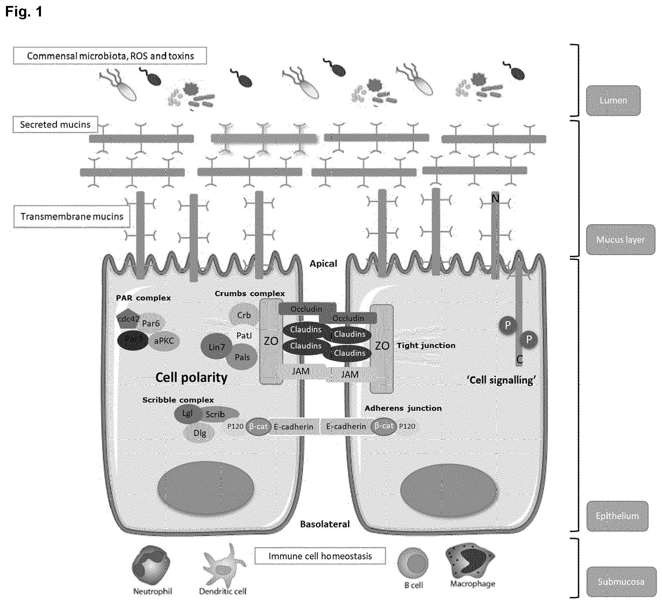

**Figure 1****. Schematic representation of the intestinal mucosal barrier.** The intestinal barrier comprises a thick layer of mucus, a single layer of epithelial cells and the inner lamina propria hosting innate and adaptive immune cells. Secreted and transmembrane mucins (MUCs) represent the major components of the mucus barrier. Besides having a protective function, transmembrane mucins also participate in intracellular signal transduction. The epithelium underneath plays an active role in innate immunity via secretion and expression of mucins and antimicrobial peptides as well as by hosting antigen presenting cells. Intestinal epithelial cells are tightly linked to each other by intercellular junctions: i.e. tight junctions (claudins (CLDNs), occludin (OCLN) and junctional adhesion molecules (JAMs)) and adherens junctions (E-cadherin and β-catenin). The PAR, Crumbs and Scribble polarity complexes regulate the polarized expression of membrane proteins in the epithelial cells.

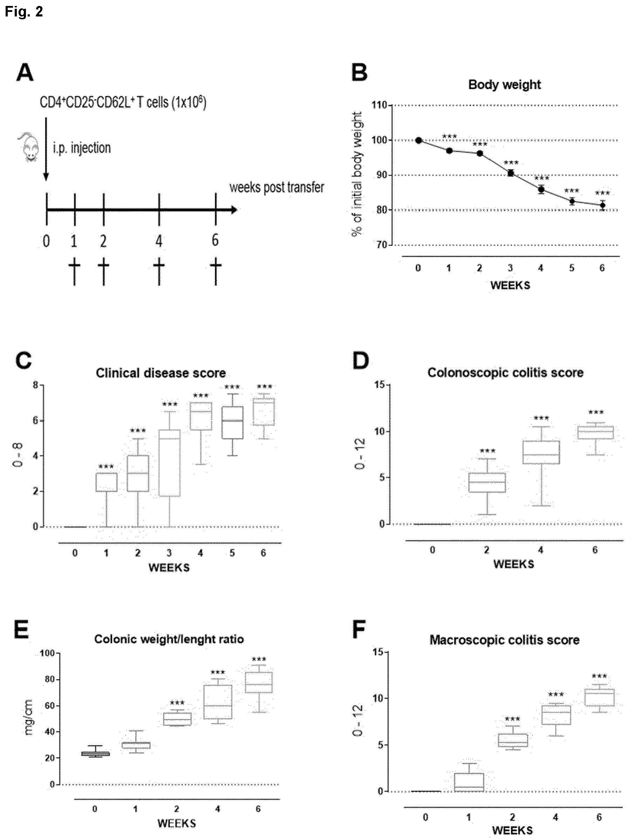

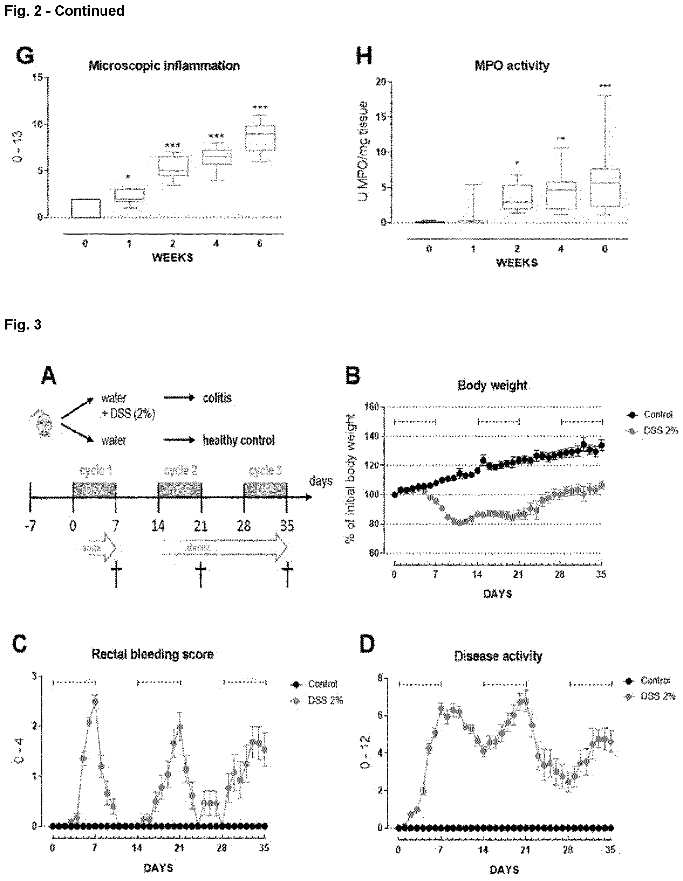

**Figure 2****. Analysis of intestinal inflammation in the adoptive T cell transfer model. (A)** Schematic overview and timeline of the adoptive T cell transfer model. **(B)** Relative changes in body weight after T cell transfer. **(C)** Weekly determination of the clinical disease score by the assessment of body weight loss, pilo-erection, mobility and stool consistency. **(D)** Colitis severity was scored every two weeks by endoscopy and was based on the morphology of the vascular pattern, bowel wall translucency, fibrin attachment and the presence of loose stools. **(E)** The colon weight/length ratio. **(F)** At sacrifice, the colon was longitudinally opened and visually inspected for the presence of ulcerations, hyperemia, bowel wall thickening and oedema. **(G)** H&E stained colon sections were evaluated blinded focusing on crypt destruction, epithelial erosion, goblet cell loss and immune cell infiltration. **(H)** Neutrophil infiltration in the colon was assessed by measuring MPO activity. Significant differences between control and colitis mice are indicated by *p<0.05, **<0.01, ***p<0.001 (One-Way ANOVA, Tukey's multiple comparison post-hoc test).

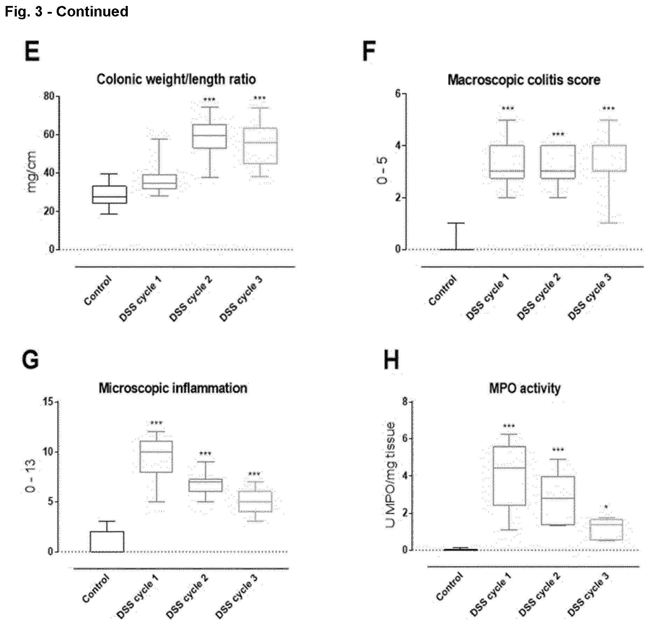

**Figure 3****. Analysis of intestinal inflammation in the DSS-induced colitis model. (A)** Schematic overview and timeline of the DSS-induced colitis model. **(B)** Body weight was daily assessed and shown as percentage of the initial body weight. **(C)** Daily determination of the disease activity index (DAI), which is the cumulative score of body weight loss, the extent of rectal bleeding and changes in stool consistency. The horizontal bars indicate periods of DSS administration. **(D)** Rectal bleeding score. **(E)** The colon weight/length ratio. **(F)** At sacrifice, the colon was longitudinally opened and inspected for the presence of ulcerations, hyperemia, bowel wall thickening and oedema. **(G)** Microscopic colonic inflammation score which was based on crypt loss, epithelial erosion, goblet cell loss, immune cell infiltration and colonic hyperplasia. **(H)** Colonic MPO activity to assess neutrophil infiltration in the colon. N = 8-14 mice/group (control, DSS cycle 1, DSS cycle 2, DSS cycle 3). Significant differences between control and colitis mice are indicated by *p<0.05, **<0.01, ***p<0.001 (One-Way ANOVA, Tukey's multiple comparison post-hoc test).

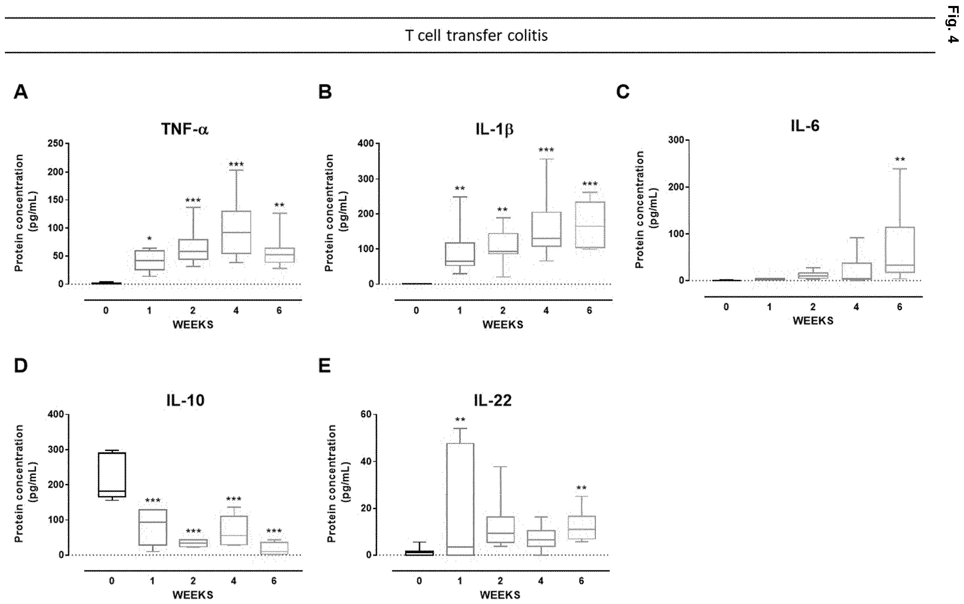

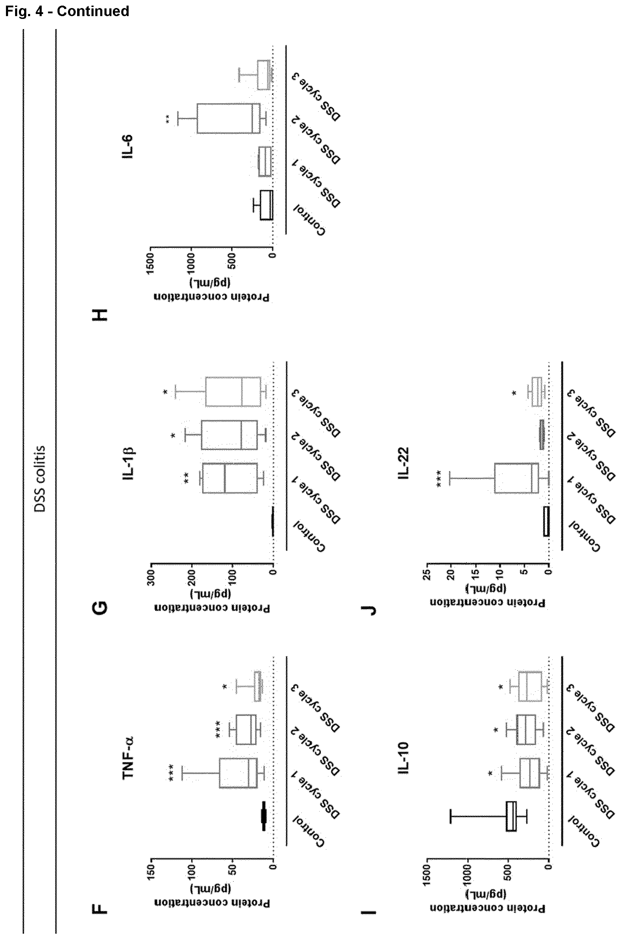

**Figure 4****. Colonic cytokine expression in the T cell transfer and DSS-induced colitis models.** Protein expression of pro- and anti-inflammatory cytokines in the colon of controls and T cell transfer- or DSS-induced colitis mice. Results are shown for TNF-α **(A&F),** IL-1β **(B&G),** IL-6 **(C&H),** IL-10 **(D&I)** and IL-22 **(E&J).** Significant differences between control and colitis mice are indicated by *p<0.05; **p<0.01; ***p<0.001 (N = 5-10 mice/group (week 0 (control), 1, 2, 4 & 6) for the T cell transfer model; N = 6-13 mice/group (control, DSS cycle 1, DSS cycle 2, DSS cycle 3) for the DSS model; One-Way ANOVA or Kruskal-Wallis, Tukey's and Dunn's multiple comparison post-hoc test).

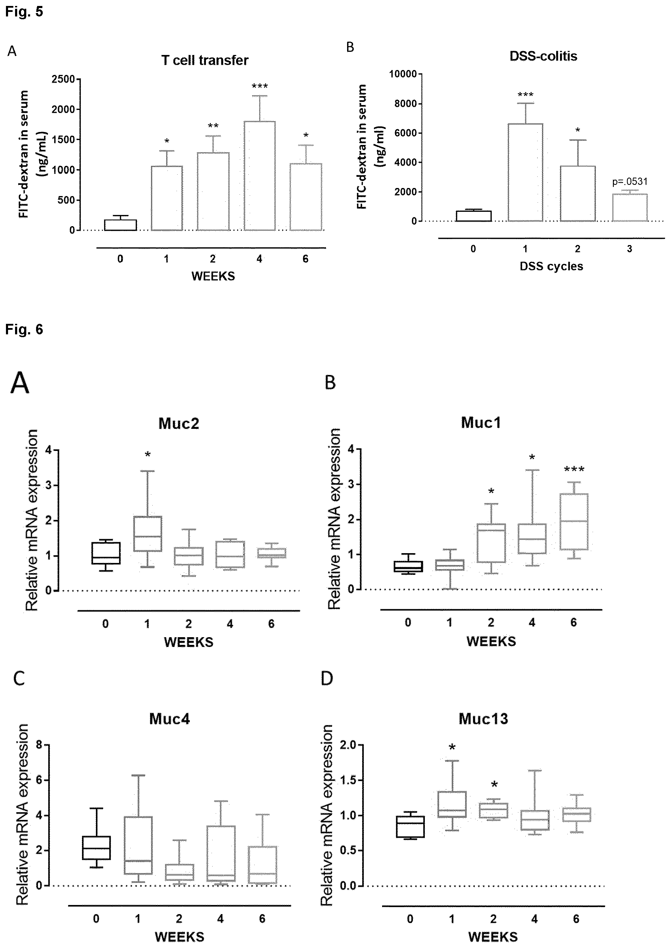

**Figure 5****. Analysis of intestinal permeability in the T-cell transfer and DSS-induced colitis models.** Relative gastrointestinal permeability of control mice compared to colitis animals: **(A)** T cell transfer model (N = 7-10 mice/group (week 0 (control), 1, 2, 4 & 6)); **(B)** DSS model (N = 8-13 mice/group (control, DSS cycle 1, DSS cycle 2, DSS cycle 3)). Significant differences between control and colitis mice are indicated by *p<0.05; **p<0.01; ***p<0.001 (Kruskal-Wallis test, Dunn's post-hoc multiple comparison test).
**Figure 6****. Colonic mucin expression in the adoptive T cell transfer model. (A-D)** mRNA expression of *Muc1, Muc2, Muc4* and *Muc13* (N = 7-10 mice/group (week 0 (control), 1, 2, 4 & 6)) in the colon of controls and T cell transfer-induced colitis mice. Significant differences between control and colitis mice are indicated by *p<0.05; **p<0.01; ***p<0.001 (One-Way ANOVA, Tukey's post-hoc multiple comparison test).

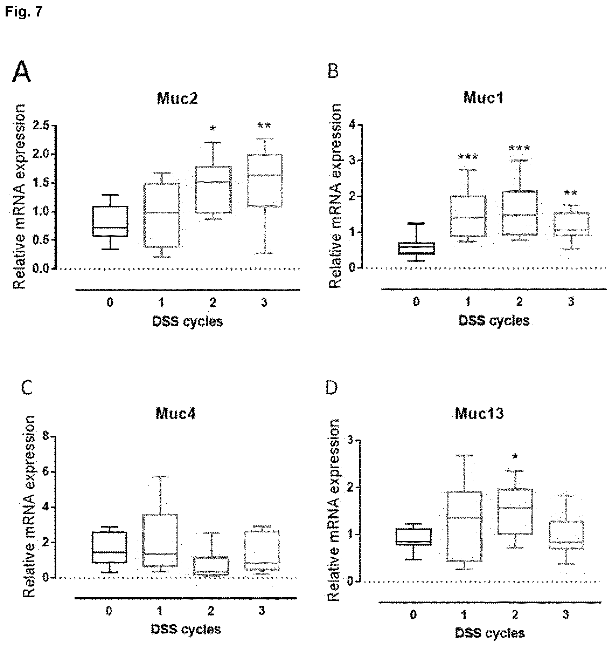

**Figure 7****. Colonic mucin expression in the DSS-induced colitis model. (A-D)** mRNA expression of *Muc1, Muc2, Muc4* and *Muc13* (N = 10-13 mice/group (control, DSS cycle 1, DSS cycle 2, DSS cycle 3)) in the colon of controls and DSS-induced colitis mice. Significant differences between control and colitis mice are indicated by *p<0.05; **p<0.01; ***p<0.001 (One-Way ANOVA, Tukey's post-hoc multiple comparison test).

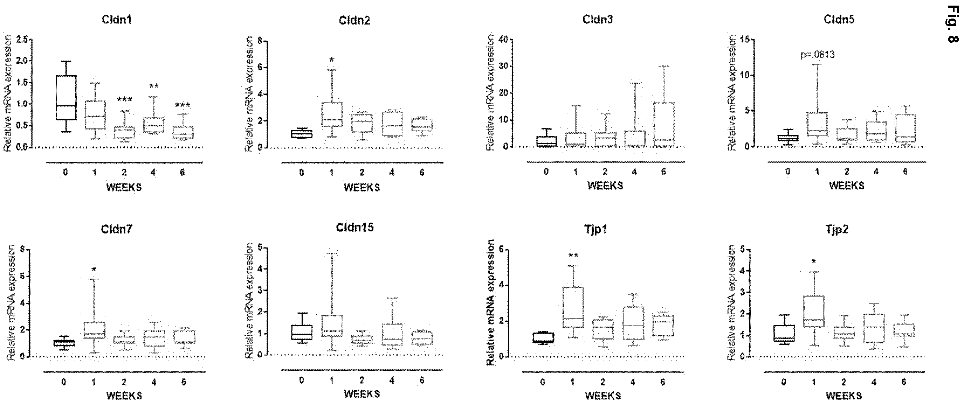

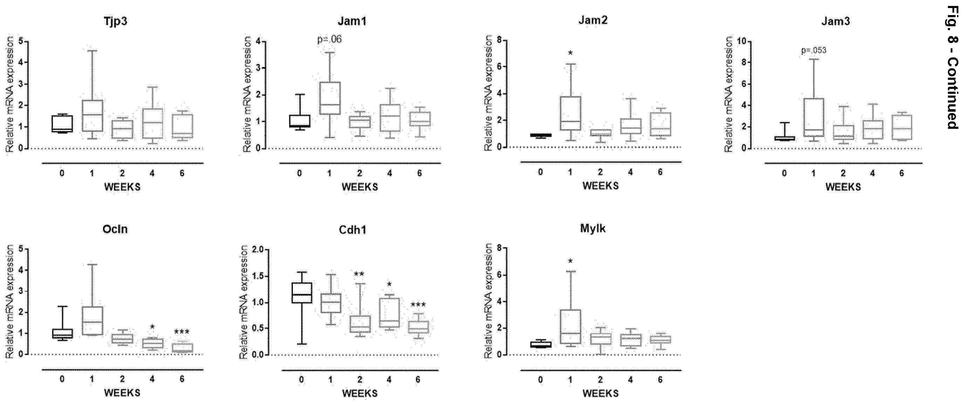

**Figure 8****. Colonic intercellular junction expression in the adoptive T cell transfer model.** mRNA expression of several Claudins (*Cldn*), Zonula-Occludens (*ZolTjp*), Junctional Adhesion Molecules (*Jam*)*,* Occludin (*Ocln*), E-cadherin (*Cdh1*) and Myosin light chain kinase (*Mylk*) in the colon of controls and T cell transfer-induced colitis mice. Significant differences between healthy control and colitis mice is indicated by *p<0.05; **p<0.01; ***p<0.001 (N = 10-13 mice/group (week 0 (control), 1, 2, 4 & 6); One-Way ANOVA or Kruskal-Wallis, Tukey's and Dunn's multiple comparison post-hoc test).

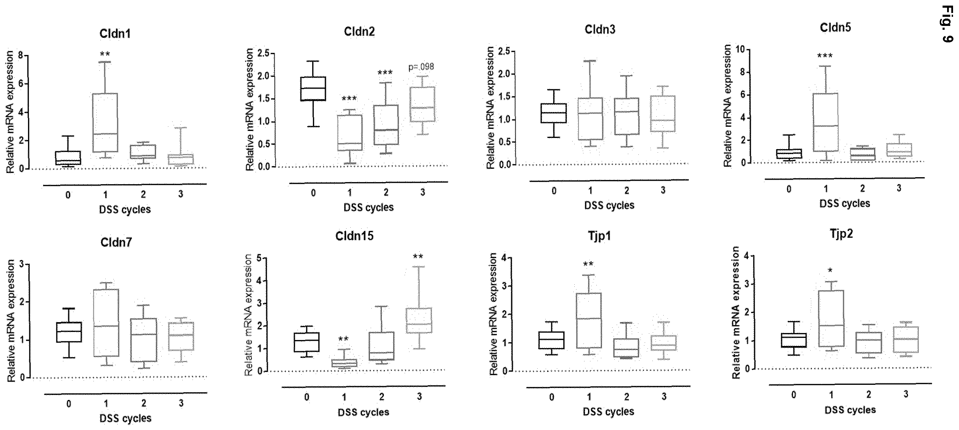

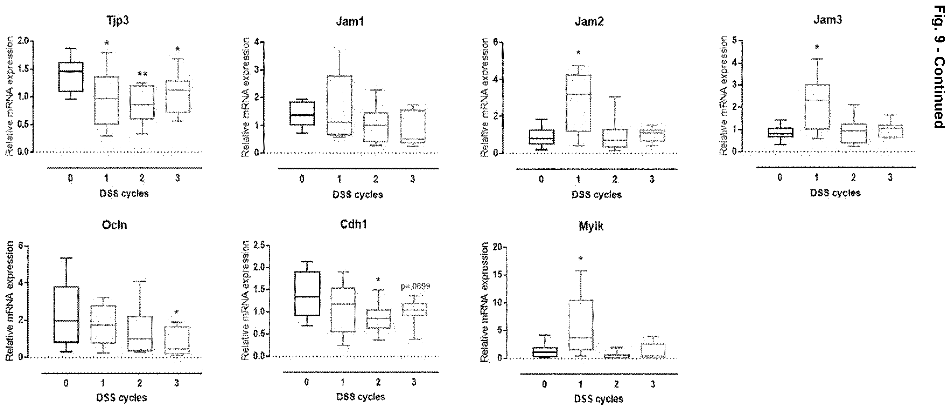

**Figure 9****. Colonic intercellular junction expression in the DSS model.** mRNA expression of several Claudins (*Cldn*), Zonula-Occludens (*ZolTjp*), Junctional Adhesion Molecules (*Jam*), Occludin (*Ocln*), E-cadherin (*Cdh1*) and Myosin light chain kinase (*Mylk*) in the colon of controls and DSS-induced colitis mice. Significant differences between control and colitis mice are indicated by *p<0.05; **p<0.01; ***p<0.001 (N = 10-13 mice/group (control, DSS cycle 1, DSS cycle 2, DSS cycle 3); One-Way ANOVA or Kruskal-Wallis, Tukey's and Dunn's multiple comparison post-hoc test).

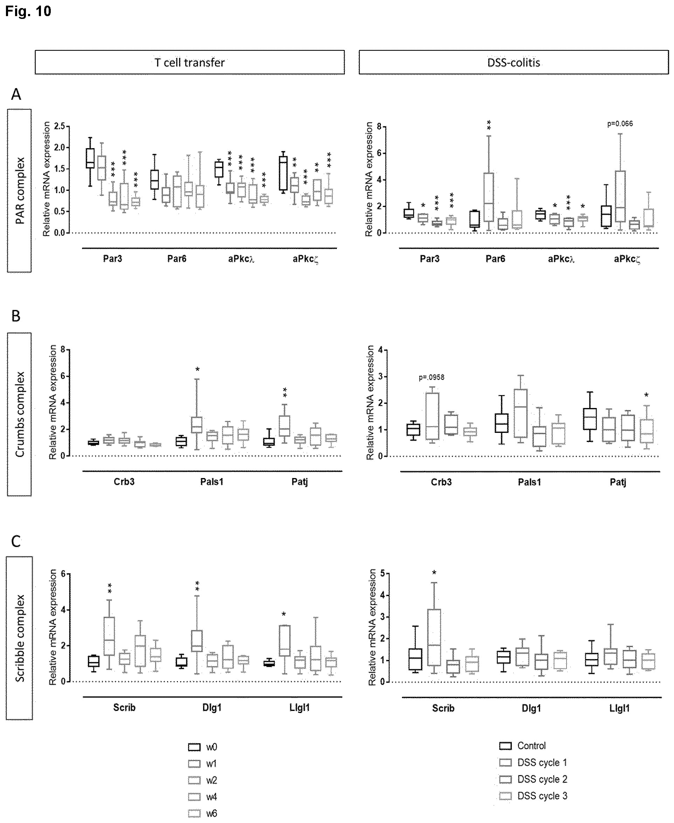

**Figure 10****. Colonic expression of cell polarity proteins during the course of colitis.** mRNA expression of **(A)** *Par3, Par6, aPkcA* and *aPke* 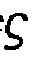 (PAR complex) **(B)** *Crb3, Pals1* and *Patj* (Crumbs complex) and **(C)** *Scrib, Dlg1* and *Llgl1* (Scribble complex) in the T cell transfer (N = 7-10 mice/group (week 0 (control), 1, 2, 4 & 6)) and DSS-induced colitis model (N = 10-13 mice/group (control, DSS cycle 1, DSS cycle 2, DSS cycle 3)). Significant differences between control and colitis mice are indicated by *p<0.05; **p<0.01; ***p<0.001 (One-Way ANOVA, Tukeys post-hoc multiple comparison test).

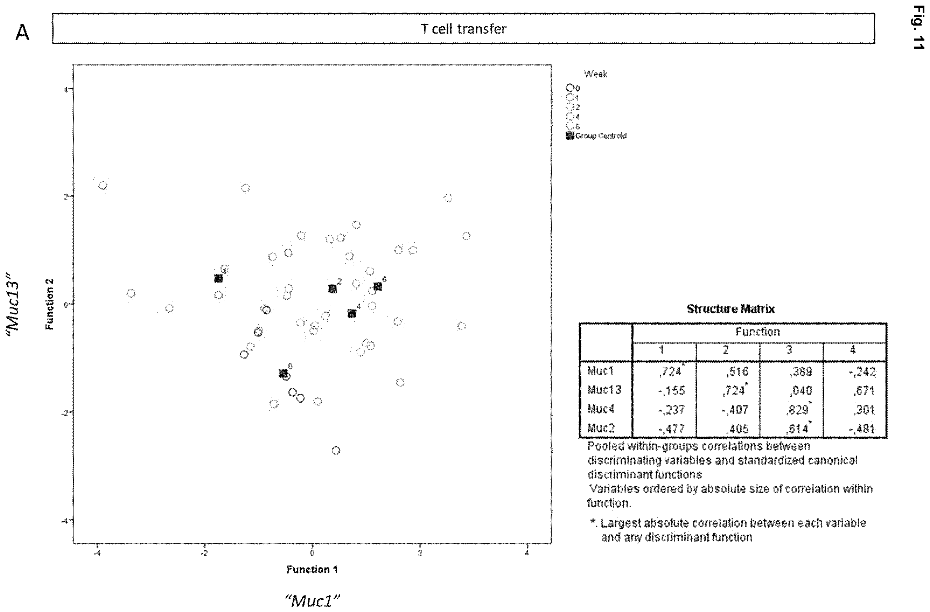

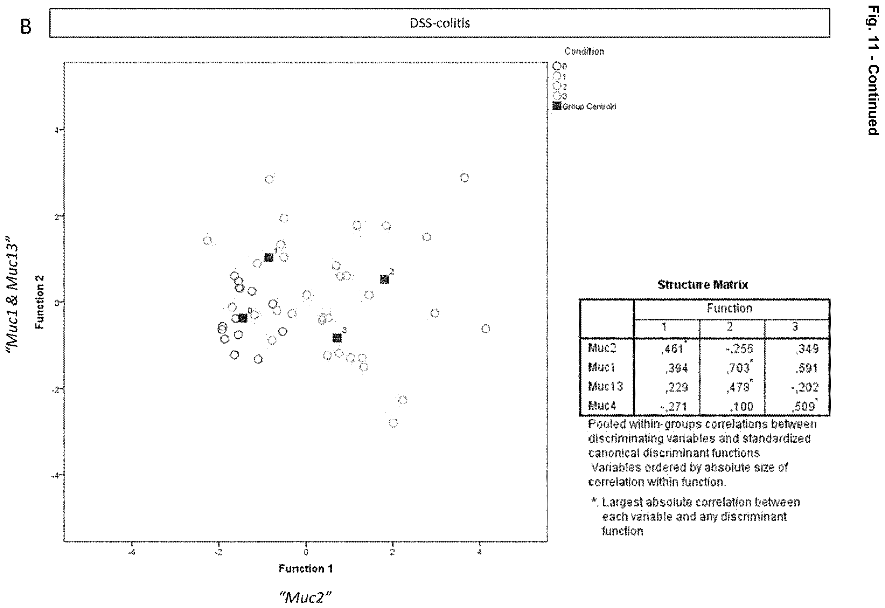

**Figure 11****. Discriminant analysis with mRNA expression values of *Muc1, Muc2, Muc4* and *Muc13* as predictors.** Discriminant analysis for the T cell transfer and DSS models to predict healthy controls and colitis groups (week 0, 1, 2, 4, 6; DSS cycle 1, DSS cycle 2, DSS cycle 3). The main predictor variables for each function are stated in the structure matrix. (A) For the T cell transfer model, the different experimental groups were mainly discriminated by *Muc1* (function 1) and *Muc13* (function 2). Individual mice were correctly annotated to their respective groups in 57.8% of the cases. **(B)** For the DSS-colitis model, the different experimental groups were primary discriminated by *Muc2* (function 1) followed by *Muc1* and *Muc13* (function 2). Individual mice were correctly annotated to their respective groups in 69.6% of the cases.

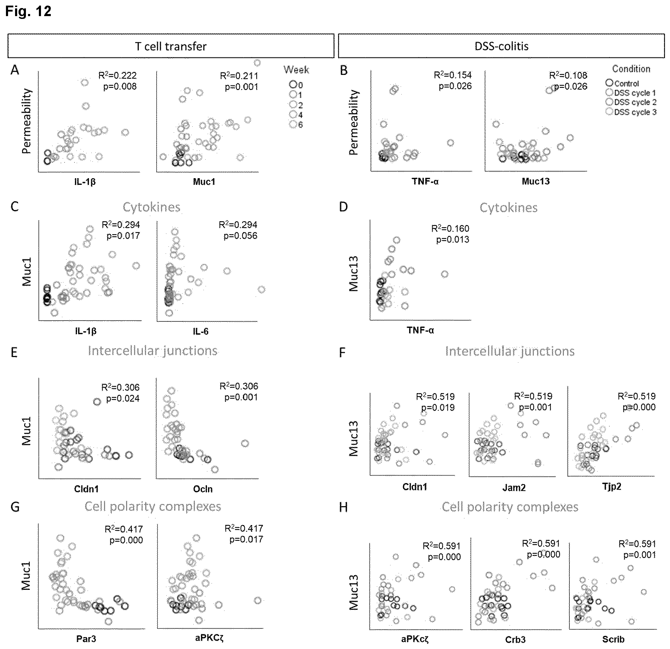

**Figure 12****. Scatter plots of correlated data for the T cell transfer model and the DSS colitis model.**
   **T cell transfer model: (A)** Correlation of intestinal permeability with IL-1β protein and *Muc1* mRNA expression levels. **(C)** Correlation of *Muc1* expression with IL-1β and IL-6 protein expression. **(E)** Correlation of *Muc1* mRNA expression with the expression levels of the intercellular junctions *Cldn1* and *Ocln.* **(G)** Correlation of *Muc1* mRNA expression with the expression levels of the cell polarity complex subunits *Par3* and *aPKCζ.* **DSS colitis model: (B)** Correlation of intestinal permeability with TNF-α protein and *Muc13* mRNA expression levels. **(D)** Correlation of *Muc13* mRNA expression with TNF-α protein expression. **(F)** Correlation of *Muc13* mRNA expression with the expression levels of the intercellular junctions *Cldn1, Jam2* and *Tjp2.* **(H)** Correlation of *Muc13* mRNA expression with the expression levels of the cell polarity complex subunits *aPKCζ, Crb3* and *Scrib.* The correlations were selected based on the results of a multiple linear regression analysis. The corresponding adjusted R²-values and p-values of the regression model are shown.

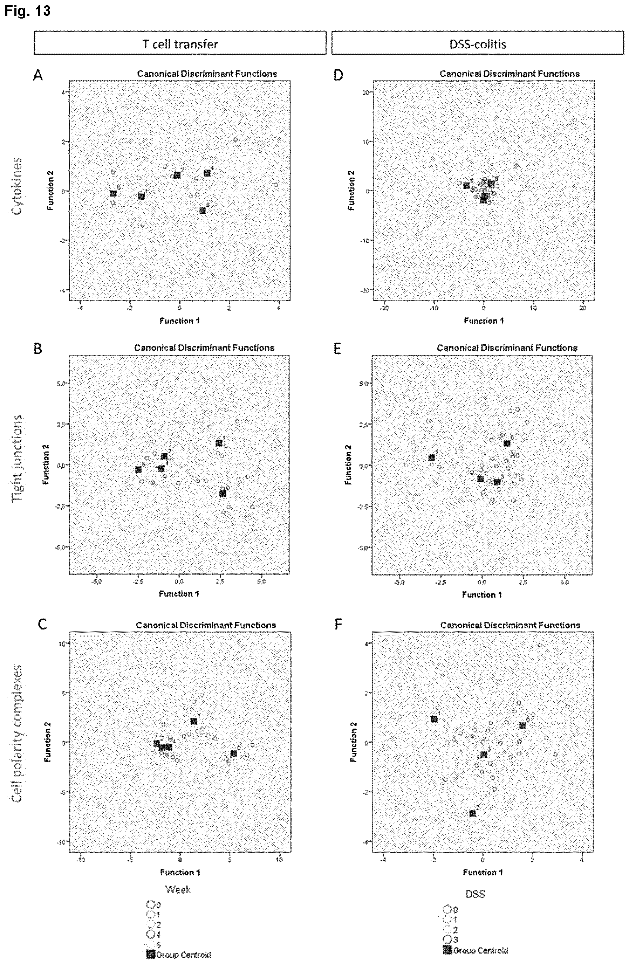

**Figure 13****. Discriminant analysis with the expression levels of cytokines, tight junctions and polarity complexes as predictors.** A discriminant analysis was performed to predict healthy controls and colitis groups (weeks after T cell transfer/cycles of DSS administration) based on the expression of cytokines (protein), tight junctions (mRNA) and cell polarity proteins (mRNA) in the T cell transfer (A-C) and DSS (D-F) colitis model. The main predictor variables for each function are stated in the legend (Pooled within-groups correlations not shown). Overall, mice sacrificed 1 week after T cell transfer and after DSS cycle 1 could be clearly discriminated from control mice and the other experimental groups. **(A)** 72,4% of cases were correctly classified based on cytokine expression and was mainly determined by the expression of IL-1β (function 1), TNF-α and IL-6 (function 2). **(B)** 72.1% of cases were correctly classified based on tight junction expression and was mainly determined by the expression of *Ocln* (function 1) and *Cldn2, Cldn1, Tjp2, Jam2* and *Jam2* (function 2). **(C)** 84,1% of cases were correctly classified based on the expression of cell polarity proteins and was mainly determined by the expression of *Par3* (function 1) and *Dlg, Patj, Scrib, Llgl1* and *Pals1* (function 2). **(D)** 37.3% of cases were correctly classified based on cytokine expression and was mainly determined by the expression of IL-1β and IL-10 (function 1) and TNF-α (function 2). In this analysis, missing values were converted to mean values, potentially explaining the bad prediction. **(E)** 76.5% of cases were correctly classified based on tight junction expression and was mainly determined by the expression of *Jam2, Cldn2, Jam3, Cldn15, Cldn5, Tjp1* and *Cldn1* (function 1) and *Tjp3, Ocln and Jam1* (function 2). **(F)** 64.7% of cases were correctly classified based on the expression of cell polarity subunits and was mainly determined by the expression of *Par3* (function 1).

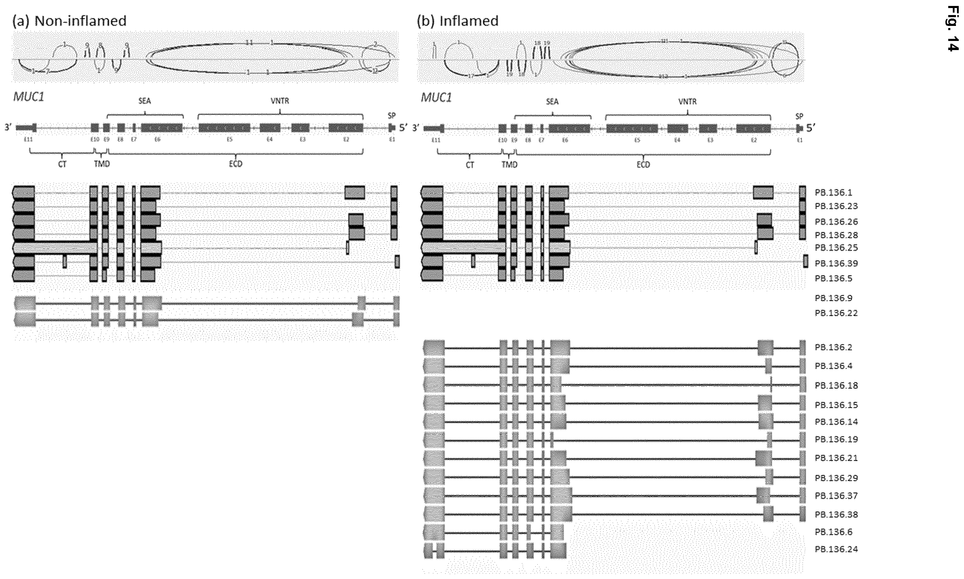

**Figure 14****: Alternative mRNA transcripts of MUC1 in (a) non-inflamed and (b) inflamed colonic tissue from IBD patients.** The upper panel indicates a Sashimi plot to summarize the splice junctions in the alternative mRNA transcripts. The gene structure highlighted in blue illustrates the overall exonic structure of MUC1 with the corresponding exon numbers and coding domains (CT = cytoplasmic tail; TMD = transmembrane domain; ECD = extracellular domain; EGF = epidermal growth factor; SEA = sea urchin sperm protein, enterokinase and agrin; VNTR = variable number tandem repeat; SP = signal peptide). The coloured transcripts are found in both non-inflamed and inflamed intestinal tissue. The gray mRNA transcripts highlight transcripts that are found in only one condition (i.e. inflamed or non-inflamed). On the right panel, the isoform identity number can be found of which the details are shown in table 5 (n = 3 paired samples).

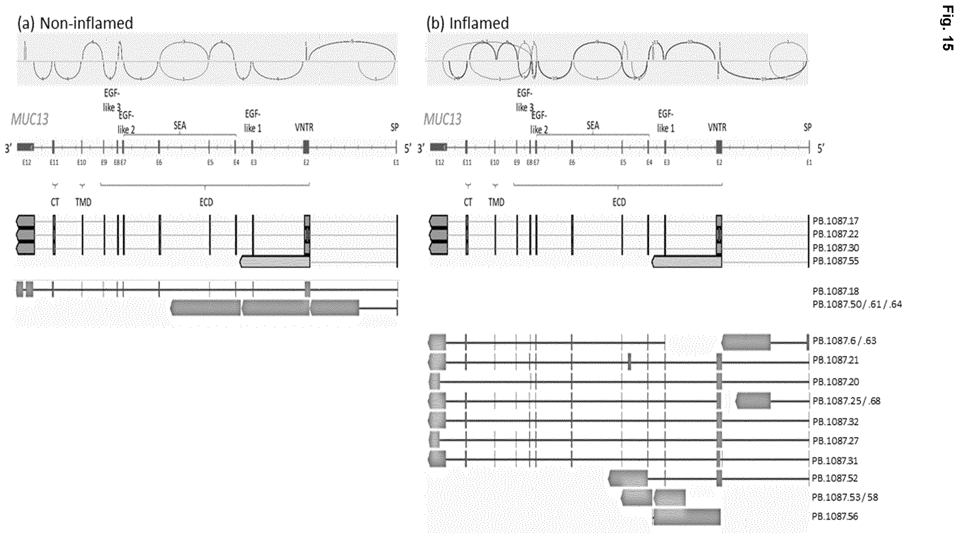

**Figure 15****: Alternative mRNA transcripts of MUC13 in (a) non-inflamed and (b) inflamed colonic tissue from IBD patients.** The upper panel indicates a Sashimi plot to summarize the splice junctions in the alternative mRNA transcripts. The gene structure highlighted in blue illustrates the overall exonic structure of MUC13 with the corresponding exon numbers and coding domains (CT = cytoplasmic tail; TMD = transmembrane domain; ECD = extracellular domain; EGF = epidermal growth factor; SEA = sea urchin sperm protein, enterokinase and agrin; VNTR = variable number tandem repeat; SP = signal peptide). The coloured transcripts are found in both non-inflamed and inflamed intestinal tissue. The gray mRNA transcripts highlight transcripts that are found in only one condition (i.e. inflamed or non-inflamed). On the right panel, the isoform identity number can be found of which the details are shown in table 5 (n = 3 paired samples).

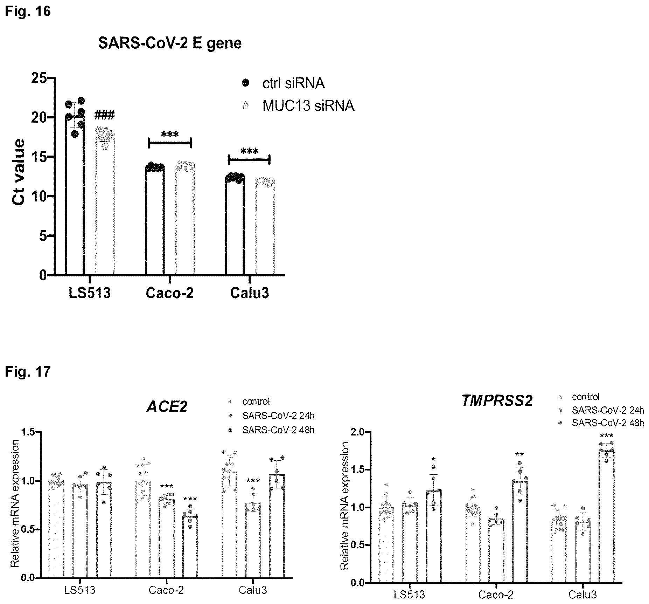

**Figure 16****:** RT-qPCR results to detect the SARS-CoV-2 E in the supernatants of ctrl and MUC13 siRNA transfected intestinal (LS513 and Caco2) and pulmonary (Calu3) epithelial cells infected with SARS-CoV-2 at 0.1 MOI for 48h. Cycle threshold values are shown. Significant differences between ctrl and MUC13 siRNA transfected cells within a cell line are indicated by ### p<0.001 and between different transfected cell lines are indicated by ***p<0.001. (One-Way ANOVA, Tukey's post-hoc multiple comparison test, N = 6). Error bars indicate SEM.
**Figure 17****:** Relative mRNA expression of ACE2 and TMPRSS2 in intestinal (LS513 and Caco2) and pulmonary (Calu3) epithelial cells infected with SARS-CoV-2 at 0.1 MOI for 24h and 48h. Cells treated with the growth medium of the virus were included as controls. Significant differences between SARS-CoV-2-infected and uninfected cells are indicated by *p<0.05; **p<0.01; ***p<0.001 (One-Way ANOVA, Tukey's post-hoc multiple comparison test, N = 6). Error bars indicate SEM.

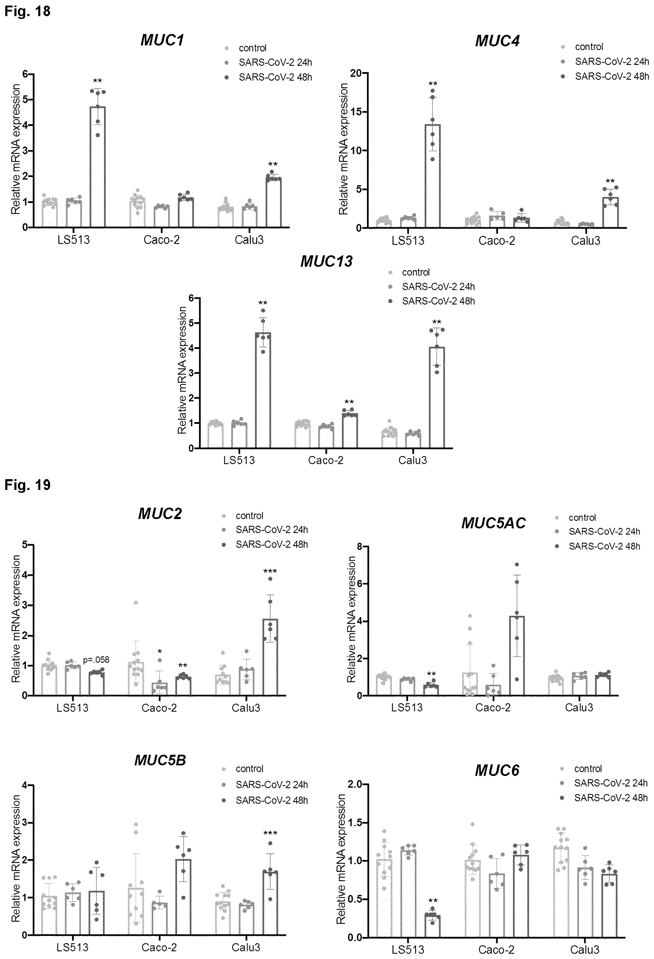

**Figure 18****:** Relative mRNA expression of the transmembrane mucins (MUC1, MUC4 and MUC13) in intestinal (LS513 and Caco2) and pulmonary (Calu3) epithelial cells infected with SARS-CoV-2 at 0.1 MOI for 24h and 48h. Cells treated with the growth medium of the virus were included as controls. Significant differences between SARS-CoV-2-infected and uninfected cells are indicated by *p<0.05; **p<0.01; ***p<0.001 (One-Way ANOVA, Tukey's post-hoc multiple comparison test, N = 6). Error bars indicate SEM.
**Figure 19****:** Relative mRNA expression of the secreted mucins (MUC2, MUC5AC, MUC5B and MUC6) in intestinal (LS513 and Caco2) and pulmonary (Calu3) epithelial cells infected with SARS-CoV-2 at 0.1 MOI for 24h and 48h. Cells treated with the growth medium of the virus were included as controls. Significant differences between SARS-CoV-2-infected and uninfected cells are indicated by *p<0.05; **p<0.01; ***p<0.001 (One-Way ANOVA, Tukey's post-hoc multiple comparison test, N = 6). Error bars indicate SEM.

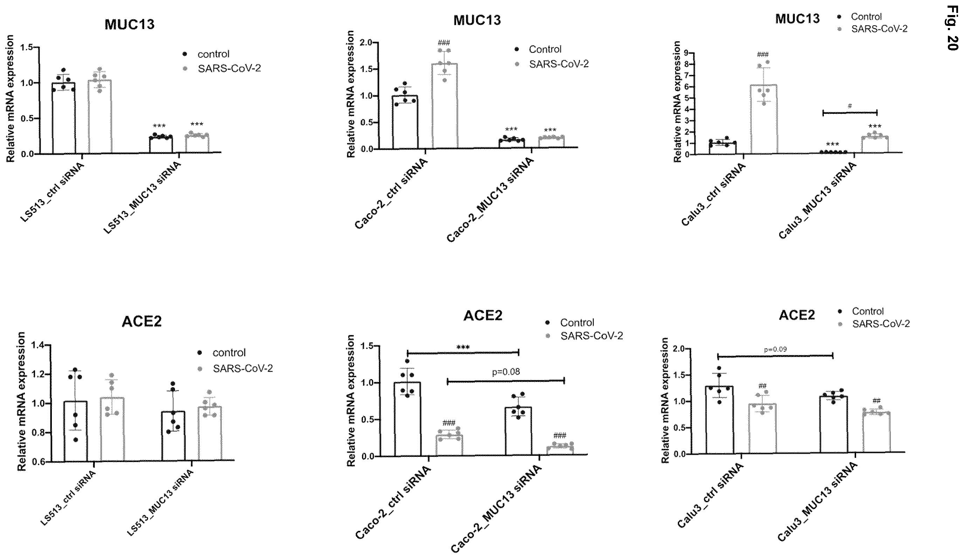

**Figure 20****:** Relative mRNA expression of MUC13 and ACE2 in ctrl siRNA and MUC13 siRNA transfected intestinal (LS513 and Caco-2) and pulmonary (Calu3) epithelial cells infected with SARS-CoV-2 at 0.1 MOI for 48h. Transfected cells treated with the growth medium of the virus were included as controls. Significant differences between SARS-CoV-2-infected and uninfected transfected cells are indicated by # p<0.05; ##p<0.01; ###p<0.001. Significant differences between ctrl siRNA and MUC13 siRNA transfected cells infected or uninfected with SARS-CoV-2 are indicated by ***p<0.001. One-Way ANOVA, Tukey's post-hoc multiple comparison test, N = 6. Error bars indicate SEM.

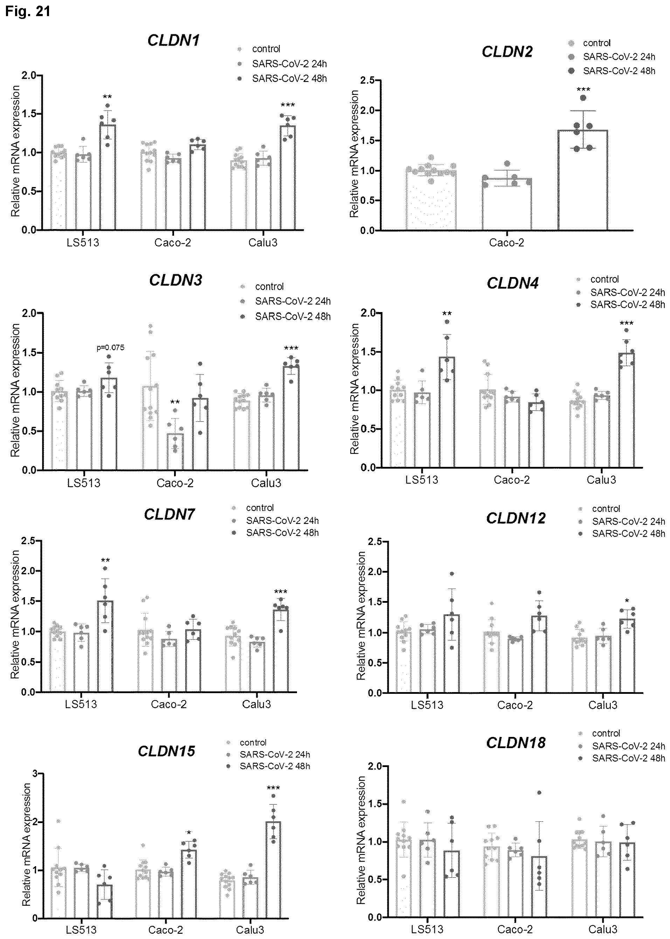

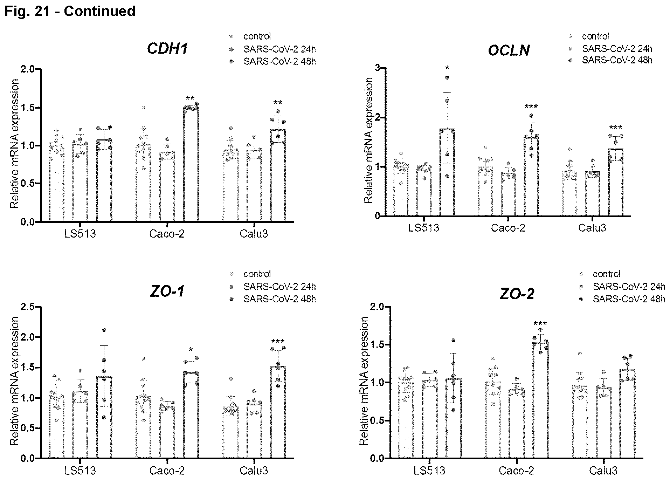

**Figure 21****:** Relative mRNA expression of junctional proteins (CLDN1, CLDN2, CLDN3, CLDN4, CLDN7, CLDN12, CLDN15, CLDN18, OCLN, ZO-1 and ZO-2 and CHD1 (E-cadherin)) in intestinal (LS513 and Caco2) and pulmonary (Calu3) epithelial cells infected with SARS-CoV-2 at 0.1 MOI for 24h and 48h. Cells treated with the growth medium of the virus were included as controls. Significant differences between SARS-CoV-2-infected and uninfected cells are indicated by *p<0.05; **p<0.01; ***p<0.001 (One-Way ANOVA, Tukey's post-hoc multiple comparison test, N = 6). Error bars indicate SEM.

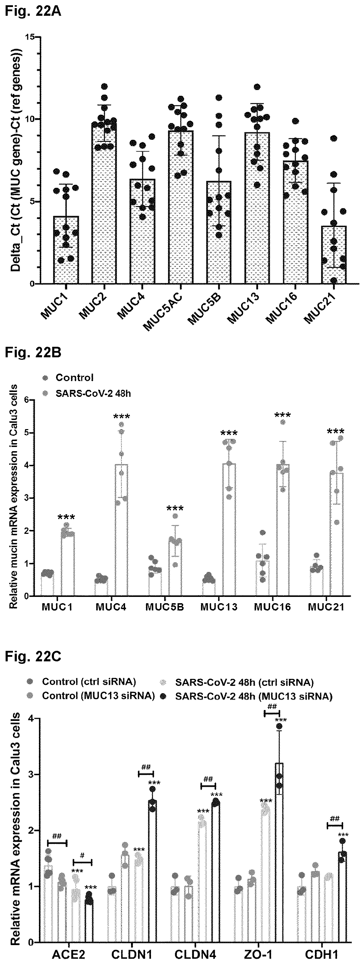

**Figure 22****: A,** Box plots of mucin mRNA expression (expressed as delta_Ct values) in mucus (endotracheal tubes) from mechanically ventilated ICU COVID-19 patients (N=10). The lower the delta_Ct value, the higher the mucin expression levels. Particularly MUC2, MUC4, MU5B, MUC5AC, MUC13, MUC16, MUC21 and MUC1 could be detected in mucus secretions from COVID-19 patients. **B,** Relative mRNA expression of MUC1, MUC4, MUC5B, MUC13, MUC16 and MUC21 in Calu3 cells infected with SARS-CoV-2 at 0.1 MOI for 48h. **C,** Relative mRNA expression of ACE2 and the junctional proteins: claudin-1 (CLDN1), CLDN4, zonula occludens 1 (ZO-1) and E-cadherin (CDH1) in ctrl siRNA and MUC13 siRNA transfected Calu3 cells infected with SARS-CoV-2 at 0.1 MOI for 48h. Significant differences between SARS-CoV-2-infected and uninfected (transfected) cells are indicated by **p<0.01; ***p<0.001 and between ctrl siRNA and MUC13 siRNA transfected cells infected or uninfected with SARS-CoV-2 are indicated by ^{#}p<0.05; ^{##}p<0.01. (ANOVA, N = 6). Error bars indicate SEM.

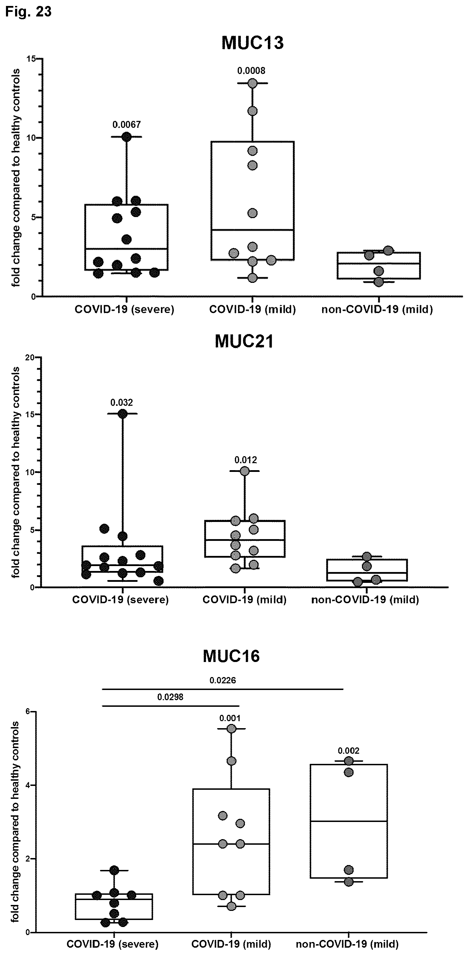

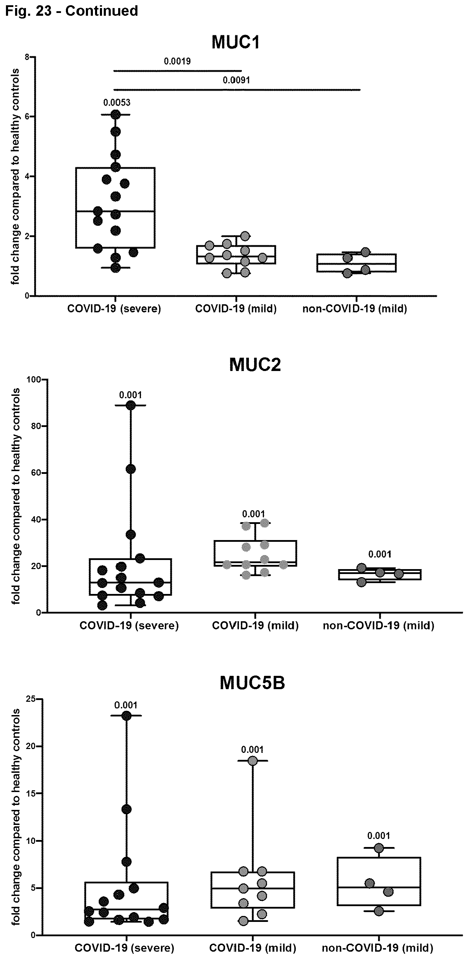

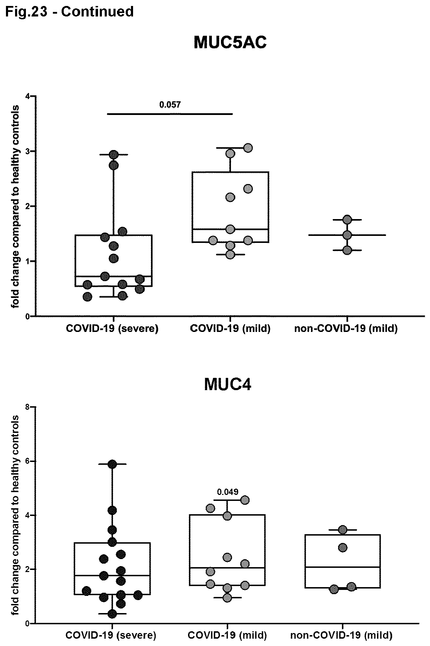

**Figure 23****:** Fold change mRNA expression levels of the mucins MUC13, MUC2, MUC4, MUC5AC, MUC5B, MUC1, MUC16 and MUC21 in blood from severely ill COVID-19 patients (N=15, blue bullets), ambulatory COVID-19 (N=10, orange bullets) and non-COVID-19 (N=4, green bullets) patients compared to healthy controls (N=4). Significant differences between severely ill COVID-19 patients, ambulatory COVID-19 or non-COVID-19 patients and healthy controls as well as among the different patients groups (i.e. COVID-19 (severe), COVID-19 (mild) and non-COVID-19 (mild)) are indicated by p-value (One-Way ANOVA, Tukey's post-hoc multiple comparison test). Error bars indicate SEM.

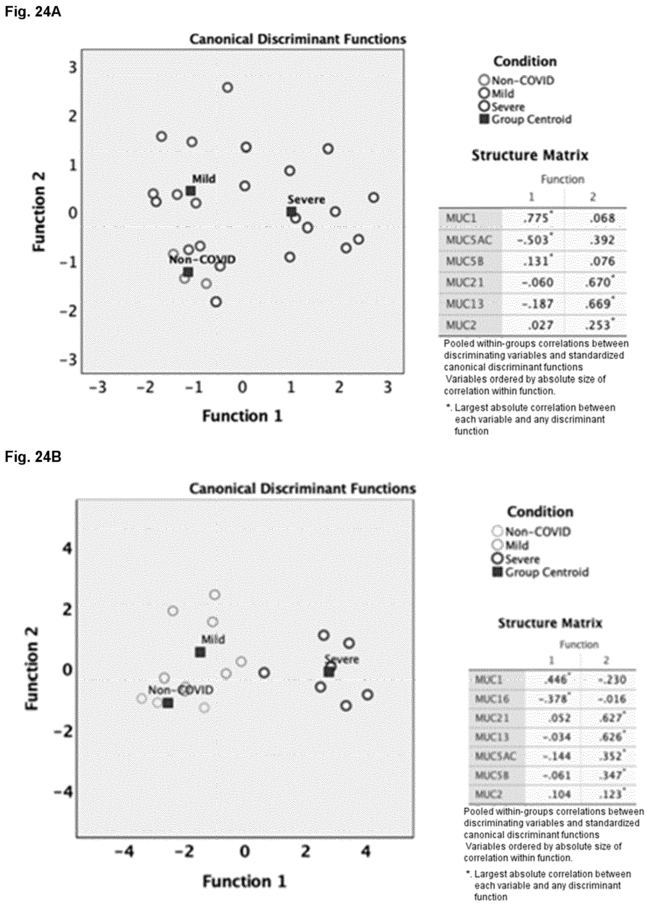

**Figure 24****: A,** Discriminant analysis with mRNA expression values of *MUC1, MUC2,* MUC5AC, *MUC5B, MUC13* and *MUC21* as predictors for COVID-19 positivity and severity. The main predictor variables for each function (MUC1 = function 1; MUC13 and MUC21 = function 2) are stated in the structure matrix. Individual patients were correctly annotated to their respective groups (i.e. mild non-COVID-19, mild COVID-19 and severe COVID-19) in 84% of the cases. **B,** Discriminant analysis with mRNA expression values of *MUC1, MUC2,* MUC5AC, *MUC5B, MUC13, MUC16* and *MUC21* as predictors for COVID-19 diagnosis and severity. The main predictor variables for each function (MUC1 and MUC16 = function 1; MUC13 and MUC21 = function 2) are stated in the structure matrix. Individual patients were correctly annotated to their respective groups (i.e. mild non-COVID-19, mild COVID-19 and severe COVID-19) in 94.4% of the cases.

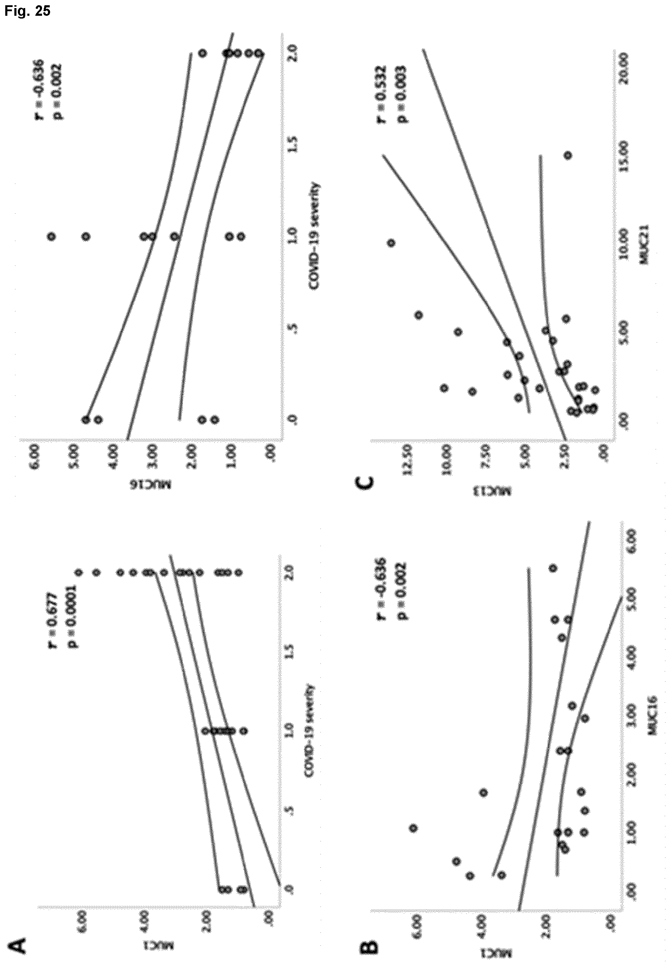

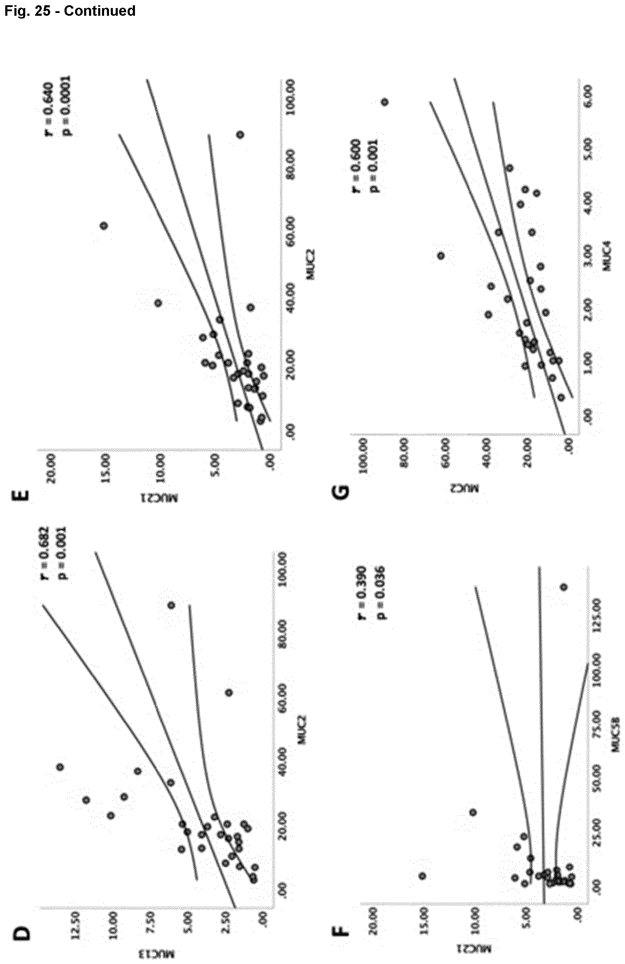

**Figure 25****: Scatter plots of the correlation data on mucin expression among the different patient groups (severe COVID-19, mild COVID-19, mild non-COVID-19). A,** correlation of *MUC1* and *MUC16* mRNA expression levels with COVID-19 severity. **B,** Correlation between *MUC1* and *MUC16* mRNA expression. **C,** Correlation between *MUC13* and *MUC21* mRNA expression. **D,** Correlation between *MUC13* and *MUC2* mRNA expression. **E,** Correlation between *MUC21* and *MUC2* mRNA expression. **F,** Correlation between *MUC21* and *MUC5B* mRNA expression. **G,** Correlation between *MUC2* and *MUC4* mRNA expression. Results from Spearman correlation tests are displayed as well as a linear regression line with 95% confidence interval.

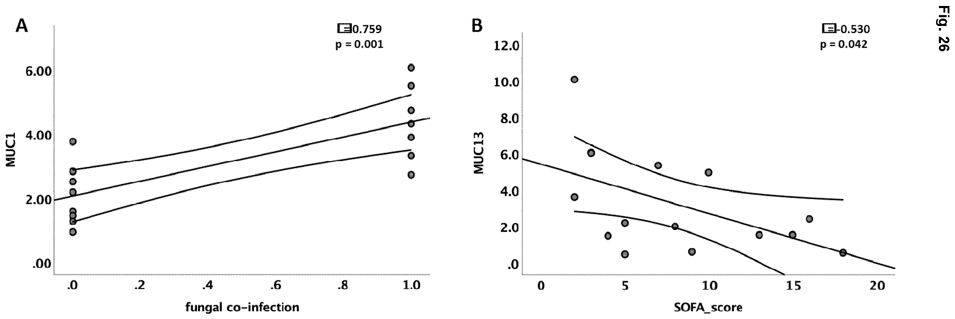

**Figure 26****: Scatter plots of correlation data between mucin expression and clinical patient data. A,** Correlation between MUC1 expression levels and fungal co-infection. **B,** Correlation of MUC13 expression levels with the SOFA score. Results from Spearman correlation tests are displayed as well as a linear regression line with 95% confidence interval.

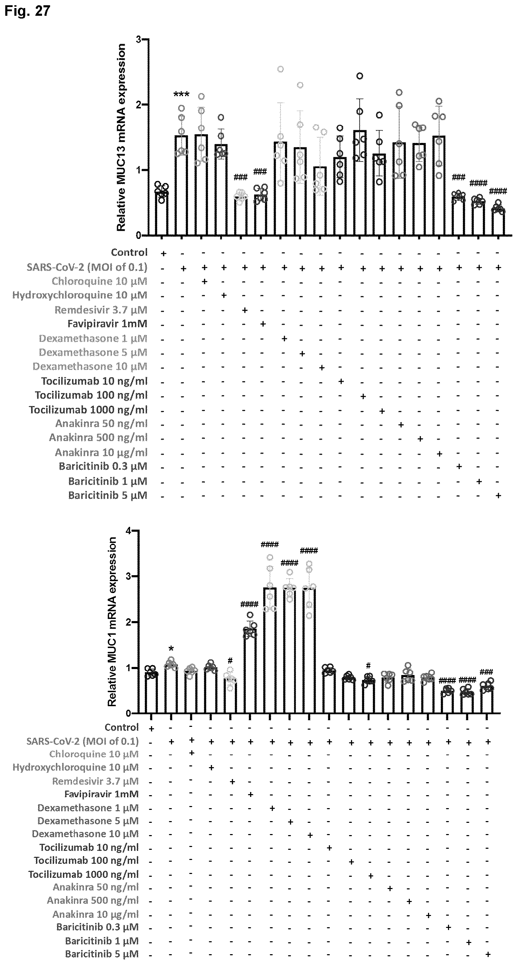

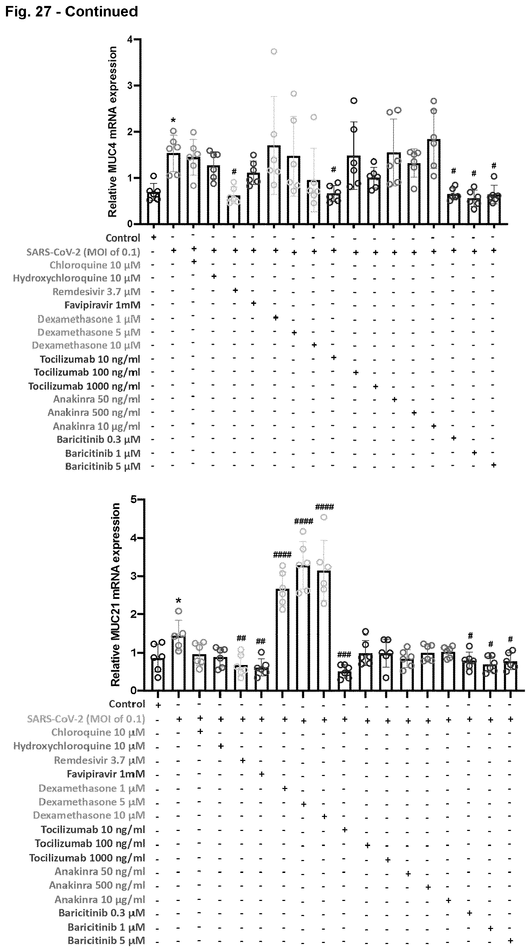

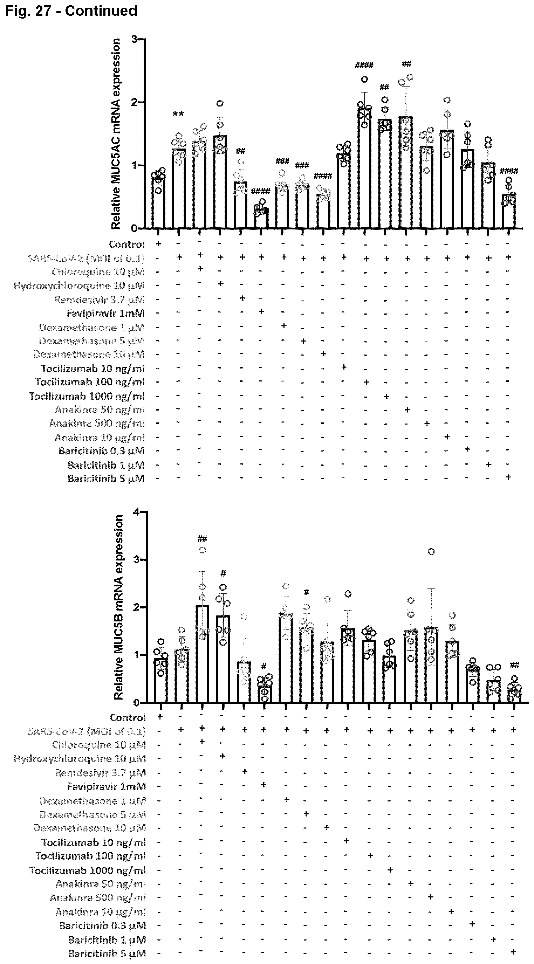

**Figure 27****:** Relative mRNA expression of MUC1, MUC4, MUC5B, MUC13, MUC16 and MUC21 in Calu3 cells infected with SARS-CoV-2 at 0.1 MOI for 2h and thereafter treated with a COVID-19 drug at different concentrations for 48h. These include: Remdesivir (antiviral; 3.7 µM); favipiravir (antiviral; 1mM), (Hydroxy)chloroquine (10µM); Dexamethasone (corticosteroid able to reduce mucin expression; 1-5-10 µM); Tocilizumab (anti-IL6; 10-100-1000 ng/ml); Anakinra (anti-IL1; 50-500 ng/ml, 10 µg/ml); and Baricitinib (JAK1/2 inhibitor; 0.3-1-5 µM). Significant differences between SARS-CoV-2-infected and uninfected cells are indicated by *p<0.05; **p<0.01; ***p<0.001. Significant differences between treated and untreated cells upon SARS-CoV-2 infection are indicated by #p<0.05; ##p<0.01; ###p<0.001. (One-Way ANOVA, Tukey's post-hoc multiple comparison test, N = 6). Error bars indicate SEM.

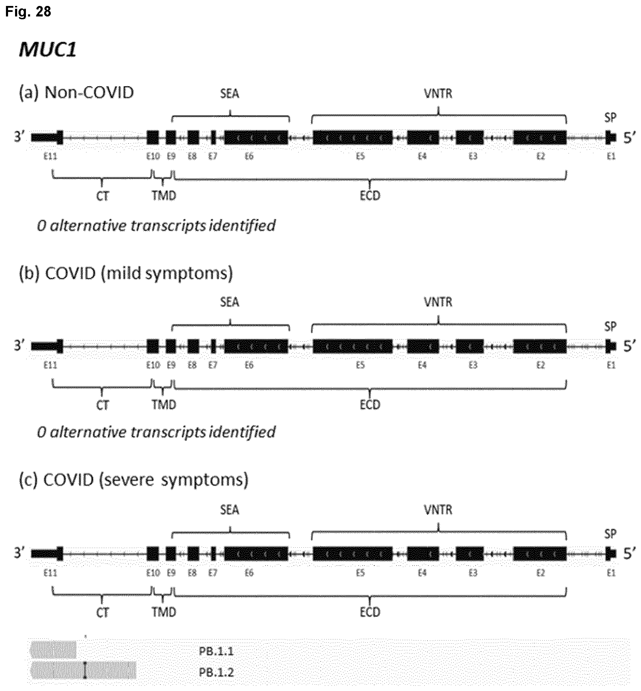

**Figure 28****. Alternative mRNA transcripts of MUC1 in the blood of (a) non-COVID19 and COVID19 patients with (b) mild and (c) severe symptoms.** The gene structure highlighted in blue illustrates the overall exonic structure of MUC1 with the corresponding exon numbers and coding domains (CT = cytoplasmic tail; TMD = transmembrane domain; ECD = extracellular domain; EGF = epidermal growth factor; SEA = sea urchin sperm protein, enterokinase and agrin; VNTR = variable number tandem repeat; SP = signal peptide). The gray mRNA transcripts highlight the alternative mRNA isoforms that are found in the blood of a particular condition (i.e. non-COVID19 (n = 2) or COVID19 with mild (n = 5) or severe (n = 5) symptoms). On the right panel, the isoform identity number can be found of which the details are shown in table 11.

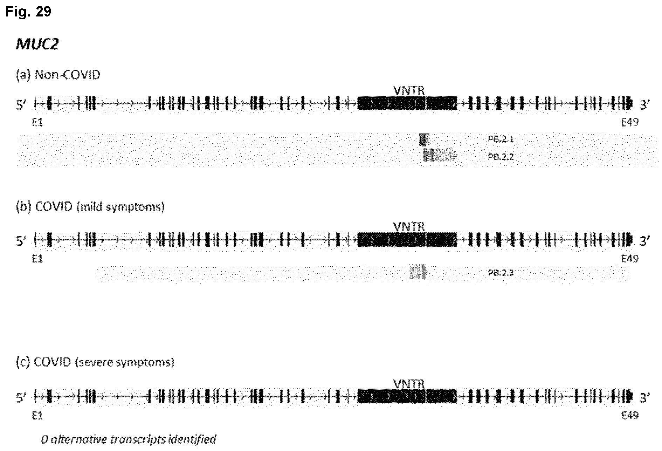

**Figure 29****. Alternative mRNA transcripts of *MUC2* in the blood of (a) non-COVID19 and COVID19 patients with (b) mild and (c) severe symptoms.** The gene structure highlighted in blue illustrates the overall exonic structure of MUC2 with the corresponding exon numbers and coding domains (VNTR = variable number tandem repeat). The gray mRNA transcripts highlight the alternative mRNA isoforms that are found in the blood of a particular condition (i.e. non-COVID19 (n = 2) or COVID19 with mild (n = 5) or severe (n = 5) symptoms). On the right panel, the isoform identity number can be found of which the details are shown in table 11.

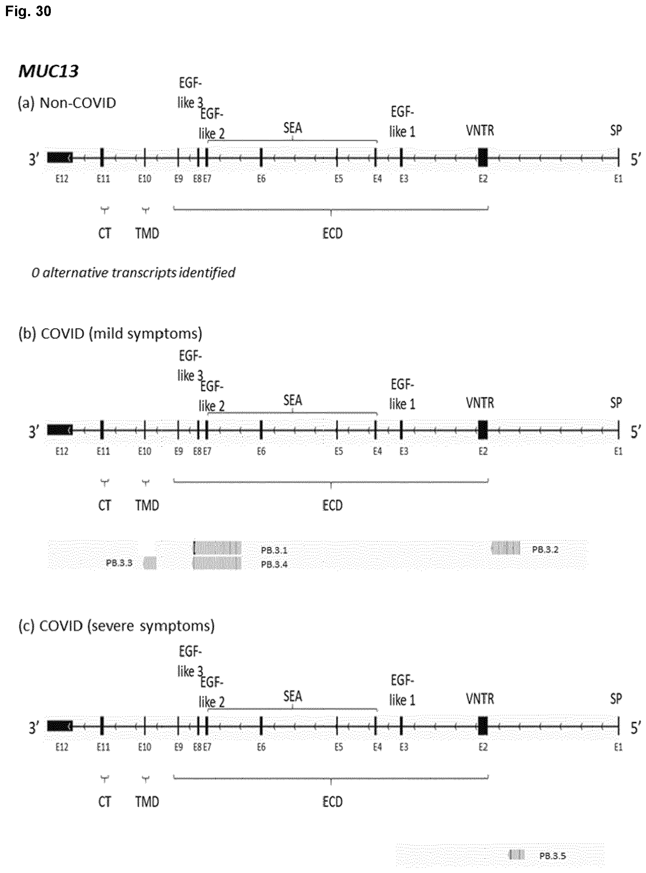

**Figure 30****. Alternative mRNA transcripts of *MUC13* in the blood of (a) non-COVID19 and COVID19 patients with (b) mild and (c) severe symptoms.** The gene structure highlighted in blue illustrates the overall exonic structure of MUC13 with the corresponding exon numbers and coding domains (VNTR = variable number tandem repeat). The gray mRNA transcripts highlight the alternative mRNA isoforms that are found in the blood of a particular condition (i.e. non-COVID19 (n = 2) or COVID19 with mild (n = 5) or severe (n = 5) symptoms). The isoform identity number can be found next to the mRNA isoform of which the details are shown in table 11.

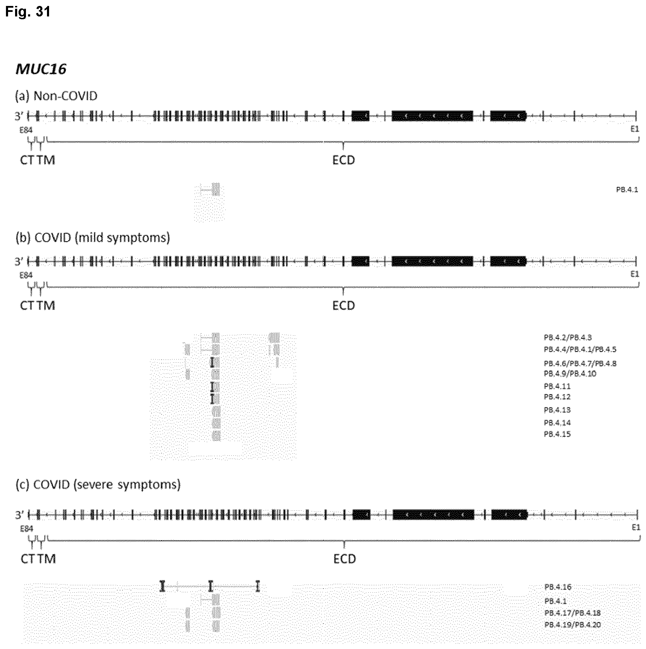

**Figure 31****. Alternative mRNA transcripts of MUC16 in the blood of (a) non-COVID19 and COVID19 patients with (b) mild and (c) severe symptoms.** The gene structure highlighted in blue illustrates the overall exonic structure of MUC16 with the corresponding exon numbers and coding domains (VNTR = variable number tandem repeat). The gray mRNA transcripts highlight the alternative mRNA isoforms that are found in the blood of a particular condition (i.e. non-COVID (n = 2) or COVID with mild (n = 5) or severe (n = 5) symptoms). The isoform identity number can be found next to the mRNA isoform of which the details are shown in table 11.

### DETAILED DESCRIPTION OF THE INVENTION

As already detailed herein above, in a first aspect, the present invention provides a mucin or mucin mRNA isoform for use in the diagnosis, monitoring, prevention and/or treatment of a disease characterized by barrier dysfunction, wherein the mucin or mucin mRNA isoform is selected from the list comprising: MUC1, MUC13, MUC1 isoforms and MUC13 isoforms; or alternatively from the list comprising MUC16, MUC21, MUC2, MUC4, MUC5AC, MUC5B, MUC13, MUC1; MUC16 mRNA isoforms, MUC 21 mRNA isoforms, MUC2 mRNA isoforms, MUC4 mRNA isoforms, MUC5AC mRNA isoforms, MUC5B mRNA isoforms, MUC13 mRNA isoforms, or MUC1 mRNA isoforms.

In a particular embodiment, the present invention provides a mucin or mucin mRNA isoform for use in the diagnosis, monitoring, prevention and/or treatment of coronaviral infection or coronaviral infectious disease, wherein the mucin or mucin mRNA isoform is selected from the list comprising: MUC16, MUC21, MUC2, MUC4, MUC5AC, MUC5B, MUC13, MUC1; MUC16 mRNA isoforms, MUC 21 mRNA isoforms, MUC2 mRNA isoforms, MUC4 mRNA isoforms, MUC5AC mRNA isoforms, MUC5B mRNA isoforms, MUC13 mRNA isoforms, or MUC1 mRNA isoforms.

Mature mucins are composed of 2 distinct regions: the amino-and carboxy-terminal regions which are lightly glycosylated but rich in cysteines which participate in establishing disulfide linkages within and among mucin monomers; and a large central region formed of multiple tandem repeats of 10 to 80 residue sequences which are rich in serine and threonine. This area becomes saturated with hundreds of O-linked oligosaccharides.

In the context of the present invention, the term "mucin isoform" or alternatively termed "mucin mRNA isoform" is meant to be a member of a set of similar mRNA molecules or encoded proteins thereof, which originate from a single mucin gene and that are the result of genetic differences. These isoforms may be formed from alternative splicing, variable promoter usage, or other post-transcriptional modifications of the gene. Through RNA splicing mechanisms, mRNA has the ability to select different protein-coding segments (exons) of a gene, or even different parts of exons from RNA to form different mRNA sequences, i.e. isoforms. Each unique sequence produces a specific form of a protein. The presence of genetic differences in mucin genes can result in different mRNA isoforms (i.e. splice variants via alternative splicing) produced from the same mucin gene locus. While most isoforms encode similar biological functions, others have the potential to alter the protein function resulting in progression toward disease. Accordingly, the present invention is specifically directed to the identification and/or use of such mucin isoforms in various disorders. The present invention in particular provides mucin isoforms as defined herein below in the examples part, specifically those referred to in tables 5, 6, 11, S2 and S3; as well as figures 14,15, 28, 29, 30 and 31. It further provides uses of such mucin isoforms as detailed in the present application.

The term "isoform" according to the present invention encompasses transcript variants (which are mRNA molecules) as well as the corresponding polypeptide variants (which are polypeptides) of a gene. Such transcription variants result, for example, from alternative splicing or from a shifted transcription initiation. Based on the different transcript variants, different polypeptides are generated. It is possible that different transcript variants have different translation initiation sites. A person skilled in the art will appreciate that the amount of an isoform can be measured by adequate techniques for the quantification of mRNA as far as the isoform relates to a transcript variant which is an mRNA. Examples of such techniques are polymerase chain reaction-based methods, in situ hybridization-based methods, microarray-based techniques and whole transcriptome long-read sequencing. Further, a person skilled in the art will appreciate that the amount of an isoform can be measured by adequate techniques for the quantification of polypeptides as far as the isoform relates to a polypeptide. Examples of such techniques for the quantification of polypeptides are ELISA (Enzyme-linked Immunosorbent Assay)-based, gel-based, blot-based, mass spectrometry-based, and flow cytometry-based methods.

In a particular embodiment, said mucin isoform is a transmembrane mucin, which is a type of integral membrane protein that spans the entirety of the cell membrane. These mucins form a gateway to permit/prevent the transport of specific substances across the membrane.

In a particular embodiment, the present invention provides an in vitro method for diagnosing and/or determining the severity of a coronaviral infection or coronaviral infectious disease and/or associated co-infections, said method comprising:
a) providing a biological sample from a subject suspected of having a coronaviral infection or coronaviral infectious disease and/or associated co-infections, and
b) determining the presence and/or quantity of one or more mucins selected from the list comprising: MUC16, MUC21, MUC2, MUC4, MUC5AC, MUC5B, MUC13, optionally in combination with MUC1;
wherein the presence and/or quantity of the one or more mucins is indicative for the presence and/or severity of a coronaviral infection or coronaviral infectious disease.

In the context of the present invention, the phrase 'determining the presence and/or quantity of mucins', is meant to be a step in the method in which the expression level of mucins is determined, in order to detect the presence and/or amount of expression of such mucins. This can be determined on the level of protein or mRNA expression levels, by means of suitable techniques including but not limited to: western blotting, ELISA assays, RT-PCR methods, mass spectrometry,...

In yet a further embodiment the presence and/or quantity of at least 2, preferably at least 3 mucins are determined and/or quantified. Accordingly, the present invention provides different combinations of mucins such as but not limited to MUC1 and MUC2, MUC1 and MUC4, MUC1 and MUC5AC, MUC1 and MUC5B, MUC1 and MUC13, MUC1 and MUC16, MUC1 and MUC21, MUC2 and MUC4, MUC2 and MUC5AC, MUC2 and MUC5B, MUC2 and MUC13, MUC2 and MUC16, MUC2 and MUC21, MUC4 and MUC5AC, MUC4 and MUC5B, MUC4 and MUC13, MUC4 and MUC16, MUC4 and MUC21, MUC5AC and MUC5B, MUC5AC and MUC13, MUC5AC and MUC16, MUC5AC and MUC21, MUC5B and MUC13, MUC5B and MUC16, MUC5B and MUC21, MUC13 and MUC16, MUC13 and MUC21, MUC16 and MUC21, either or not further in combination with one or more other mucins selected from the list comprising MUC1, MUC2, MUC4, MUC5AC, MUC5B, MUC13, MUC16, or MUC21.
In a specific embodiment, said method comprises determining the presence and/or quantity of MUC13 and MUC21; and wherein high levels of MUC13 and MUC21 are indicative of a coronaviral infection or coronaviral infectious disease.

In yet a further embodiment, said method comprises determining the presence and/or quantity of MUC1 and MUC16; and wherein high levels of MUC1 and low levels of MUC16 are indicative of a more severe coronaviral infection or coronaviral infectious disease.

In a specific embodiment, said one or more mucin mRNA isoforms are selected from the group comprising MUC1 mRNA isoforms, MUC13 mRNA isoforms, MUC16 mRNA isoforms, or MUC21 mRNA isoforms.

In yet a further embodiment at least 2, preferably at least 3 mucin mRNA isoforms are determined and/or quantified. Accordingly, the present invention provides different combinations of mucin isoforms such as but not limited to MUC1 and MUC2 mRNA isoforms, MUC1 and MUC4 mRNA isoforms, MUC1 and MUC5AC mRNA isoforms, MUC1 and MUC5B mRNA isoforms, MUC1 and MUC13 mRNA isoforms, MUC1 and MUC16 mRNA isoforms, MUC1 and MUC21 mRNA isoforms, MUC2 and MUC5AC mRNA isoforms, MUC2 and MUC5B mRNA isoforms, MUC2 and MUC13 mRNA isoforms, MUC2 and MUC16 mRNA isoforms, MUC2 and MUC21 mRNA isoforms, MUC4 and MUC5AC mRNA isoforms, MUC4 and MUC5B mRNA isoforms, MUC4 and MUC13 mRNA isoforms, MUC4 and MUC16 mRNA isoforms, MUC4 and MUC21 mRNA isoforms, MUC5AC and MUC5B mRNA isoforms, MUC5AC and MUC13 mRNA isoforms, MUC5AC and MUC16 mRNA isoforms, MUC5AC and MUC21 mRNA isoforms, MUC5B and MUC13 mRNA isoforms, MUC5B and MUC16 mRNA isoforms, MUC5B and MUC21 mRNA isoforms, MUC13 and MUC16 mRNA isoforms, MUC13 and MUC21 mRNA isoforms, MUC16 and MUC21 mRNA isoforms, either or not further in combination with one or more other mRNA isoforms selected from the list comprising MUC1 mRNA isoforms, MUC2 mRNA isoforms, MUC4 mRNA isoforms, MUC5AC mRNA isoforms, MUC5B mRNA isoforms, MUC13 mRNA isoforms, MUC16 mRNA isoforms, or MUC21 mRNA isoforms.

In a further embodiment, of the present invention, the presence and/or quantity of MUC1 mRNA isoforms, MUC13 mRNA isoforms, MUC16 mRNA isoforms and MUC21 mRNA isoforms are determined and wherein the presence and/or quantity of said mucin mRNA isoforms is indicative for the presence and/or severity of a coronaviral infection or coronaviral infectious disease.

In yet a further embodiment of the present invention, the presence and/or quantity of MUC13 mRNA isoforms and/or MUC21 mRNA isoforms is determined and wherein the presence and/or quantity of MUC13 mRNA isoforms and/or MUC21 mRNA isoforms is indicative for the diagnosis of a coronaviral infection or coronaviral infectious disease.

In another embodiment of the present invention, the presence and/or quantity of MUC1 and/or MUC16 mRNA isoforms is determined and wherein the presence and/or quantity of MUC1 and/or MUC16 mRNA isoforms is indicative for the severity of the coronaviral infection or coronaviral infectious disease.

The present invention further provides one or more mucin mRNA isoforms for use in the diagnosis and/or monitoring of a coronaviral infection or coronaviral infectious disease, wherein the one or more mucin mRNA isoforms are selected from the list comprising: MUC1 mRNA isoforms, MUC2 mRNA isoforms, MUC4 mRNA isoforms, MUC5AC mRNA isoforms, MUC5B mRNA isoforms, MUC13 isoforms, MUC16 mRNA isoforms, MUC21 mRNA isoforms.

In a particular embodiment, said mucin mRNA isoform is a transmembrane mucin.

In a specific embodiment, the one or more mucin mRNA isoforms for use in the present invention are selected from the list comprising: MUC1 mRNA isoforms, MUC13 isoforms, MUC16 mRNA isoforms, or MUC21 mRNA isoforms.

The present invention also provides a combination of MUC1 mRNA isoforms, MUC13 mRNA isoforms, MUC16 mRNA isoforms and MUC21 mRNA isoforms for use in the diagnosis and/or monitoring of a coronaviral infection or coronaviral infectious disease.

Furthermore, the present invention provides a MUC1 mRNA isoform and/or MUC16 mRNA isoform for use in determining the severity of a coronaviral infection or coronaviral infectious disease.

The present invention also provides a combination of MUC13 mRNA isoforms and MUC21 mRNA isoforms for use in the diagnosis of a coronaviral infection or coronaviral disease.

In another embodiment, the coronaviral infection or coronaviral disease is a SARS-CoV-2 infection or SARS-CoV-2 associated disease.

The invention also provides a diagnostic kit for performing the in vitro method according to present invention, said kit comprising agents for detecting the presence of one or more mucin mRNA isoforms selected from the list comprising MUC1 mRNA isoforms, MUC2 mRNA isoforms, MUC4 mRNA isoforms, MUC5AC mRNA isoforms, MUC5B mRNA isoforms, MUC13 isoforms, MUC16 mRNA isoforms, MUC21 mRNA isoforms.

The specific set of disorders focused on in this application, is that they are characterized by barrier dysfunction. The term barrier dysfunction is meant to be the partial or complete disruption of the natural function of an internal barrier of a subject. Such barriers may for example include the brain barriers, the gastrointestinal mucosal barrier, the respiratory mucosal barrier, the reproductive mucosal barrier and the urinary mucosal barrier.

The gastrointestinal mucosal barrier separates the luminal content from host tissues and plays a pivotal role in the communication between the microbial flora and the mucosal immune system. Emerging evidence suggests that loss of barrier integrity, also referred to 'leaky gut', is a significant contributor to the pathophysiology of gastrointestinal diseases, including IBD (Inflammatory Bowel Diseases).

The blood-brain barrier is a highly selective semipermeable border of endothelial cells that prevents solutes in the circulating blood from non-selectively crossing into the extracellular fluid of the central nervous system. The blood-brain barrier restricts the passage of pathogens, the diffusion of solutes in the blood and large or hydrophilic molecules into the cerebrospinal fluid, while allowing diffusion of hydrophobic molecules (e.g. O₂, CO₂, hormones...) and small polar molecules. Accordingly, an improperly functioning blood-brain barrier can be linked to neurological disorders, in particular neurodegenerative disorders. Not only the blood-brain barrier may have a role in neurological disorders, also other brain barriers, such as the blood-cerebrospinal fluid barrier, may be linked to neurological disorders.

The respiratory mucosal barrier's main function is to form a physical barrier, between the environment and the inside of an organism. It is the first barrier against continuously inhaled substances such as pathogens and allergens. Increased mucus production is often associated with respiratory infections or respiratory diseases, such as for example COPD (Chronic Obstructive Pulmonary Disease). It was moreover found that severely ill COVID-19 patients (i.e. having a SARS-CoV-2 infection) requiring intensive care, may specifically develop mucus hyperproduction in the bronchioles and alveoli of the lungs, an observation which hampers ICU stay and recovery. Accordingly, the present invention may have a significant impact on the diagnosis, monitoring, prevention and/or treatment of respiratory infections, in particular coronaviral infections such as SARS-CoV-2 infections.

Therefore, in a specific embodiment of the present invention, said disease characterized by barrier dysfunction may be a gastrointestinal disorder; a neurodegenerative disorder; cancer, or a respiratory infection.

In a particular embodiment, said gastrointestinal disorder may be selected from the list comprising: Inflammatory Bowel Disease (IBD), Irritable Bowel Syndrome (IBS), cancer, gastro-intestinal infections, obesitas, non-alcoholic fatty liver disease (NAFLD). In another embodiment of the present invention, said Inflammatory Bowel Disease may be selected from the list comprising: Crohn's disease and ulcerative colitis.

In another particular embodiment of the present invention, said cancer may be selected from the list comprising: esophageal cancer, gastric cancer, colorectal cancer, pancreas cancer, liver cancer, kidney cancer, lung cancer, ovarian cancer, colon cancer and prostate cancer.

In a further embodiment of the present invention, said gastro-intestinal infection may be selected from the list comprising: *Helicobacter* infection, *Campylobacter* infection, *Clostridioides difficile* infection and *Salmonella* infection.

In yet a further embodiment of the present invention, said neurodegenerative disorder may be selected from the list comprising: Parkinson's Disease, Alzheimer's Disease, Multiple Sclerosis (MS) and Autism.

In yet a further embodiment, said respiratory infection may be selected from the list comprising: respiratory syncytial viral infections, influenza viral infections, rhinoviral infections, metapneumoviral infections, Pseudomonas aeruginosa viral infections and coronaviral infections. Said coronaviral infection for example being a SARS-CoV-2 infection.

As used herein, the terms "treatment", "treating", "treat" and the like, refer to obtaining a desired pharmacologic and/or physiologic effect. The effect can be prophylactic in terms of completely or partially preventing a disease or symptom thereof and/or can be therapeutic in terms of a partial or complete cure for a disease and/or adverse effect attributable to the disease. "Treatment," as used herein, covers any treatment of a disease or condition in a mammal, particularly in a human, and includes: (a) preventing the disease from occurring in a subject which can be predisposed to the disease but has not yet been diagnosed as having it; (b) inhibiting the disease, i.e., arresting its development; and (c) relieving the disease, i.e., causing regression of the disease.

A "therapeutically effective amount" of an agent described herein is an amount sufficient to provide a therapeutic benefit in the treatment of a condition or to delay or minimize one or more symptoms associated with the condition. A therapeutically effective amount of an agent means an amount of therapeutic agent, alone or in combination with other therapies, which provides a therapeutic benefit in the treatment of the condition. The term "therapeutically effective amount" can encompass an amount that improves overall therapy, reduces or avoids symptoms, signs, or causes of the condition, and/or enhances the therapeutic efficacy of another therapeutic agent.

Prevention of a disease may involve complete protection from disease, for example as in the case of prevention of infection with a pathogen or may involve prevention of disease progression. For example, prevention of a disease may not mean complete foreclosure of any effect related to the diseases at any level, but instead may mean prevention of the symptoms of a disease to a clinically significant or detectable level. Prevention of diseases may also mean prevention of progression of a disease to a later stage of the disease.

The term "patient" is generally synonymous with the term "subject" and includes all mammals including humans. Examples of patients include humans, livestock such as cows, goats, sheep, pigs, and rabbits, and companion animals such as dogs, cats, rabbits, and horses. Preferably, the patient is a human.

The term "diagnosing" as used herein means assessing whether a subject suffers from a disease as disclosed herein or not. As will be understood by those skilled in the art, such an assessment is usually not intended to be correct for all (i.e. 100%) of the subjects to be identified. The term, however, requires that a statistically significant portion of subjects can be identified. The term diagnosis also refers, in some embodiments, to screening. Screening for diseases, in some embodiments, can lead to earlier diagnosis in specific cases and diagnosing the correct disease subtype can lead to adequate treatment.

In another particular embodiment, the present invention provides a mucin isoform as defined herein, for use as a biomarker for diagnosis and disease surveillance or monitoring.

By monitoring the progression and change of MUC mRNA isoform status of the individual using the methods of the present invention, the clinician or practitioner is able to make informed decisions relating to the treatment approach adopted for any one individual. For example, in certain embodiments, it may be determined that patients having specific mucin isoforms may or may not react to a particular treatment. Thus, by monitoring the response of mucin isoform carriers to various treatment approaches using the methods of the present invention, it is also possible to tailor an approach which combines two or more treatments, each targeting different subsets of isoforms in the individual.

In another particular embodiment, the present invention provides a mucin isoform as defined herein, for use as a new therapeutic target. In particular, said mucin isoform may be specifically targeted by monoclonal antibodies, small molecules or antisense technology.

### EXAMPLES

### EXAMPLE 1: In-depth study of transmembrane mucins in association with intestinal barrier dysfunction during the course of T cell transfer and DSS-induced colitis

### 1. BACKGROUND TO THE INVENTION

Inflammatory bowel diseases (IBD), including Crohn's disease (CD) and ulcerative colitis (UC), remain disease entities with a high morbidity burden and are characterized by perpetual chronic relapsing inflammation of the intestines. At this moment, there is no curative treatment for IBD, which is why patients require life-long medication and often need surgery. Treatment mainly focuses on immunosuppression and still a substantial number of patients fail to respond or obtain full remission.

The etiology and pathogenesis of IBD are believed to involve inappropriate immune responses to the complex microbial flora in the gut in genetically predisposed persons. The intestinal mucosal barrier separates the luminal content from host tissues and plays a pivotal role in the communication between the microbial flora and the mucosal immune system. Emerging evidence suggests that loss of barrier integrity, also referred to 'leaky gut', is a significant contributor to the pathophysiology of IBD. The intestinal mucosal barrier comprises a thick layer of mucus, a single layer of epithelial cells and the lamina propria hosting innate and adaptive immune cells. Integrity of this barrier is maintained in several ways as depicted in Figure 1. Secreted (e.g. MUC2) and transmembrane (e.g. MUC1, MUC4, MUC13) mucins represent the major components of the mucus barrier and are characterized by domains rich in proline, threonine, and serine that are heavily glycosylated (i.e. PTS domains). In addition to having a protective function, transmembrane mucins possess extracellular EGF-like domains and intracellular phosphorylation sites which enable them to also participate in the intracellular signal transduction. In this way, they can modulate intestinal inflammation by affecting epithelial cell proliferation, survival, differentiation and cell-cell interactions. The intestinal epithelium underneath plays an active role in innate immunity via the secretion and expression of mucins and antimicrobial peptides as well as by hosting antigen presenting cells. At this level, intense communication takes place between intestinal epithelial cells (IECs), immune cells, the microbiome and environmental antigens shaping immune responses towards tolerance or activation. IECs are mechanically tied to one another and are constantly renewed to maintain proper barrier function. This linkage is achieved by three types of intercellular junctions, listed from the apical to basal direction: tight junctions, adherens junctions and desmosomes. Whereas the adherens junctions and desmosomes are essential to maintain cell-cell adhesion by providing mechanical strength to the epithelium, tight junctions regulate paracellular permeability and seal the intestinal barrier. Tight junctions mainly consist of claudins (CLDNs), occludin (OCLN) and junctional adhesion molecules (JAMs). Apart from linking neighbouring cells, they associate with peripheral intracellular membrane proteins, such as zonula occludens (ZO) proteins, which anchor them to the actin cytoskeleton. Furthermore, tight junctions are also involved in regulating cell polarity which is established by the mutual interaction of three evolutionary conserved complexes: defective partitioning (PAR; PAR3 - PAR6 - aPKC), Crumbs (CRB3 - PALS1 - PATJ) and Scribble (SCRIB - DLG - LGL) complexes (Figure 1). The Crumbs complex defines the apical membrane whereas the PAR and Scribble complexes are responsible for the establishment of the apical-lateral junctions between cells and the basolateral membrane, respectively. These polarity complexes are thus complementary and act together to initiate and maintain apical-basal polarity.

To date, the mechanisms underlying altered function of the intestinal mucosal barrier in IBD remain largely unexplored, particularly the role of mucins. Moehle et al., 2006 described a downregulation of *MUC2* mRNA in the colon of CD patients and increased colonic mRNA levels of *MUC13* in patients with UC. This latter finding was also confirmed by another study (Sheng et al., 2011), whereas Vancamelbeke and colleagues showed a stable upregulation of *MUC1* and *MUC4* mRNA in both the ileum and colon of IBD patients compared to controls (Vancamelbeke et al., 2017). Upon inflammation, MUC1 and MUC13 have been shown to possess divergent actions to modulate mucosal epithelial signalling, with MUC1 being anti-inflammatory and MUC13 pro-inflammatory (Linden et al., 2008; Sheng et al., 2012). Initially, elevated MUC13 during inflammation inhibits epithelial cell apoptosis, and impairment of its expression could lower the level of protection (Sheng et al., 2011). Similarly, MUC1 protects the gastrointestinal epithelial cells from infection-induced apoptosis and enhances the rate of wound healing after injury. It should also be noted that inappropriate overexpression of transmembrane mucins could affect barrier integrity by modulating apical-basal cell polarity and cell-cell interactions, resulting in tight junction dysfunction, and may thus be responsible for the progression from local inflammation to more severe diseases, including IBD.

Therefore, in order to enhance our understanding of the role of transmembrane mucins as novel players in intestinal mucosal barrier dysfunction in IBD, we conducted an *in vivo* study to characterize changes in barrier components affecting integrity during the course of colitis using two complementary mouse models.

### 2. MATERIAL AND METHODS

### 2.1.Animals

Eight- to nine-week-old female immunocompromised SCID (C.B-17/lcr-*Prkdc*^{scid}/lcrlcoCrl) and BALB/c mice (T cell transfer model) and 7- to 8-week-old male C57BL/6J mice (DSS model) were purchased from Charles River (France). All animals were housed in a conventional animal facility with ad libitum access to food and water and a light cycle of 12 hours. After arrival in the animal facility, mice were allowed to acclimatize for 7 days before the onset of the experiments.

### 2.2. Colitis models and experimental design

Mouse models of colitis have been major tools in understanding the pathogenesis of IBD, yet each separate model has its limitations in that it not fully recapitulates the complexity of this human disease. Among these, the adoptive T cell transfer model has mainly been used to investigate the immunological mechanisms of intestinal inflammation mediated by T cells, and to a lesser extent to study barrier integrity. By contrast, the dextran sodium sulphate (DSS) model has been described as a useful model to examine the innate immune mechanisms involved in the development of intestinal inflammation and barrier dysfunction. More specifically, DSS is toxic to the colonic epithelium and oral administration of this chemical compound causes epithelial cell injury and innate immune responses which alter mucosal barrier integrity. As each colitis model provides valuable insights into a certain aspect of IBD, using multiple models with different initiation of pathology will thus yield a broader picture of the pathophysiology of these diseases, including barrier dysfunction.

**T-cell transfer model:** colitis was induced in SCID mice by the adoptive transfer of CD4⁺ CD25⁻ CD62L⁺ T cells isolated from the spleens of BALB/c donor mice as described before (Figure 2A). To monitor disease progression, animals were weighed every week and clinically scored based on the following clinical disease parameters: weight loss, piloerection, stool consistency and mobility. Each parameter was graded from 0 to 2 according to disease severity (0 = absent, 1 = moderate, 2 = severe; for weight loss, 0 = weight gain, 1 = stable, 2 = weight loss). The cumulative score hence ranged from 0 to 8. In addition, intestinal inflammation was also monitored in a continuous manner in individual mice by colonoscopy at fixed time points (weeks 0, 2, 4 and 6) using a flexible Olympus URF type P5 ureteroscope with an outer diameter of 3.0 mm (Olympus Europe GmbH). Briefly, mice were sedated with a mixture of ketamine (60mg/kg, Ketalar, Pfizer) and xylazine (6.67 mg/kg, Rompun, Bayer) (intraperitoneally (i.p.)) and placed in prone position. The anal sphincter was lubricated with gel (RMS-endoscopy) to facilitate insertion of the endoscope. Subsequently, the scope was carefully inserted through the anus as far as possible into the colon of the sedated mouse. A score was given during the withdrawal of the scope for the following parameters: morphology of the vascular pattern, bowel wall translucency, fibrin attachment and presence of loose stools (each ranging from 0 to 3), with a cumulative minimum of 0 (no inflammation) and a maximum of 12 (severe inflammation).

**DSS-induced colitis model:** acute colitis was induced by administering 2% DSS (36-50 kDa) to autoclaved drinking water for 7 days ad libitum. This cycle was repeated two more times with intermediate recovery phases of normal drinking water for 7 days to induce more chronic forms of colitis. Control mice received only autoclaved drinking water (Figure 3A). Water levels were checked every day and were refreshed every other day. Each day, an individual disease activity index (DAI) was calculated by analysing weight loss (0 = <1%, 1 = 1-5%, 2 = 5-10%, 3 = 10-20%, 4 = >20%), stool consistency (0 = normal, 1 = semi-solid, 2 = loose stools, 4 = diarrhea) and rectal bleeding (0 = no bleeding, 2 = blood visible, 4 = gross bleeding) to obtain a cumulative score of these parameters ranging from 0 (healthy) to 12 (severe colitis).
At 1, 2, 4 and 6 weeks post-transfer and at the end of each DSS treatment (Figure 2A & 3A), 10-14 animals per group (control, T cell transfer and DSS) were sacrificed by exsanguination under anesthesia (90 mg/kg ketamine and 10 mg/kg xylazine; i.p.). The collected blood was centrifuged to obtain serum for further analysis. Subsequently, the colon was resected, feces were removed and the weight as well as the length of the colon were determined and expressed as the weight/length ratio (mg/cm). Macroscopic inflammation was then scored based on the following parameters: presence of ulcerations, hyperemia, bowel wall thickening and mucosal edema. For the T cell transfer model, each parameter was scored from 0 to 3 depending on the severity, leading to a maximum cumulative score of 12 as described by Heylen *et al., 2013.* For the DSS model, the macroscopic scoring system of Wallace *et al.,* 1992. was used resulting in a score from 0 to 5. Thereafter, different samples from the colon (distal side) were taken and processed immediately or stored in RNA later, snapfrozen or embedded in paraffin or cryoprotectant until further analysis (see below).

### 2.3. Myeloperoxidase (MPO) activity assay

Myeloperoxidase (MPO) activity was measured in colonic tissue as a parameter for neutrophil infiltration (Heylen et al., *2013*). Briefly, colonic samples were immersed in potassium phosphate (pH 6.0) containing 0.5% hexadecyltrimethylammonium bromide (0.02 mL/mg tissue). Thereafter, samples were homogenized, subjected to two freeze-thawing cycles and subsequently centrifuged at 15000 rpm for 15 min at 4°C. An aliquot (0.1 mL) of the supernatant was then added to 2.9 mL of o-dianisidine solution (i.e. 16.7 mg of o-dianosidine dihydrochloride in 1 mL of methyl alcohol, 98 mL of 50 mM potassium phosphate buffer at pH 6.0 and 1 mL of 0.005% H₂O₂ solution). Immediately afterwards, the change in absorbance of the samples was read at 460 nm over 60 sec using a Spectronic Genesys 5 spectrophotometer (Milton Roy). One unit of MPO activity equals the amount of enzyme able to convert 1 mmol of H₂O₂ to H₂O per min at 25°C.

### 2.4. RNA extraction and RT-qPCR for gene expression

Total RNA from colonic tissue stored in RNA later, was extracted using the NucleoSpin^{®} RNA plus kit (Macherey-Nagel) following the manufacturer's instructions. The concentration and quality of the RNA were evaluated using the NanoDrop^{®} ND-1000 UV-Vis Spectrophotometer (Thermo Fisher Scientific). Subsequently, 1 µg RNA was converted to cDNA by reverse transcription using the SensiFast^{™} cDNA synthesis kit (Bioline). Relative gene expression was then determined by SYBR Green RT-qPCR using the GoTaq qPCR master mix (Promega) on a QuantStudio 3 Real-Time PCR instrument (Thermo Fisher Scientific). Primer sequences are shown in Supplementary Table 1.

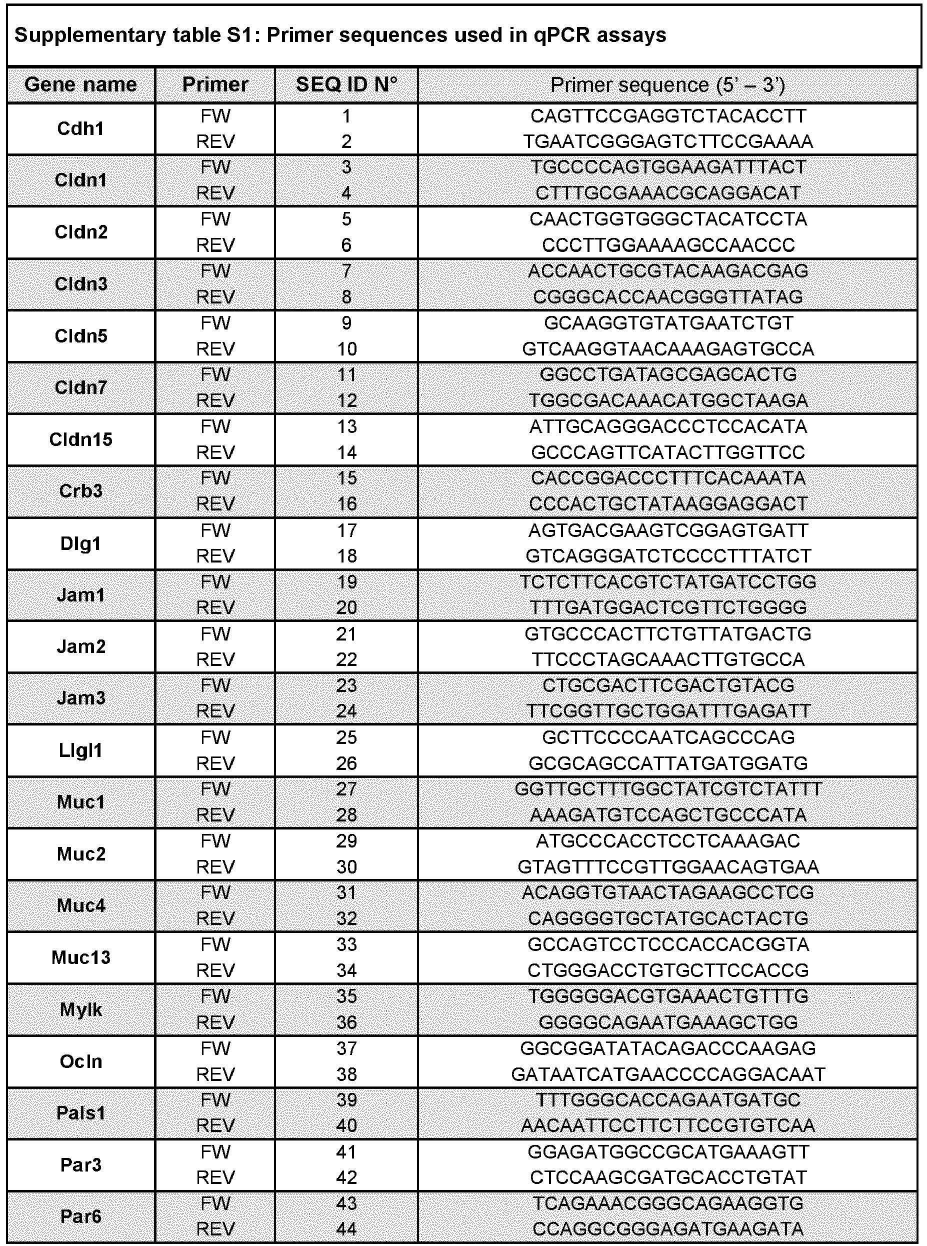

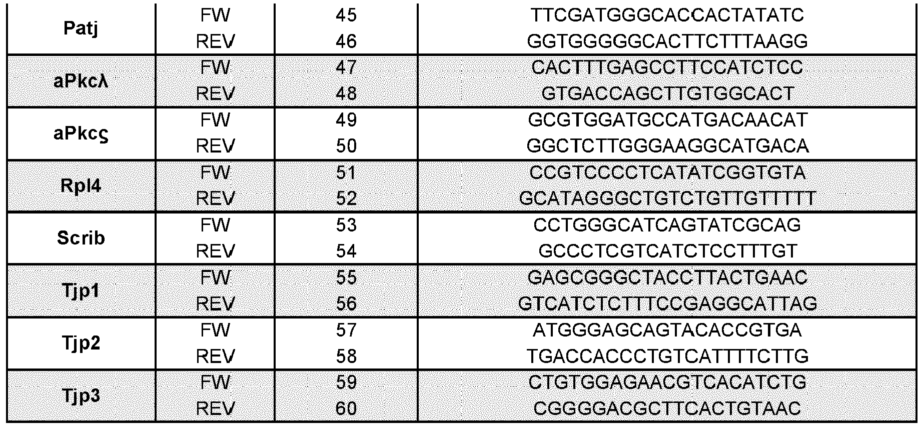

All RT-qPCR reactions were performed in duplicate and involved an initial DNA polymerase activation step for 2 min at 95°C, followed by 40 cycles of denaturation at 95°C for 15 sec and annealing/extension for 1 min at 60°C. Analysis and quality control were performed using qbase+ software (Biogazelle). Relative expression of the target genes was normalized to the expression of the housekeeping genes *Actb* and *Rpl4.*

### 2.5. Quantification of intestinal permeability

To assess *in vivo* intestinal permeability, the FITC-dextran intestinal permeability assay was performed as described by Gupta *et al., 2014.* In brief, mice were intragastrically inoculated 4 hours prior to euthanasia with FITC-dextran (44 mg/100 g body weight (T cell transfer), 60 mg/100 g body weight (DSS model), 4 kDa, Sigma). Upon euthanasia, blood was collected via cardiac puncture and transferred into SSTII Advance Blood Collection Tubes (BD Vacutainer). After centrifugation (10000 rpm, 5 min), serum was collected and equally diluted with PBS. Subsequently, aliquots of 100 µl were added in duplo to a 96-well microplate and the concentration of FITC was measured by spectrophotofluorometry (Fluoroskan Microplate Fluorometer, Thermo Fisher Scientific) at an excitation wavelength of 480 nm and an emission wavelength of 530 nm. The exact FITC-dextran concentration per well was calculated using a standard curve with serially diluted FITC-dextran solutions.

### 2.6. Cytokine measurements

To determine colonic inflammatory mediators at protein level, two different approaches were applied. First, fresh colonic segments were rinsed with PBS, blotted dry and weighed. Subsequently, the samples were stored on ice until further processing in a Tris-EDTA buffer (i.e. PBS containing 10 mM Tris, 1 mM EDTA, 0.5% *v*/*v* Tween-20 and a protease-inhibitor cocktail (Sigma-Aldrich)) at a ratio of 100 mg tissue per ml buffer. Samples were then homogenized, centrifuged (11 000 rpm, 10 min, 4°C) and the supernatants were stored at - 80°C until further analysis. Colonic cytokine levels were quantified using cytometric bead arrays (CBA) (BD Biosciences) for Tumour Necrosis Factor (TNF)-α, Interferon (IFN)-γ, Interleukin (IL)-1β and IL-6 according to the manufacturer's instructions. Fluorescence detection was performed on a BD Accuri C6 flow cytometer and the FCAP Array software was used for data analysis.

Second, snap frozen colonic tissues were homogenized using beads and total protein was extracted in ice cold NP-40 buffer (i.e. 20 mM Tris HCI (pH 8), 137 mM NaCl, 10% glycerol, 1% nonidet-40, 2 mM EDTA) supplemented with protease and phosphatase inhibitor cocktail tablets (Roche). After centrifugation (14.000 rpm, 10 min, 4°C) to remove cell debris, the protein concentration was determined using the Pierce BCA protein assay kit (Thermo Fisher Scientific). Enzyme-Linked ImmunoSorbent Assay (ELISA) was then performed to quantify colonic cytokine expression at the protein level. To this end, the mouse uncoated ELISA kits (Invitrogen) were used according to the manufacturer's instructions to measure protein concentrations of IL-1β, TNF-α, IL-6, IL-10 and IL-22. A standard curve was created by performing 2-fold serial dilutions of the top standards included in the kits. For each sample, 100µl of a 2.5 µg/ml protein solution was analysed by ELISA in duplicate.

### 2.7. Histopathology and immunohistochemistry

In order to evaluate inflammation at the microscopic level, full thickness colonic segments were fixed for 24 h in 4% formaldehyde and subsequently embedded in paraffin. Cross sections (5 µm thick) were deparaffinized and rehydrated. Sections were then stained with Hematoxylin Gill III Prosan (Merck) and Eosin Yellow (VWR) according to the standardized protocols. Inflammation was scored based on the degree of inflammatory infiltrates (0-3), presence of goblet cells (0-1), crypt architecture (0-3), mucosal erosion and/or ulceration (0-2), presence of crypt abscesses (0-1) and the number of layers affected (0-3), resulting in a cumulative score ranging from 0 to 13 (Moreels et al., 2004). Periodic Acid-Schiff (PAS) staining was performed to detect mucin glycoproteins in paraffin-embedded colon sections. In brief, rehydrated 5 µm thick colon sections were placed in Schiff reagent for 15 min after an initial oxidation step in 0.5% periodic acid solution for 5 min. Then, colon sections were washed with tap water, counterstained with hematoxylin and analysed by light microscopy (Olympus BX43).

Several immunohistochemical mucin stainings were also applied on paraffin-embedded colonic tissue using the following primary antibodies: the polyclonal rabbit Muc1 (Abcam (ab15481), 1/1000), Muc2 (Novus Biologicals (NBP1-31231), 1/3000), Muc4 (Novus Biologicals (NBP1-52193SS), 1/3000) and the in-house Muc13 (1/2000) antibodies. Briefly, heat-induced antigen retrieval was performed in EDTA (pH 8) (MUC1 and MUC13) or citrate buffer (10 mM, pH 6) (MUC2 and MUC4). Subsequently, endogenous peroxidase activity was blocked by incubating the slides with 3% H₂O₂ in methanol (5 min). Primary antibody incubation was performed overnight at 4°C. Subsequently, the mucins were visualized by incubating the colon sections with a goat anti-rabbit biotinylated secondary antibody (EnVision detection system for MUC13) for 60 min at room temperature, followed by incubation with HRP-avidin complexes. Finally, visualization of the target antigen was performed by a short incubation with aminoethyl carbazole (AEC), after which the sections were counterstained with hematoxylin. Washing steps were performed using Tris-buffered saline containing 0.1% Triton X-100 (pH 7.6). The stainings were analysed by light microscopy (Olympus BX43).

To visualize tight junctions in the colon, fresh colonic tissue was transversally placed and immersed in Richard-Allan Scientific^{™} Neg-50^{™} Frozen Section Medium (Thermo Fisher Scientific) and snap frozen, after which 6 µm cryosections were mounted on SuperFrost slides (Thermo Fisher Scientific). After a short fixation period of 5 min in aceton, the sections were dried and rinsed with Tris-buffered saline supplemented with 1% albumin. The sections were then incubated overnight with the following primary antibodies: ZO-1 (Invitrogen (61-7300), 1/1000) and CLDN1 (abcam (ab15098), 1/2000). The next day, secondary antibody incubation was performed for 60 min using a goat anti-rabbit Alexa Fluor 594 secondary antibody (Invitrogen, 1/800). After rinsing in distilled water, the colon sections were counterstained and protected against fading using Vectashield mounting medium containing DAPI (Vector Laboratories). Washing steps were performed using Tris-buffered saline supplemented with 0.1% Triton X-100. For visualization, a Nikon Eclipse Ti inverted fluorescence microscope equipped with a Nikon DS-Qi2 camera was used. All sections were blinded to obtain the representative images.

### 2.8. Statistics

Statistical analysis using the GraphPad Prism 8.00 software (licence DFG170003) was performed to determine significant differences between control and the different colitis groups within a certain model (T cell transfer or DSS). Data were analysed by the One-way Analysis of Variance (ANOVA) and non-parametric Kruskal-Wallis tests and are presented as means ± standard error of mean (SEM) or boxplots (min to max), unless stated otherwise. Significance levels are indicated on the graphs by *p<0.05, **<0.01, ***p<0.001 and were corrected for multiple testing using the Tukey-Kramer's and Dunn's post-hoc multiple comparisons tests.

A discriminant function analysis was performed to determine whether colitis mice could be distinguished from control animals based on a set of predictor variables (i.e. the expression of cytokines, mucins or other barrier mediators). The results are depicted as scatter plots showing the two main discriminant functions (i.e. function 1 and function 2) with the according main predictor variables summarized in a table. Furthermore, a multiple linear regression analysis was carried out to investigate associations (1) between changes in barrier integrity and the expression of mucins, cytokines and barrier mediators; (2) between the expression of mucins, cytokines and barrier mediators. Scatter plots are shown distinguishing between different experimental groups with the corresponding p-value of the regression model. A p-value below 0.05 was considered statistically significant. These analyses were performed using IBM SPSS Statistics 24 software.

### 3. RESULTS

### 3.1. Macroscopic and microscopic observations of colitis evolution over time

In the **T cell transfer model,** SCID mice started to develop clinical signs of colitis one week after the adoptive transfer of naïve T cells. Body weight was decreased at 1week post-transfer compared to the initial body weight pre-transfer and this decrease gradually continued until week 6 (Figure 2B). The clinical disease score increased over time starting from week 1 to week 4, while stagnating afterwards (Figure 2C). A colonoscopy was performed every 2 weeks to monitor signs of colitis in the bowel wall, showing a time-dependent increase in inflammatory scores at weeks 2, 4 and 6 post-transfer compared to the control mice (Figure 1D). After sacrifice, the mucosal damage in the colon was scored at both a macro- and microscopic level. Mice that were sacrificed at 2, 4- and 6-weeks post-transfer showed a gradual increase in macroscopic inflammation (Figure 2F). This phenomenon was also seen for another macroscopic marker of colonic inflammation, the colonic weight/length ratio, which is a quantification of colonic edema (Figure 2E). In contrast, the infiltration of neutrophils and lymphocytes became already visible on H&E-stained colonic segments of colitis mice at week 1 post-transfer (Figure 2G). This mucosal and submucosal infiltration of immune cells gradually increased and was associated with a remarkable increase in colon thickness as disease progressed to weeks 2, 4 and 6 (Figure 2G). Furthermore, MPO activity, which is caused by neutrophil infiltration in the mucosa, was increased starting from 2 weeks post-transfer, with a gradual increase over time to weeks 4 and 6 (Figure 2H).
In the **DSS colitis model,** mice treated with DSS started to lose weight after 5 days of DSS administration in the first cycle. The body weight further decreased when normal drinking water was reintroduced at day 8, with a maximal weight loss at day 11 of the experimental protocol (Figure 3B). The colitis mice started to regain weight at the end of the second DSS cycle (day 21) until the initial body weight was reached at the end of the experiment. Healthy control mice gained weight over time (Figure 3B). As a result of DSS administration, mice in each DSS group showed maximal changes in stool consistency and rectal bleeding after 7 days of DSS administration, which decreased and completely disappeared in the recovery phase (Figure 3). The above-described parameters to assess clinical disease in this model (body weight, stool consistency and rectal bleeding) are combined in the DAI score, which is shown in Figure 3D. Control mice did not show any signs of disease throughout the experiment, whereas administration of 2% DSS for 7 days stably induced a mild acute colitis after DSS cycle 1. The two subsequent DSS cycles, however, led to the development of a chronic colitis with an increased interindividual variability.

To assess the effect of DSS-induced colitis on macro- and microscopic inflammatory parameters of the colon, a group of mice was sacrificed after each cycle of DSS administration (DSS cycle 1, DSS cycle 2 and DSS cycle 3, respectively, Figure 3A). The colonic weight/length ratio was increased in all three groups (cycles 1, 2 and 3) compared to the control group. The macroscopic inflammation score was increased in all DSS cycles (Figure 3F) with hyperemia and ulcerations abundantly present after DSS cycle 1, whereas colon thickening appeared after DSS cycles 2 and 3. Microscopic inflammation was present in all DSS groups as scored on H&E-stained colon sections (Figure 3G) and showed crypt loss, epithelial erosions and marked infiltration of neutrophils in the colon of acute DSS treated mice (data not shown). At the end of DSS cycles 2 and 3, the colon sections showed epithelial regeneration compared to the acute stage, yet with remarkable hyperplasia. Infiltration of neutrophils and lymphocytes in the submucosa and mucosa could also be observed (data not shown). In addition, some mice even showed massive focal ulcerations in the colon. At the molecular level, MPO activity was increased during DSS-induced colitis progression (Figure 3H), which confirmed the infiltration of neutrophils into the colon due to DSS administration. Interestingly, mice treated with 3 DSS cycles showed a significant lower colonic MPO activity compared to mice treated only once.

### 3.2. Colonic inflammatory markers

In both colitis models, colonic protein levels of several inflammatory markers were quantified as shown in Figure 4. At all timepoints post-transfer and after each cycle of DSS administration, expression of IL-1β and TNF-α was increased whereas IL-10 was reduced in expression (Figure 4A-B, D, F-G, I). Interestingly, IL-22 protein levels were only increased at 1 and 6 weeks post-transfer and at the end of DSS cycles 1 and 3 (Figure 4E, J). In contrast, expression of IL-6 was only increased in the more chronic phase of colitis, i.e. at week 6 post-transfer (Figure 4C) and after the second cycle of DSS administration (Figure 4H).

### 3.3. Mucosal barrier function during colitis progression

As loss of intestinal barrier integrity is recognized as a major hallmark of the IBD pathophysiology¹⁸, changes in barrier permeability during colitis progression were investigated in both models. Results of the FITC-dextran intestinal permeability assays showed that integrity of the intestinal mucosal barrier was affected in both models (Figure 5). More specifically, intestinal permeability progressively increased during colitis progression in the T cell transfer model levelling off at week 6, but remaining increased as compared to control mice (Figure 5A). In the DSS model, intestinal permeability showed a strong increase after the first cycle of DSS administration, after which it declined in the chronic stages of colitis with only a significant increase left after the second DSS cycle but not after the third cycle (Figure 5B). To further substantiate intestinal mucosal barrier dysfunction upon colitis, the expression of several components that are the building stones of and regulate the mucosal barrier were measured.

We first investigated mucin expression since mucins constitute the main part of the mucus layer and are the first barrier luminal pathogens and toxins encounter. *Muc2* (i.e. the main secreted mucin of the large intestine) mRNA expression was increased after 1 week post-transfer (Figure 6A) whereas it was upregulated during the chronic stages of DSS-induced colitis (Figure 7A). mRNA expression of *Muc1,* a transmembrane mucin expressed only at low levels in the healthy intestines, was upregulated after 2, 4 and 6 weeks post-transfer (Figure 6B) and after all cycles of DSS administration (Figure 7B). The transmembrane *Muc13* mucin, which is normally expressed in the healthy intestines, showed aberrant expression patterns at the RNA level in both models with an increased expression seen at 1 and 2 weeks after T cell transfer and DSS cycle 2 (Figures 6D & 7D). In contrast, mRNA expression of *Muc4,* another membrane-bound mucin, was not significantly altered during experimental colitis in either model (Figures 6C & 7C). The changes in mucin mRNA expression were verified at protein level by immunohistochemical stainings (data not shown). In the DSS model, we observed increased Muc2 staining intensity during colitis progression, whereas in the T cell transfer model, overall Muc2 staining intensities were not altered compared to control animals. In control animals, Muc1 was mainly observed on the apical side of epithelial cells lining the villi, whereas colitis induction was associated with increased Muc1 staining intensities in the cytoplasm and the crypts in both colitis models. Muc13 intensity was mainly increased after the first two cycles of DSS administration and from week 2 post-transfer in the T cell transfer model. Concerning its cellular localisation, Muc13 showed a strong apical staining intensity in intestinal epithelial cells, which became apparent in the cytoplasm during colitis. For Muc4, no clear changes were observed during colitis progression compared to control animals.

Several interesting alterations were observed in both models as far as the expression patterns of junction constituents at RNA level were concerned (Figures 8 & 9). mRNA expression levels of *Zo1* (*Tjp1*)*, Tjp2, Jam2, Jam3* and Myosin Light Chain Kinase (*Mylk*) were significantly increased at week 1 post-transfer and after the first cycle of DSS administration (Figures 8 & 9). E-cadherin (*Cdh1*) and *Ocln* mRNA expression levels were significantly decreased during the more chronic stages of experimental colitis in both models (Figures 8 & 9). mRNA levels of *Cldn1,* a major regulator of paracellular permeability, were elevated after the first DSS cycle, whereas it decreased throughout colitis progression in the T cell transfer model (Figures 8 & 9). In contrast, *Cldn2* mRNA expression was increased at 1 week post-transfer, yet its expression declined at the end of each DSS cycle (Figures 8 & 9). In addition, *Cldn5* and *Cldn7* showed a model-specific response. More specifically, expression of *Cldn7* and *Cldn5* mRNA was upregulated at the initial stage of colitis in the T cell transfer and the DSS model, respectively (Figures 8 & 9). Furthermore, *Tjp3* mRNA expression was reduced throughout colitis progression in the DSS-induced colitis model only, whereas *Cldn15* mRNA expression was significantly decreased during the acute phase of DSS-induced colitis and became significantly increased in the chronic phases (Figure 9). Expression of *Cldn3* and *Jam1* was not altered throughout colitis progression in either model (Figures 8 & 9). Immunohistochemical stainings for ZO-1 and CLDN1 were also performed to analyse alterations in intercellular junctions at the protein level. These results showed that mainly CLDN1 showed an increased staining intensity during the course of colitis in both models highlighting dysfunction of this tight junction protein, whereas no clear alterations could visually be observed for ZO-1 (data not shown).

In addition to appropriate expression of intercellular junctions, a well organised apical-to-basal cell polarity is indispensable for the formation of a functional and tight intestinal epithelial cell monolayer. Gene expression analysis showed that subunits of the different polarity complexes were affected in both our experimental colitis mouse models (Figure 10). The expression of *Par3* and *aPkcλ,* two major coordinators of tight junction localization, was downregulated at all DSS cycles and time points post-transfer (Figure 10A). On the other hand, *aPkc* 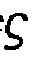 mRNA expression was only decreased in the T cell transfer model, whereas *Par6* mRNA expression was only elevated at the acute phase of DSS-induced colitis (Figure 10A). Regarding the subunits of the Crumbs polarity complex as shown in Figure 10B, *Patj* mRNA expression tended to be decreased at all DSS cycles, whereas its expression was upregulated at week 1 post-transfer. Also mRNA expression of *Pals1* (*Mpp5*) was upregulated at the first time-point of the T cell transfer model (Figure 10B). No significant alterations in *Crb3* expression were observed in either colitis models (Figure 10B). Interestingly, *Scrib* expression, which is known to be a negative regulator of the PAR complex, was increased at 1 week post-transfer and after the first DSS cycle (Figure 10C). Although expression of *Dlg1* and *Llgl1* was altered in the T cell transfer model at 1 and 2 weeks post-transfer, respectively, no changes in expression of these subunits were observed in the DSS-colitis model (Figure 10C). The above results highlight that epithelial cell polarity is disturbed as a consequence of colitis induction, both in the acute and chronic stages.

### 3.4.Aberrant mucin expression associated with loss of barrier integrity upon inflammation

It has been suggested that overexpression of transmembrane mucins in many cancer types can contribute to loss of epithelial barrier integrity by mediating junctional and cell polarity dysfunction. To elucidate the involvement of aberrantly expressed transmembrane mucins as potential mediators in intestinal mucosal barrier disruption upon inflammation-induced colitis, the mucin mRNA expression data were used to perform a discriminant analysis on both models and to correlate the changes in intestinal permeability and colonic inflammation (Figures 11 & 12).

In the T cell transfer model, *Muc1* and *Muc13* expression were the best factors to discriminate whether mice developed colitis by the adoptive transfer of T cells or were controls (Figure 11A). In the DSS colitis model, *Muc2* expression was found to be the major determinant for identifying mice receiving a DSS treatment, followed by expression of *Muc1* and *Muc13* (Figure 11B). Interestingly, increased *Muc1* expression correlated significantly with increased intestinal permeability (based on FITC dextran levels in sera) in the T cell transfer model (Figure 12A), whereas a positive significant correlation between aberrant *Muc13* expression and increased intestinal permeability was seen in the DSS model (Figure 12B). Furthermore, whereas IL-1β was associated with increased permeability and aberrant *Muc1* expression in T cell transfer colitis (Figure 12A&C), TNF-α positively correlated with intestinal permeability and increased *Muc13* expression in DSS-induced colitis (Figure 12B&D). Besides, the expression levels of *Muc13* also correlated with *Muc1* (p = 0.013) and *Muc2* (p = 0.026) expression in the DSS model (data not shown).

In both colitis models, altered expression of several junctional and polarity proteins correlated significantly with each other (data not shown), further indicating mutual dependence and their involvement in regulating barrier integrity. Moreover, their expression levels could also be used to discriminate between colitis mice and controls (Figure 13). Furthermore, significant associations between aberrant *Muc1, Cldn1, Ocln, Par3 and aPKCζ* expression in the T cell transfer model (Figure 12E&G) and between aberrant *Muc13, Cldn1, Jam2, Tjp2, aPkcζ, Crb3* and *Scrib* expression in the DSS model (Figure 12F&H) further suggested a potential role for Muc1 and Muc13 in intestinal mucosal barrier dysfunction.

### 4. DISCUSSION

The intestinal mucosal barrier plays a critical role in gut health and function. Not only is it a physical barrier between the microbiome, toxins and food antigens in the lumen and the internal host tissues, it also is a dynamic barrier that regulates inflammatory responses. Loss of barrier integrity is generally accepted as a major hallmark in the pathophysiology of IBD. However, whether intestinal barrier dysfunction is a primary contributor to or rather a consequence of intestinal inflammation has not yet been fully elucidated. In this study, we investigated intestinal barrier integrity and inflammation during the course of colitis using the T cell transfer and DSS mouse models. These two models have a different mechanism of initiation of colitis and both are standard IBD models. In both models, increased intestinal permeability in association with an innate inflammatory response, as characterized by increased expression of the pro-inflammatory cytokines TNF-α and IL-1β and decreased expression of the anti-inflammatory cytokine IL-10, was already seen at 1 week post-transfer and after the first DSS administration, and was maintained during the course of disease. Excessive production of TNF-α and IL-1β has been described in IBD patients and these harmful cytokines, produced by T cells, macrophages and neutrophils, are likely to affect intestinal homeostasis leading to further aggravation of inflammation. In our study, increased expression of IL-6 appeared only in later stages of colitis progression. This pro-inflammatory cytokine has been shown to be an important mediator of Th17 cell differentiation, further promoting intestinal inflammation in IBD and modulating intestinal epithelial cells. Also IL-22 was increasingly expressed at the beginning of colitis induction and even at week 6 post-transfer and after the last DSS cycle. This cytokine is normally able to promote mucosal healing in the intestine, but when uncontrolled, it can lead to intestinal inflammation. Based on the above findings, we cannot clearly substantiate whether loss of barrier integrity precedes intestinal inflammation as suggested by several studies, that showed that increased intestinal permeability was present in first-degree relatives of IBD patients before intestinal inflammation occurred. However, expression analysis of junctional proteins and polarity complexes in both our models revealed that most changes already occurred at the beginning of colitis development. This would suggest that loss of barrier integrity is not only a result of an innate inflammatory response but might also be a primary contributor in the pathophysiology of IBD. The key mediators underlying mucosal barrier dysfunction upon inflammation in IBD still remain to be further elucidated. Often overlooked in intestinal barrier research are the mucins. These heavily glycosylated proteins make up the first part of the barrier, the mucus layer, which is four times thicker than the actual epithelial cell layer and plays an important role in limiting contact between the host and the luminal content. MUC2 is the main component of the secreted mucus layer and provides the first line of defence against invading pathogens and toxins in the intestines. In IBD, this secretory mucin is critical for colonic protection since it has been shown that *Muc2*^{*-*/*-*} mice spontaneously develop colitis. The gradual increase in Muc2 expression seen during the course of colitis in the DSS model can thus be assigned to the host defence to overcome the toxic effects of DSS on the colonic epithelium. Furthermore, this mucin is downregulated in the intestinal mucosae of IBD patients.

Since transmembrane mucins are increasingly expressed in IBD and given their role in signalling pathways involved in cell-cell adhesion and cell differentiation, they are excellent candidates to be involved in the regulation of the barrier function. In our study, expression of the transmembrane Muc1 and Muc13 mucins was increased during colitis progression in both models, whereas Muc4 showed variable expression patterns in the inflamed colon. Variable MUC4 expression has also been reported in IBD patients and increased MUC4 expression was mainly observed in UC patients with neoplastic conditions. Altered expression of MUC1 and MUC13 has been shown in the inflamed mucosa of IBD patients and such inappropriate overexpression induced by pro-inflammatory cytokines could lead to aberrant modulation of mucosal epithelial cell inflammatory signalling, which in turn could lead to pathological inflammation. Furthermore, acute DSS studies with knockout animals showed that *Muc1*^{*-*/*-*} mice were resistant to inflammation-induced colitis whereas *Muc13^{-l-}* mice developed more inflammation compared to wildtype animals. In our DSS model, Muc13 expression was altered in both the acute and chronic phases of DSS-induced colitis. This increase in expression in the more chronic stage of colitis was also confirmed in the T cell transfer model. Unlike MUC1, MUC13 is highly expressed by the intestinal epithelium playing at first a protective role against cytotoxic agents. Furthermore, Sheng and colleagues (Sheng et al., 2012) demonstrated that MUC13 has a pro-inflammatory activity in the intestinal epithelium modulating inflammatory responses induced by TNF-α. Also, in our DSS models, increased TNF-α expression was significantly associated with altered *Muc13* expression, further suggesting that expression of this mucin is regulated by TNF-α upon inflammation and thus, the role of this mucin upon chronic colitis should be further investigated. In addition, we were able to correctly annotate individual mice to their experimental group (i.e. control or different time points of colitis) based on *Muc1* and *Muc13* expression (Figure 11). Interestingly, three main clusters could be distinguished in both colitis models. In particular, mice that were sacrificed during the initial stages of colitis (after 1 cycle of DSS administration and after 1 week of T cell transfer) were separated from both the control mice and the other experimental groups. Mice that were sacrificed at later time points could clearly be distinguished from control mice yet were more closely associated. These results further indicate the importance of Muc1 and Muc13 during the course of colitis.

To the best of our knowledge, a clear association between increased expression of transmembrane mucins and barrier dysfunction in IBD, has so far never been reported. Here, we found a positive correlation between increased *Muc1* and *Muc13* expression and increased *in vivo* intestinal barrier permeability during colitis progression, which was further substantiated by a strong correlation between expression of these mucins and altered expression of barrier mediators, including junctional and polarity proteins. Also observed was a model-specific response for both mucins, which could be explained by the different mechanisms of colitis induction. Whereas colitis in the T cell transfer model is induced by disrupting systemic T cell homeostasis, DSS is toxic to the intestinal epithelium leading to the penetration of luminal bacteria and antigens through the intestinal barrier resulting in a strong innate inflammatory response. Since MUC13 is highly expressed at the healthy intestinal epithelium, its role in modulating the integrity of the intestinal barrier could be related to immediate threats from the external environment. MUC1, on the other hand, is expressed at low levels in the healthy intestine and thus its involvement in barrier dysfunction could be dependent on the infiltration of T lymphocytes upon an inflammatory stimulus. Another possibility is that subtle differences in cytokine secretion could induce specific changes in mucin expression in both models. Although similar cytokine profiles were associated with disease activity in both models, IL-1β was correlated to increased *Muc1* expression and *in vivo* intestinal permeability in the T cell transfer model and TNF-α to increased *Muc13* expression and *in vivo* intestinal permeability in the DSS-induced colitis model. Nevertheless, based on the above findings, we can conclude that aberrantly expressed Muc1 and Muc13 could play a role in modulating intestinal barrier dysfunction during the course of colitis.

Overexpression of transmembrane mucins can result in a repositioning over the whole cell membrane, causing physical hindrance of neighbouring cells to make cell contact⁶. In our control animals, Muc1 and Muc13 were expressed at the apical side of the epithelial membrane, whereas they became generally visible throughout the cell during colitis progression. Transmembrane mucins can affect cell-cell interactions, and thus barrier functionality, in multiple ways. First, via extracellular EGF-like domains and intracellular phosphorylation sites, they can interact with receptor tyrosine kinases, such as ERBB2.

Activation of this membrane-bound receptor can then result in a disruption of the PAR polarity complex and subsequent tight junction dysfunction by associating with Par6 and aPKC and blocking the interaction with Par3. In our colitis models, a correlation between increased *Muc1* expression and decreased *Par3* expression was found suggesting that loss of barrier integrity mediated by Muc1 might be caused by sequestering with ERBB2 and subsequent dissociation of the PAR complex. Interaction of MUC1, but also MUC4 and MUC13, with ERBB2 has been described in many cancer types and the role of ERBB2 in barrier functionality in IBD remains to be further investigated. Second, the cytoplasmic domain of transmembrane mucins can be transported into the nucleus and suppress transcription of crumbs and scribble polarity genes, via interaction with a transcription factor on the promoter of these polarity genes. In this way, loss of cell polarity and tight junction dysfunction can be induced as well. Here, we found a correlation between the expression levels of *Muc13, Crb3 and Scrib* in the DSS model, highlighting that these mucins could probably also act according to the mechanism described above. Additionally, it has also been described that MUC1 can intracellularly interact with β-catenin, which results in the disruption of the E-cadherin/β-catenin complex and eventually leads to loss of adherens junction stability. In our colitis models, however, increased *Muc1* and *Muc13* expression was not associated with altered *Cdh1* (E-cadherin) expression.

Taken together, the results from our study clearly show the association of aberrant Muc1 and Muc13 expression with intestinal mucosal barrier dysfunction during the course of colitis. A model-specific response was observed, indicating a complex transcriptional regulation of mucin expression that results from the combined effects of the host inflammatory response, the microbiome and the type and course of disease. Nevertheless, the exact mechanisms by which these mucins affect barrier integrity and to prove their functional role in barrier integrity in IBD require further investigation.

Most available therapies in IBD are directed against the inflammatory response. Due to the clinical heterogeneity of these diseases, biologicals are limited in efficacy and safety and still a substantial number of patients fail to respond or obtain full remission. Targeting the barrier, and particularly MUC1 and MUC13, could also have therapeutic potential. These transmembrane mucins have already shown their potential in antibody-based therapy in different cancer types, including colon cancer, making them valuable therapeutic targets in medicine. Furthermore, mucins are highly polymorphic and gene polymorphisms affecting mucin expression have been reported to influence susceptibility towards disease. The presence of genetic differences in mucin genes can result in different mRNA isoforms (i.e. splice variants via alternative splicing) produced from the same mucin gene locus. While most isoforms encode similar biological functions, others have the potential to alter the protein function resulting in progression toward disease¹⁶. So far, only the MUC13-R502S polymorphism has been related to UC and the MUC1-rs3180018 to CD but the MUC1 and MUC13 isoforms associated with IBD remain unknown as well. Inhibiting inflammation-induced MUC1 and MUC13 isoforms to restore intestinal barrier integrity may thus achieve greater efficacy with fewer side effects.

Overall, it is highlighted here that aberrantly expressed Muc1 and Muc13 might be involved in intestinal mucosal barrier dysfunction upon inflammation by affecting tight junction and cell polarity proteins and that they can act as possible targets for novel therapeutic interventions.

### EXAMPLE 2: Targeted PacBio Isoform Sequencing to Analyze Isoform Expression of MUC1 and MUC13 in Colonic Biopsies From IBD Patients

### 1. Background to the invention

Here, we analyzed the expression of MUC1 and MUC13 isoforms in inflamed and non-inflamed colonic tissue from patients with active IBD to improve our understanding of mucin signaling during chronic inflammation.

### 2. Methods

### 2.1. IBD patients and clinical specimens

IBD patients that underwent an endoscopy for clinical reasons (i.e. the presence of an acute flare), were recruited via the policlinic of the University Hospital of Antwerp (UZA), Belgium. Colonic biopsies were collected from 3 patients with active disease (1 Crohn's disease, 2 ulcerative colitis) and stored in RNA later at -80°C until further use. All patients were previously diagnosed with IBD based on bowel complaints, blood and stool tests, radiography, endoscopy and histology. Disease activity was mainly based on the presence of active symptoms and endoscopic and microscopic evaluation of the colon. Prior to endoscopy, informed consent from each patient was obtained. This study was approved by the Ethical Committee of the UZA (Belgian Registration number B300201733423).

### 2.2. RNA isolation and quality control

Total RNA from human colonic tissue stored in RNA later, was extracted using the NucleoSpin^{®} RNA plus kit (Macherey-Nagel) following the manufacturer's instructions. The concentration and purity of the RNA were evaluated using the NanoDrop^{®} ND-1000 UV-Vis Spectrophotometer (Thermo Fisher Scientific) and Qubit Fluorometer (Qubit Broad Range RNA kit, Thermo Fisher Scientific). Quality control of the RNA was performed by capillary electrophoresis using an Agilent 2100 Fragment Analyzer (Agilent).

### 2.3. cDNA library preparation and multiplexing

Initially, 1600 - 2000 ng of input RNA per sample was used. The reactions from each sample were first labeled with a barcoded oligo dT nucleotide for multiplexing purposes as shown in Table 1. Subsequently, first-strand cDNA synthesis was performed using the SMARTer PCR cDNA synthesis kit (Takara Bio) according to the manufacturer's instructions. The reactions were then diluted 1:10 in Elution Buffer (PacBio) and large-scale amplification was performed using 16 reactions per sample. Each reaction of 50 µL consisted of 10 µL of the diluted cDNA sample, 10 µL 5X PrimeSTAR GXL buffer (Takara Bio), 4 µL dNTP Mix (2.5 mM each), 1 µL 5' PCR Primer IIA (12 µM), 1 µL PrimeSTAR GXL DNA Polymerase (1.25 U/µL, Takara Bio) and 24 µL nuclease-free water. The samples were then incubated in a thermocyler using the following program: an initial denaturation step at 98°C for 30s, followed by 14 cycles of amplification at 98°C for 10s, 65°C for 15s and 68°C for 10 min, and a final extension step at 68°C for 5 min. From these PCR products, two fractions were purified using AMPure magnetic purification beads. After equimolar pooling of both fractions, the samples were finally pooled and the DNA concentration and fragment length evaluated using a Qubit fluorometer (Qubit dsDNA HS kit, ThermoFisher) and an Agilent 2100 Bioanalyzer.

**Table 1. Barcoded primers used for multiplexing purposes.**

| **Sample** | **Barcode** | **SEQ ID N** | **Sequence** |
|---|---|---|---|
| P1 colon non-inflamed | dT_BC1001_PB | 61 | AAGCAGTGGTATCAACGCAGAGTACCACATATCAG AGTGCGTTTTTTTTTTTTTTTTTTTTTTTTTTTTTTVN |
| P1 colon inflamed | dT_BC1002_PB | 62 | AAGCAGTGGTATCAACGCAGAGTACACACACAGAC TGTGAGTTTTTTTTTTTTTTTTTTTTTTTTTTTTTTVN |
| P2 colon non-inflamed | dT_BC1003_PB | 63 | AAGCAGTGGTATCAACGCAGAGTACACACATCTCG TGAGAGTTTTTTTTTTTTTTTTTTTTTTTTTTTTTTVN |
| P2 colon inflamed | dT_BC1004_PB | 64 | AAGCAGTGGTATCAACGCAGAGTACCACGCACACA CGCGCGTTTTTTTTTTTTTTTTTTTTTTTTTTTTTTVN |
| P3 colon non-inflamed | dT_BC1005_PB | 65 | AAGCAGTGGTATCAACGCAGAGTACCACTCGACTC TCGCGTTTTTTTTTTTTTTTTTTTTTTTTTTTTTTTTVN |
| P3 colon inflamed | dT_BC1006_PB | 66 | AAGCAGTGGTATCAACGCAGAGTACCATATATATCA GCTGTTTTTTTTTTTTTTTTTTTTTTTTTTTTTTVN |

| | | | |
|---|---|---|---|
| *In accordance with the IUPAC nucleotide code, N is meant to be any base (A, G, T or C) and V is meant to be A, C or G. | | | |

### 2.4. cDNA capture using SeqCap EZ probes

Initially, 1 µL of SMARTer PCR oligo (1000 µM) and 1 µL PolyT blocker (1000 µM) were added to 1.5µg cDNA and subsequently dried for 1 hour in a DNA vacuum-concentrator. The cDNA was then hybridized with pre-designed SeqCap EZ probes targeting several mucin coding regions (Table 2 & 3) for 16 hours at 47°C. The captured cDNA was purified using Dynabeads M-270 (Thermo Fisher Scientific) according to the manufacturer's instructions and amplified by preparing a mixture containing 20 µl 10X LA PCR Buffer, 16 µl 2.5 mM dNTP's, 8.3 SMARTer PCR Oligos (12 µM each), 1.2 µl Takara LA Taq DNA polymerase, 50 µl cDNA supplemented with nuclease-free water to an end volume of 200 µl. For the actual PCR, the following program was ran on a thermocycler: an initial denaturation step at 95°C for 2 min, followed by 11 cycles of amplification at 95°C for 20s and 68°C for 10 min, and a final extension step at 72°C for 10 min. A final clean-up of the amplified captured cDNA was performed using AMPure purification beads. The DNA concentration and fragment length were evaluated using a Qubit fluorometer (Qubit dsDNA HS kit, ThermoFisher) and an Agilent 2100 Bioanalyzer for subsequent SMRTbell library construction.

**Table 2. Genomic regions targeted with SeqCap EZ probes.**

| **Mucin** | **Chromosome** | **Chromosomal location (GRCh38/hg38 genome annotation)** |
|---|---|---|
| | | |
| MUC1 | Chr 1 | 155,185,324 - 155,193,416 |
| MUC2 | Chr11 | 1,074,375 - 1,111,008 |
| MUC3 - MUC12 - MUC17 | Chr7 | 100,944,420 - 101,074,859 |
| MUC4 | Chr3 | 195,746,558 - 195,826,889 |
| MUC5AC - MUC5B | Chr11 | 1,146,953 - 1,272,672 |
| MUC6 | Chr11 | 1,012,323 - 1,037,218 |
| MUC13 | Chr3 | 124,905,442 - 124,940,751 |
| MUC15 | Chr11 | 26,558,532 - 26,572,763 |
| MUC16 | Chr19 | 8,848,344 - 9,001,342 |
| MUC20 | Chr3 | 195,720,384 - 195,738,123 |

**Table 3. SeqCap EZ probe coverage of targeted mucin regions.**

| | **Probe coverage** | **Estimated coverage** |
|---|---|---|
| Target Bases Covered | 493.161 (78.7 %) | 561.699 (89.7 %) |
| Target Bases Not Covered | 133.225 (21.3 %) | 64.687 (10.3 %) |

### 2.5. SMRTbell library construction and sequencing on the PacBio Sequel system

Using the SMRTbell template prep kit (PacBio), 5 µg of captured cDNA was used for SMRTbell library construction. According to the manufacturer's instructions, the following steps were performed in chronological order: DNA damage repair, end repair, ligation of blunt adapters, Exo III and Exo VII treatment. One intermediate and two final purification steps were performed using AMPure purification beads. The DNA concentration and fragment length were evaluated using a Qubit fluorometer (Qubit dsDNA HS kit, ThermoFisher) and an Agilent 2100 Bioanalyzer for subsequent SMRTbell library construction. Following the instructions on SMRTlink, the Sequel Binding kit (PacBio) and Sequel Sequencing kit (PacBio) were used to dilute the DNA and internal control complexes, anneal the sequencing primer and bind the sequencing polymerase to the SMRTbell templates. Finally, the sample was loaded on a 1M v3 SMRT cell.

### 2.6. Data analysis

Highly accurate (> 99%) polished circular consensus sequencing (ccs) reads were used as initial input for data processing using the command line interface. The lima tool v1.10.0 was used for demultiplexing and primer removal. Subsequently, the isoseq3 v3.2.2 package was used for further read processing to generate high quality mRNA transcripts. First, the refine tool was used for trimming of Poly(A) tails and identification and removal of concatemers. The data of the individual samples were then pooled together according to the condition (i.e. 3 samples from non-inflamed tissue, 3 samples from inflamed tissue or all samples together) and analyzed in parallel. The isoseq3 cluster algorithm was used for transcript clustering. Minimap2 was used for the alignment of the processed reads to the human reference genome (GRCh38). After mapping, ToFU scripts from the cDNA_Cupcake GitHub repository were used to collapse redundant isoforms (minimal alignment coverage and minimal alignment identity set at 0.95), identify associated count information and filter away 5' degraded isoforms. Finally, the SQANTI2 tool was used for extensive characterization of MUC1 and MUC13 mRNA isoforms. The eventual isoforms were then further inspected by visualization in the Integrative Genomics Viewer (IGV) version 2.8.0 and by the analysis of the classification and junction files in Excel.

### 3. Results

### 3.1. Patient and sample characteristics

The samples were collected from the colon of 3 patients with known and active IBD, of which two were diagnosed with ulcerative colitis and one with Crohn's disease. Year of diagnosis and medication use was different for all patients. During endoscopy, the samples were collected from a macroscopically inflamed region in the colon and from an adjacent macroscopically non-inflamed region. A detailed overview of the patient characteristics as well as the location of the colon biopsies is shown in table 4.

**Table 4. Summary of patient characteristics and primary disease location from which biopsies were collected.**

| **Patient** | **Sex** | **Age** | **Diagnosis** | **Years since diagnosis** | **Primary medication use** | **Primary disease location** |
|---|---|---|---|---|---|---|
| Patient 1 | Female | 34 | Crohn's disease | 20 | Remicade | Rectum |
| Patient 2 | Female | 36 | Ulcerative colitis | 10 | No | Rectum / anus |
| Patient 3 | Female | 45 | Ulcerative colitis | 3 | Mesalamine | Sigmoid and descending colon |

### 3.2. General features of sequencing run

Sequencing of all samples initially generated 103 699 ccs reads. Sequencing yield and read quality was high and comparable across all samples. The average read length was 2082 bp. 24592 (24%) reads were lost during primer removal and demultiplexing as a consequence of undesired barcoded primer combinations. After clustering, 55312 reads were remained corresponding to 6617 different transcripts. As visual analysis of targeted mucin regions in IGV showed complete and dense coverage of the full genomic region of only *MUC1* and *MUC13,* further analysis was limited to these two mucin glycoproteins.

### 3.3. MUC1 isoforms

Targeted PacBio isoform sequencing revealed the identification of both known and novel MUC1 isoforms in colonic tissue from IBD patients that were all found to be coding transcripts (Figure 14 & Table 5). In particular, 7 alternative mRNA transcripts (= isoforms) were found in both non-inflamed and inflamed colonic tissue, of which 1 (PB.136.39) matched to a known isoform (ENST00000462317.5) and 6 had not been described elsewhere. Interestingly, from these alternative transcripts, 3 were increased in expression based on the read counts in the inflamed tissue as compared to the non-inflamed tissue (PB.136.1, PB.136.25, PB.136.28). Additionally, 2 other novel isoforms were found which were only reported in non-inflamed colonic tissue, whereas in the inflamed colonic tissue, 1 known (PB.136.19; ENST00000368390.7) and 11 novel alternative transcripts were found. Interestingly, 2 newly identified isoforms showed dominant expression in the inflamed tissue (PB.136.2, PB.136.15). Concerning the overall exonic structure of the alternative transcripts, no transcripts were found that contained exon 3 to 5 (VNTR). Exon 2 (VNTR) and exon 6 (SEA domain) were most prone to alternative splicing in both non-inflamed and inflamed colonic tissue (Figure 14 & Table 5). All novel alternative transcripts found resulted from the partial retention of intronic regions (Table 5). A detailed overview of splice junctions can be found in supplementary table S2.

The results of these limited number of samples clearly shows that different alternative transcripts of *MUC1* are formed in the colon and that inflammation stimulates alternative splicing as well as increasing the expression of particular transcripts. This is the first study that highlights the potential importance of *MUC1* isoforms in IBD. Only in cancer research, a few papers investigating the pathogenic significance of *MUC1* splice variants are available. More specifically, it has been shown that different *MUC1* isoforms might interact together to form a ligand-receptor complex, associate with other host receptors or influence cytokine expression mediating inflammatory signaling pathways (Zaretsky et al., 2006). Alternative splicing of *MUC1* isoforms was also shown to be cancer-type dependent and able to distinguish cancer samples from benign samples (Obermair et al., 2002). In breast cancer, for instance, it has been described that a shorter *MUC1* isoform was specifically expressed in tumor tissue but not in the adjacent healthy tissue (Zrihan-Licht et al., 1994), whereas estrogen treatment induced the expression of another variant (Zartesky et al., 2006). All this highlight the intriguing complexity and biological role of alternative splicing.

**Tabel 5: Detailed overview of characteristics of MUC1 mRNA isoforms in colonic biopsies from IBD patients**

| **Both conditions** | | | | | | | |
|---|---|---|---|---|---|---|---|
| Isoform ID | Chrom | Length (bp) | Exons | Coding | Transcript | Main mechanism of alternative splicing | Counts |
| PB.136.1 | chr1 | 1712 | 8 | Coding | Novel | Intron retention | NI: 3 I: 11 |
| PB.136.23 | chr1 | 1257 | 7 | Coding | Novel | Intron retention | NI: 8 I: 9 |
| PB.136.26 | chr1 | 1619 | 8 | Coding | Novel | Intron retention | NI: 2 I: 5 |
| PB.136.28 | chr1 | 1551 | 8 | Coding | Novel | Intron retention | NI: 8 I: 21 |
| PB.136.25 | chr1 | 2306 | 6 | Coding | Novel | Intron retention | NI: 5 I: 15 |
| PB. 136.39 | chr1 | 1377 | 8 | Coding | ENST00000462317.5 | Multi-exon | NI: 3 I: 3 |
| PB.136.5 | chr1 | 1090 | 6 | Coding | Novel | Intron retention | NI: 2 I: 3 |

| **Non-inflamed** | | | | | | | |
|---|---|---|---|---|---|---|---|
| Isoform ID | Chrom | Length (bp) | Exons | Coding | Transcript | subcategory | Counts |
| PB.136.9 | chr1 | 1497 | 8 | Coding | Novel | Intron retention | 7 |
| PB.136.22 | chr1 | 1493 | 8 | Coding | Novel | Intron retention | 2 |

| **Inflamed** | | | | | | | |
|---|---|---|---|---|---|---|---|
| Isoform ID | Chrom | Length (bp) | Exons | Coding | Transcript | subcategory | Counts |
| PB.136.2 | chr1 | 1652 | 8 | Coding | Novel | Intron retention | 30 |
| PB.136.4 | chr1 | 1470 | 8 | Coding | Novel | Intron retention | 2 |
| PB.136.18 | chr1 | 1233 | 8 | Coding | Novel | Intron retention | 2 |
| PB.136.15 | chr1 | 1526 | 8 | Coding | Novel | Intron retention | 24 |
| PB.136.14 | chr1 | 1564 | 8 | Coding | Novel | Intron retention | 3 |

| **Both conditions** | | | | | | | |
|---|---|---|---|---|---|---|---|
| PB.136.19 | chr1 | 1141 | 8 | Coding | ENST00000368390.7 | Multi-exon | 3 |
| PB.136.21 | chr1 | 1590 | 8 | Coding | Novel | Intron retention | 3 |
| PB.136.29 | chr1 | 1493 | 8 | Coding | Novel | Intron retention | 2 |
| PB.136.37 | chr1 | 1640 | 8 | Coding | Novel | Intron retention | 2 |
| PB.136.38 | chr1 | 1583 | 8 | Coding | Novel | Intron retention | 5 |
| PB.136.6 | chr1 | 1055 | 6 | Coding | Novel | Intron retention | 3 |
| PB.136.24 | chr1 | 1088 | 7 | Coding | Novel | Intron retention | 2 |

### 3.4. MUC13 isoforms

Twenty-one alternative MUC13 mRNA transcripts were found in colonic tissue from IBD patients (Figure 15 & Table 6). Of these, 17 transcripts were identified as being coding isoforms and 4 as non-coding splice variants. Such long untranslated mucin isoforms can function similar to long noncoding RNA and act as a scaffold for assembly of multimeric protein complexes involved in the regulation of cellular processes. Importantly, the full-length known isoform (ENST00000616727.4) was present in both conditions but was highly upregulated in the inflamed colonic tissue (Table 6). In both conditions, 3 additional isoforms were found that had not been reported previously. Other isoforms showed a condition-specific expression pattern. More specifically, 4 mRNA isoforms were uniquely found in the non-inflamed tissue, whereas 13 mRNA isoforms were only reported in the inflamed colonic tissue. Several mechanisms of alternative splicing were identified concerning *MUC13* isoforms. Exon skipping was observed in two alternative transcripts in the inflamed colon (i.e. exon 9 (EGF-like) and 10 (TMD) in PB.1087.32 ; exon 9 (EGF-like), 10 (TMD) and 11 (CT) in PB.1087.20). Some mono-exonic transcripts were found that resulted from intron retention in the genomic region coding for the ECD (i.e. PB.1087.50, PB.1087.53, PB.1087.58, PB.1087.61). The other isoforms resulted from more subtle recombinations using both known and novel splice sites mainly in the ECD-coding regions of *MUC13* (Figure 15 & Table 6). A detailed overview of all splice junctions can be found in Supplementary table S3.

To our knowledge, the heterogeneity of MUC13 isoform expression during inflammation and cancer has not been studied in much detail before. Here, evidence is provided that MUC13 is alternatively spliced in both non-inflamed and inflamed colonic tissue from IBD patients.

**Table 6: Detailed overview of characteristics of MUC13 mRNA isoforms in colonic biopsies from IBD patients**

| Isoform ID | Chrom | Strand | Length (bp) | Exons | Coding | Transcript | Main mechanism of alternative splicing | Counts |
|---|---|---|---|---|---|---|---|---|
| PB.1087.17 | chr3 | - | 2878 | 12 | Coding | ENST0000 0616727.4 | Constitutive | NI: 518 I: 936 |
| PB.1087.22 | chr3 | - | 2830 | 13 | Coding | Novel | At least one novel splicesite | NI: 3 I: 7 |
| PB.1087.30 | chr3 | - | 2859 | 12 | Coding | Novel | At least one novel splicesite | NI: 2 I: 2 |
| PB.1087.55 | chr3 | - | 5414 | 3 | Coding | Novel | Intron retention | NI: 2 I: 4 |

| **Non-infalmed** | | | | | | | | |
|---|---|---|---|---|---|---|---|---|
| Isoform ID | Chrom | Strand | Length (bp) | Exons | Coding | Transcript | Main mechanism of alternative splicing | Counts |
| PB.1087.18 | chr3 | - | 2725 | 13 | Coding | Novel | At least one novel splicesite | 2 |
| PB.1087.50 | chr3 | - | 5304 | 1 | Non-coding | Novel | Mono-exon / intron retention | 2 |
| PB.1087.61 | chr3 | - | 5106 | 1 | Coding | Novel | Mono-exon / intron retention | 2 |
| PB.1087.64 | chr3 | - | 3860 | 2 | Coding | Novel | At least one novel splicesite | 3 |

| **Inflamed** | | | | | | | | |
|---|---|---|---|---|---|---|---|---|
| Isoform ID | Chrom | Strand | Length (bp) | Exons | Coding | Transcript | Main mechanism of alternative splicing | Counts |
| PB.1087.6 | chr3 | - | 2243 | 10 | Coding | Novel | At least one novel splicesite | 2 |
| PB.1087.63 | chr3 | - | 3962 | 2 | Coding | Novel | At least one novel splicesite | 2 |
| PB.1087.21 | chr3 | - | 3195 | 13 | Coding | Novel | At least one novel splicesite | 2 |
| PB.1087.20 | chr3 | - | 1979 | 9 | Coding | Novel | At least one novel splicesite | 2 |
| PB.1087.25 | chr3 | - | 2671 | 11 | Coding | Novel | At least one novel splicesite | 2 |
| PB.1087.68 | chr3 | - | 2643 | 2 | Coding | Novel | At least one novel splicesite | 2 |
| PB.1087.32 | chr3 | - | 2754 | 10 | Coding | Novel | Novel combination of known splicesites | 8 |
| PB.1087.27 | chr3 | - | 2328 | 12 | Coding | Novel | At least one novel splicesite | 2 |
| PB.1087.31 | chr3 | - | 2795 | 13 | Coding | Novel | At least one novel splicesite | 2 |
| PB.1087.52 | chr3 | - | 3622 | 4 | Coding | Novel | Intron retention | 2 |
| PB.1087.53 | chr3 | - | 2303 | 1 | Non-coding | Novel | Mono-exon / intron retention | 2 |
| PB.1087.58 | chr3 | - | 2362 | 1 | Non-coding | Novel | Mono-exon / intron retention | 4 |
| PB.1087.56 | chr3 | - | 5246 | 2 | Coding | Novel | Intron retention | 3 |

### 4. Concluding remarks

Based on the PacBio isoform sequencing data gathered from a limited number of samples, we were able to identify both known and novel mRNA isoforms of *MUC1* and *MUC13* in non-inflamed and inflamed colonic tissue from IBD patients. Alternative splicing of *MUC1* and *MUC13* mucin genes was clearly increased upon inflammation. Although some isoforms were found in both inflamed and non-inflamed tissue, several other isoforms were uniquely attributed to inflammation.

In conclusion, mucin isoform expression is altered upon inflammation in IBD patients, highlighting its potential for disease surveillance or treatment. Moreover, these novel insights could be extrapolated to other inflammatory diseases and cancer that involve a dysfunctional mucosal epithelial barrier. The unexplored world of mucin isoforms provides thus a unique opportunity to understand their biological significance, utility as biomarker and pathology-specific targeting.

**Supplementary table S2: Detailed overview of splice junctions of MUC1 alternative mRNA transcripts**

| **Both conditions** | | | | | | | | |
|---|---|---|---|---|---|---|---|---|
| isoform | Chrom. | strand | junction_number | genomic_start_coord | genomic_end_coord | junction_category | splice_site | canonical |
| PB.136.1 | chr1 | - | junction_1 | 155186210 | 155187224 | known | GTAG | canonical |
| PB.136.1 | chr1 | - | junction_2 | 155187375 | 155187454 | known | GTAG | canonical |
| PB.136.1 | chr1 | - | junction_3 | 155187577 | 155187721 | known | GTAG | canonical |
| PB.136.1 | chr1 | - | junction_4 | 155187859 | 155188007 | known | GTAG | canonical |
| PB.136.1 | chr1 | - | junction_5 | 155188064 | 155188162 | known | GTAG | canonical |
| PB.136.1 | chr1 | - | junction_6 | 155188528 | 155191938 | novel | CCAG | non_canonical |
| PB.136.1 | chr1 | - | junction_7 | 155192311 | 155192785 | known | GTAG | canonical |
| PB.136.23 | chr1 | - | junction_1 | 155186210 | 155187224 | known | GTAG | canonical |
| PB.136.23 | chr1 | - | junction_2 | 155187375 | 155187454 | known | GTAG | canonical |
| PB.136.23 | chr1 | - | junction_3 | 155187577 | 155187721 | known | GTAG | canonical |
| PB.136.23 | chr1 | - | junction_4 | 155187859 | 155188007 | known | GTAG | canonical |
| PB.136.23 | chr1 | - | junction_5 | 155188064 | 155188162 | known | GTAG | canonical |
| PB.136.23 | chr1 | - | junction_6 | 155188452 | 155192787 | novel | AGAG | non_canonical |
| PB.136.26 | chr1 | - | junction_2 | 155187375 | 155187454 | known | GTAG | canonical |
| PB.136.26 | chr1 | - | junction_3 | 155187577 | 155187721 | known | GTAG | canonical |
| PB.136.26 | chr1 | - | junction_4 | 155187859 | 155188007 | known | GTAG | canonical |
| PB.136.26 | chr1 | - | junction_5 | 155188064 | 155188162 | known | GTAG | canonical |
| PB.136.26 | chr1 | - | junction_6 | 155188538 | 155192008 | novel | ACCC | non_canonical |
| PB.136.26 | chr1 | - | junction_7 | 155192284 | 155192785 | known | GTAG | canonical |
| PB.136.28 | chr1 | - | junction_1 | 155186210 | 155187224 | known | GTAG | canonical |
| PB.136.28 | chr1 | - | junction_2 | 155187375 | 155187454 | known | GTAG | canonical |
| PB.136.28 | chr1 | - | junction_3 | 155187577 | 155187721 | known | GTAG | canonical |
| PB.136.28 | chr1 | - | junction_4 | 155187859 | 155188007 | known | GTAG | canonical |
| PB.136.28 | chr1 | - | junction_5 | 155188064 | 155188162 | known | GTAG | canonical |
| PB.136.28 | chr1 | - | junction_6 | 155188452 | 155192017 | novel | GGAG | non_canonical |
| PB.136.28 | chr1 | - | junction_7 | 155192311 | 155192785 | known | GTAG | canonical |
| PB.136.25 | chr1 | - | junction_1 | 155187375 | 155187454 | known | GTAG | canonical |
| PB.136.25 | chr1 | - | junction_2 | 155187577 | 155187721 | known | GTAG | canonical |
| PB.136.25 | chr1 | - | junction_3 | 155187859 | 155188007 | known | GTAG | canonical |
| PB.136.25 | chr1 | - | junction_4 | 155188064 | 155188162 | known | GTAG | canonical |
| PB.136.25 | chr1 | - | junction_5 | 155188557 | 155191967 | novel | GCAG | canonical |
| PB.136.39 | chr1 | - | junction_1 | 155186210 | 155186729 | known | GTAG | canonical |
| PB.136.39 | chr1 | - | junction_2 | 155186805 | 155187224 | known | GTAG | canonical |
| PB.136.39 | chr1 | - | junction_3 | 155187375 | 155187454 | known | GTAG | canonical |
| PB.136.39 | chr1 | - | junction_4 | 155187577 | 155187721 | known | GTAG | canonical |
| PB.136.39 | chr1 | - | junction_5 | 155187859 | 155188007 | known | GTAG | canonical |
| PB.136.39 | chr1 | - | junction_6 | 155188064 | 155188162 | known | GTAG | canonical |
| PB.136.39 | chr1 | - | junction_7 | 155188541 | 155192863 | novel | CCCC | non_canonical |
| PB.136.5 | chr1 | - | junction_1 | 155186210 | 155187224 | known | GTAG | canonical |
| PB.136.5 | chr1 | - | junction_2 | 155187375 | 155187454 | known | GTAG | canonical |
| PB.136.5 | chr1 | - | junction_3 | 155187545 | 155187721 | known | GTAG | canonical |
| PB.136.5 | chr1 | - | junction_4 | 155187859 | 155188007 | known | GTAG | canonical |
| PB.136.5 | chr1 | - | junction_5 | 155188064 | 155188162 | known | GTAG | canonical |

| **Non-inflamed** | | | | | | | | |
|---|---|---|---|---|---|---|---|---|
| isoform | Chrom. | strand | junction_number | genomic_start_coord | genomic_end_coord | junction_category | splice_site | canonical |
| PB.136.9 | chr1 | - | junction_1 | 155186210 | 155187224 | known | GTAG | canonical |
| PB.136.9 | chr1 | - | junction_2 | 155187375 | 155187454 | known | GTAG | canonical |
| PB.136.9 | chr1 | - | junction_3 | 155187577 | 155187721 | known | GTAG | canonical |
| PB.136.9 | chr1 | - | junction_4 | 155187859 | 155188007 | known | GTAG | canonical |
| PB.136.9 | chr1 | - | junction_5 | 155188064 | 155188162 | known | GTAG | canonical |
| PB.136.9 | chr1 | - | junction_6 | 155188533 | 155192128 | novel | ACCC | non_canonical |
| PB.136.9 | chr1 | - | junction_7 | 155192284 | 155192785 | known | GTAG | canonical |
| PB.136.22 | chr1 | - | junction_1 | 155186210 | 155187224 | known | GTAG | canonical |
| PB.136.22 | chr1 | - | junction_2 | 155187375 | 155187454 | known | GTAG | canonical |
| PB.136.22 | chr1 | - | junction_3 | 155187577 | 155187721 | known | GTAG | canonical |
| PB.136.22 | chr1 | - | junction_4 | 155187859 | 155188007 | known | GTAG | canonical |
| PB.136.22 | chr1 | - | junction_5 | 155188064 | 155188162 | known | GTAG | canonical |
| PB.136.22 | chr1 | - | junction_6 | 155188467 | 155192028 | novel | GGAA | non_canonical |
| PB.136.22 | chr1 | - | junction_7 | 155192248 | 155192785 | novel | GTAG | canonical |

| **Inflamed** | | | | | | | | |
|---|---|---|---|---|---|---|---|---|
| isoform | Chrom. | strand | junction_number | genomic_start_coord | genomic_end_coord | junction_category | splice_site | canonical |
| PB.136.2 | chr1 | - | junction_1 | 155186210 | 155187224 | known | GTAG | canonical |
| PB.136.2 | chr1 | - | junction_2 | 155187375 | 155187454 | known | GTAG | canonical |
| PB.136.2 | chr1 | - | junction_3 | 155187577 | 155187721 | known | GTAG | canonical |
| PB.136.2 | chr1 | - | junction_4 | 155187859 | 155188007 | known | GTAG | canonical |
| PB.136.2 | chr1 | - | junction_5 | 155188064 | 155188162 | known | GTAG | canonical |
| PB.136.2 | chr1 | - | junction_6 | 155188538 | 155192008 | novel | ACCC | non_canonical |
| PB.136.2 | chr1 | - | junction_7 | 155192311 | 155192785 | known | GTAG | canonical |
| PB.136.4 | chr1 | - | junction_1 | 155186210 | 155187224 | known | GTAG | canonical |
| PB.136.4 | chr1 | - | junction_2 | 155187375 | 155187454 | known | GTAG | canonical |
| PB.136.4 | chr1 | - | junction_3 | 155187577 | 155187721 | known | GTAG | canonical |
| PB.136.4 | chr1 | - | junction_4 | 155187859 | 155188007 | known | GTAG | canonical |
| PB.136.4 | chr1 | - | junction_5 | 155188064 | 155188162 | known | GTAG | canonical |
| PB.136.4 | chr1 | - | junction_6 | 155188528 | 155192153 | novel | CCAG | non_canonical |
| PB.136.4 | chr1 | - | junction_7 | 155192284 | 155192785 | known | GTAG | canonical |
| PB.136.18 | chr1 | - | junction_1 | 155186210 | 155187224 | known | GTAG | canonical |
| PB.136.18 | chr1 | - | junction_2 | 155187375 | 155187454 | known | GTAG | canonical |
| PB.136.18 | chr1 | - | junction_3 | 155187577 | 155187721 | known | GTAG | canonical |
| PB.136.18 | chr1 | - | junction_4 | 155187859 | 155188007 | known | GTAG | canonical |
| PB.136.18 | chr1 | - | junction_5 | 155188064 | 155188162 | known | GTAG | canonical |
| PB.136.18 | chr1 | - | junction_6 | 155188375 | 155192244 | novel | GATG | non_canonical |
| PB.136.18 | chr1 | - | junction_7 | 155192284 | 155192785 | known | GTAG | canonical |
| PB.136.15 | chr1 | - | junction_1 | 155186210 | 155187224 | known | GTAG | canonical |
| PB.136.15 | chr1 | - | junction_2 | 155187375 | 155187454 | known | GTAG | canonical |
| PB.136.15 | chr1 | - | junction_3 | 155187577 | 155187721 | known | GTAG | canonical |
| PB.136.15 | chr1 | - | junction_4 | 155187859 | 155188007 | known | GTAG | canonical |
| PB.136.15 | chr1 | - | junction_5 | 155188064 | 155188162 | known | GTAG | canonical |
| PB.136.15 | chr1 | - | junction_6 | 155188452 | 155192017 | novel | GGAG | non_canonical |
| PB.136.15 | chr1 | - | junction_7 | 155192284 | 155192785 | known | GTAG | canonical |
| PB.136.14 | chr1 | - | junction_1 | 155186210 | 155187224 | known | GTAG | canonical |
| PB.136.14 | chr1 | - | junction_2 | 155187375 | 155187454 | known | GTAG | canonical |
| PB.136.14 | chr1 | - | junction_3 | 155187577 | 155187721 | known | GTAG | canonical |
| PB.136.14 | chr1 | - | junction_4 | 155187859 | 155188007 | known | GTAG | canonical |
| PB.136.14 | chr1 | - | junction_5 | 155188064 | 155188162 | known | GTAG | canonical |
| PB.136.14 | chr1 | - | junction_6 | 155188471 | 155192025 | novel | CAGC | non_canonical |
| PB.136.14 | chr1 | - | junction_7 | 155192311 | 155192785 | known | GTAG | canonical |
| PB.136.19 | chr1 | - | junction_1 | 155186210 | 155187224 | known | GTAG | canonical |
| PB.136.19 | chr1 | - | junction_2 | 155187375 | 155187454 | known | GTAG | canonical |
| PB.136.19 | chr1 | - | junction_3 | 155187577 | 155187721 | known | GTAG | canonical |
| PB.136.19 | chr1 | - | junction_4 | 155187859 | 155188007 | known | GTAG | canonical |
| PB.136.19 | chr1 | - | junction_5 | 155188064 | 155188162 | known | GTAG | canonical |
| PB.136.19 | chr1 | - | junction_6 | 155188232 | 155192182 | known | GTAG | canonical |
| PB.136.19 | chr1 | - | junction_7 | 155192284 | 155192785 | known | GTAG | canonical |
| PB.136.21 | chr1 | - | junction_1 | 155186210 | 155187224 | known | GTAG | canonical |
| PB.136.21 | chr1 | - | junction_2 | 155187375 | 155187454 | known | GTAG | canonical |
| PB.136.21 | chr1 | - | junction_3 | 155187577 | 155187721 | known | GTAG | canonical |
| PB.136.21 | chr1 | - | junction_4 | 155187859 | 155188007 | known | GTAG | canonical |
| PB.136.21 | chr1 | - | junction_5 | 155188064 | 155188162 | known | GTAG | canonical |
| PB.136 .21 | chr1 | - | junction_6 | 155188467 | 155191967 | novel | GCAA | non_canonical |
| PB.136.21 | chr1 | - | junction_7 | 155192284 | 155192785 | known | GTAG | canonical |
| PB.136.29 | chr1 | - | junction_1 | 155186210 | 155187224 | known | GTAG | canonical |
| PB.136.29 | chr1 | - | junction_2 | 155187375 | 155187454 | known | GTAG | canonical |
| PB.136.29 | chr1 | - | junction_3 | 155187577 | 155187721 | known | GTAG | canonical |
| PB.136.29 | chr1 | - | junction_4 | 155187859 | 155188007 | known | GTAG | canonical |
| PB.136.29 | chr1 | - | junction_5 | 155188064 | 155188162 | known | GTAG | canonical |
| PB.136.29 | chr1 | - | junction_6 | 155188528 | 155192153 | novel | CCAG | non_canonical |
| PB.136.29 | chr1 | - | junction_7 | 155192311 | 155192785 | known | GTAG | canonical |
| PB.136.37 | chr1 | - | junction_1 | 155186210 | 155187224 | known | GTAG | canonical |
| PB.136.37 | chr1 | - | junction_2 | 155187375 | 155187454 | known | GTAG | canonical |
| PB.136.37 | chr1 | - | junction_3 | 155187577 | 155187721 | known | GTAG | canonical |
| PB.136.37 | chr1 | - | junction_4 | 155187859 | 155188007 | known | GTAG | canonical |
| PB.136.37 | chr1 | - | junction_5 | 155188064 | 155188162 | known | GTAG | canonical |
| PB.136.37 | chr1 | - | junction_6 | 155188580 | 155191990 | novel | GTGT | non_canonical |
| PB.136.37 | chr1 | - | junction_7 | 155192248 | 155192785 | novel | GTAG | canonical |
| PB.136.38 | chr1 | - | junction_1 | 155186210 | 155187224 | known | GTAG | canonical |
| PB.136.38 | chr1 | - | junction_2 | 155187375 | 155187454 | known | GTAG | canonical |
| PB.136.38 | chr1 | - | junction_3 | 155187577 | 155187721 | known | GTAG | canonical |
| PB.136.38 | chr1 | - | junction_4 | 155187859 | 155188007 | known | GTAG | canonical |
| PB.136.38 | chr1 | - | junction_5 | 155188064 | 155188162 | known | GTAG | canonical |
| PB.136.38 | chr1 | - | junction_6 | 155188580 | 155192110 | novel | GTGT | non_canonical |
| PB.136.38 | chr1 | - | junction_7 | 155192311 | 155192785 | known | GTAG | canonical |
| PB.136.6 | chr1 | - | junction_1 | 155186210 | 155187224 | known | GTAG | canonical |
| PB.136.6 | chr1 | - | junction_2 | 155187375 | 155187454 | known | GTAG | canonical |
| PB.136.6 | chr1 | - | junction_3 | 155187577 | 155187721 | known | GTAG | canonical |
| PB.136.6 | chr1 | - | junction_4 | 155187804 | 155188007 | novel | GTAT | non_canonical |
| PB.136.6 | chr1 | - | junction_5 | 155188064 | 155188162 | known | GTAG | canonical |
| PB.136.24 | chr1 | - | junction_1 | 155185989 | 155186052 | novel | CTCC | non_canonical |
| PB.136.24 | chr1 | - | junction_2 | 155186210 | 155187224 | known | GTAG | canonical |
| PB.136.24 | chr1 | - | junction_3 | 155187375 | 155187454 | known | GTAG | canonical |
| PB.136.24 | chr1 | - | junction_4 | 155187577 | 155187721 | known | GTAG | canonical |
| PB.136.24 | chr1 | - | junction_5 | 155187859 | 155188007 | known | GTAG | canonical |
| PB.136.24 | chr1 | - | junction_6 | 155188064 | 155188162 | known | GTAG | canonical |

**Supplementary table S3: Detailed overview of splice junctions of MUC13 alternative mRNA transcripts**

| **Both conditions** | | | | | | | | |
|---|---|---|---|---|---|---|---|---|
| Isoform | chromosome | strand | junction_number | genomic_start_coord | genomic_end_coord | junction_category | splice_site | Canonical |
| PB.1087.17 | chr3 | - | junction_1 | 124906743 | 124908146 | known | GTAG | canonical |
| PB.1087.17 | chr3 | - | junction_2 | 124908349 | 124910414 | known | GTAG | canonical |
| PB.1087.17 | chr3 | - | junction_3 | 124910500 | 124912103 | known | GTAG | canonical |
| PB.1087.17 | chr3 | - | junction_4 | 124912142 | 124913110 | known | GTAG | canonical |
| PB.1087.17 | chr3 | - | junction_5 | 124913241 | 124913561 | known | GTAG | canonical |
| PB.1087.17 | chr3 | - | junction_6 | 124913682 | 124916316 | known | GTAG | canonical |
| PB.1087.17 | chr3 | - | junction_7 | 124916481 | 124920233 | known | GTAG | canonical |
| PB.1087.17 | chr3 | - | junction_8 | 124920290 | 124922196 | known | GTAG | canonical |
| PB.1087.17 | chr3 | - | junction_9 | 124922304 | 124923526 | known | GTAG | canonical |
| PB.1087.17 | chr3 | - | junction_10 | 124923650 | 124927531 | known | GTAG | canonical |
| PB.1087.17 | chr3 | - | junction_11 | 124927994 | 124934660 | known | GTAG | canonical |
| PB.1087.22 | chr3 | - | junction_1 | 124906743 | 124908146 | known | GTAG | canonical |
| PB.1087.22 | chr3 | - | junction_2 | 124908349 | 124910414 | known | GTAG | canonical |
| PB.1087.22 | chr3 | - | junction_3 | 124910500 | 124912103 | known | GTAG | canonical |
| PB.1087.22 | chr3 | - | junction_4 | 124912142 | 124913110 | known | GTAG | canonical |
| PB.1087.22 | chr3 | - | junction_5 | 124913241 | 124913561 | known | GTAG | canonical |
| PB.108 7.22 | chr3 | - | junction_6 | 124913682 | 124916316 | known | GTAG | canonical |
| PB.1087.22 | chr3 | - | junction_7 | 124916481 | 124920233 | known | GTAG | canonical |
| PB.1087.22 | chr3 | - | junction_8 | 124920290 | 124922196 | known | GTAG | canonical |
| PB.1087.22 | chr3 | - | junction_9 | 124922304 | 124923526 | known | GTAG | canonical |
| PB.1087.22 | chr3 | - | junction_10 | 124923650 | 124927531 | known | GTAG | canonical |
| PB.1087.22 | chr3 | - | junction_11 | 124927748 | 124927795 | novel | CATA | non-canonical |
| PB.1087.22 | chr3 | - | junction_12 | 124927994 | 124934660 | known | GTAG | canonical |
| PB.1087.30 | chr3 | - | junction_1 | 124906743 | 124908146 | known | GTAG | canonical |
| PB.1087.30 | chr3 | - | junction_2 | 124908349 | 124910414 | known | GTAG | canonical |
| PB.1087.30 | chr3 | - | junction_3 | 124910500 | 124912103 | known | GTAG | canonical |
| PB.1087.30 | chr3 | - | junction_4 | 124912142 | 124913110 | known | GTAG | canonical |
| PB.1087.30 | chr3 | - | junction_5 | 124913241 | 124913561 | known | GTAG | canonical |
| PB.1087.30 | chr3 | - | junction_6 | 124913682 | 124916316 | known | GTAG | canonical |
| PB.1087.30 | chr3 | - | junction_7 | 124916463 | 124920233 | novel | GTAG | canonical |
| PB.1087.30 | chr3 | - | junction_8 | 124920290 | 124922196 | known | GTAG | canonical |
| PB.1087.30 | chr3 | - | junction_9 | 124922304 | 124923526 | known | GTAG | canonical |
| PB.1087.30 | chr3 | - | junction_10 | 124923650 | 124927531 | known | GTAG | canonical |
| PB.1087.30 | chr3 | - | junction_11 | 124927994 | 124934660 | known | GTAG | canonical |
| PB.1087.55 | chr3 | - | junction_1 | 124922615 | 124922693 | novel | GTTC | non-canonical |
| PB.1087.55 | chr3 | - | junction_2 | 124927994 | 124934660 | known | GTAG | canonical |

| **Non-inflamed** | | | | | | | | |
|---|---|---|---|---|---|---|---|---|
| isoform | chromosome | strand | junction_number | genomic_start_coord | genomic_end_coord | junction_category | splice_site | canonical |
| PB.1087.18 | chr3 | - | junction_1 | 124905998 | 124906137 | novel | AGAG | non-canonical |
| PB.1087.18 | chr3 | - | junction_2 | 124906743 | 124908146 | known | GTAG | canonical |
| PB.1087.18 | chr3 | - | junction_3 | 124908349 | 124910414 | known | GTAG | canonical |
| PB.1087.18 | chr3 | - | junction_4 | 124910500 | 124912103 | known | GTAG | canonical |
| PB.1087.18 | chr3 | - | junction_5 | 124912142 | 124913110 | known | GTAG | canonical |
| PB.1087.18 | chr3 | - | junction_6 | 124913241 | 124913561 | known | GTAG | canonical |
| PB.1087.18 | chr3 | - | junction_7 | 124913682 | 124916316 | known | GTAG | canonical |
| PB.1087.18 | chr3 | - | junction_8 | 124916481 | 124920233 | known | GTAG | canonical |
| PB.1087.18 | chr3 | - | junction_9 | 124920290 | 124922196 | known | GTAG | canonical |
| PB.1087.18 | chr3 | - | junction_10 | 124922304 | 124923526 | known | GTAG | canonical |
| PB.1087.18 | chr3 | - | junction_11 | 124923650 | 124927531 | known | GTAG | canonical |
| PB.1087.18 | chr3 | - | junction_12 | 124927994 | 124934660 | known | GTAG | canonical |
| PB.1087.64 | chr3 | - | junction_1 | 124931778 | 124934586 | novel | CTAG | non-canonical |

| **Inflamed** | | | | | | | | |
|---|---|---|---|---|---|---|---|---|
| isoform | chromosome | strand | junction_number | enomic_start_coord | genomic_end_coord | junction_category | splice_site | canonical |
| PB.1087.6 | chr3 | - | junction_1 | 124906743 | 124908146 | known | GTAG | canonical |
| PB.1087.6 | chr3 | - | junction_2 | 124908349 | 124910414 | known | GTAG | canonical |
| PB.1087.6 | chr3 | - | junction_3 | 124910500 | 124912103 | known | GTAG | canonical |
| PB.1087.6 | chr3 | - | junction_4 | 124912142 | 124913161 | novel | GTAG | canonical |
| PB.1087.6 | chr3 | - | junction_5 | 124913241 | 124913561 | known | GTAG | canonical |
| PB.1087.6 | chr3 | - | junction_6 | 124913682 | 124916316 | known | GTAG | canonical |
| PB.1087.6 | chr3 | - | junction_7 | 124916481 | 124920233 | known | GTAG | canonical |
| PB.1087.6 | chr3 | - | junction_8 | 124920290 | 124922196 | known | GTAG | canonical |
| PB.1087.6 | chr3 | - | junction_9 | 124922304 | 124923526 | known | GTAG | canonical |
| PB.1087.63 | chr3 | - | junction_1 | 124931778 | 124934480 | novel | CAAG | non-canonical |
| PB.1087.21 | chr3 | - | junction_1 | 124906743 | 124908146 | known | GTAG | canonical |
| PB.1087.21 | chr3 | - | junction_2 | 124908349 | 124910414 | known | GTAG | canonical |
| PB.1087.21 | chr3 | - | junction_3 | 124910500 | 124912103 | known | GTAG | canonical |
| PB.1087.21 | chr3 | - | junction_4 | 124912142 | 124913110 | known | GTAG | canonical |
| PB.1087.21 | chr3 | - | junction_5 | 124913241 | 124913561 | known | GTAG | canonical |
| PB.1087.21 | chr3 | - | junction_6 | 124913682 | 124916316 | known | GTAG | canonical |
| PB.1087.21 | chr3 | - | junction_7 | 124916481 | 124920233 | known | GTAG | canonical |
| PB.1087.21 | chr3 | - | junction_8 | 124920290 | 124920708 | novel | GTAG | canonical |
| PB.1087.21 | chr3 | - | junction_9 | 124921025 | 124922196 | novel | GTAG | canonical |
| PB.1087.21 | chr3 | - | junction_10 | 124922304 | 124923526 | known | GTAG | canonical |
| PB.1087.21 | chr3 | - | junction_11 | 124923650 | 124927531 | known | GTAG | canonical |
| PB.1087.21 | chr3 | - | junction_12 | 124927994 | 124934660 | known | GTAG | canonical |
| PB.1087.20 | chr3 | - | junction_1 | 124906256 | 124913196 | novel | CAAG | non-canonical |
| PB.1087.20 | chr3 | - | junction_2 | 124913241 | 124913561 | known | GTAG | canonical |
| PB.1087.20 | chr3 | - | junction_3 | 124913682 | 124916316 | known | GTAG | canonical |
| PB.1087.20 | chr3 | - | junction_4 | 124916481 | 124920233 | known | GTAG | canonical |
| PB.1087.20 | chr3 | - | junction_5 | 124920290 | 124922196 | known | GTAG | canonical |
| PB.1087.20 | chr3 | - | junction_6 | 124922304 | 124923526 | known | GTAG | canonical |
| PB.1087.20 | chr3 | - | junction_7 | 124923650 | 124927531 | known | GTAG | canonical |
| PB.1087.20 | chr3 | - | junction_8 | 124927994 | 124934660 | known | GTAG | canonical |
| PB.1087.25 | chr3 | - | junction_1 | 124906743 | 124908146 | known | GTAG | canonical |
| PB.1087.25 | chr3 | - | junction_2 | 124908349 | 124910414 | known | GTAG | canonical |
| PB.1087.25 | chr3 | - | junction_3 | 124910500 | 124912103 | known | GTAG | canonical |
| PB.1087.25 | chr3 | - | junction_4 | 124912142 | 124913110 | known | GTAG | canonical |
| PB.1087.25 | chr3 | - | junction_5 | 124913203 | 124913577 | novel | AGAG | non-canonical |
| PB.1087.25 | chr3 | - | junction_6 | 124913682 | 124916316 | known | GTAG | canonical |
| PB.1087.25 | chr3 | - | junction_7 | 124916481 | 124920233 | known | GTAG | canonical |
| PB.1087.25 | chr3 | - | junction_8 | 124920290 | 124922196 | known | GTAG | canonical |
| PB.1087.25 | chr3 | - | junction_9 | 124922304 | 124923526 | known | GTAG | canonical |
| PB.1087.68 | chr3 | - | junction_1 | 124931778 | 124934705 | novel | CCAG | non-canonical |
| PB.1087.32 | chr3 | - | junction_1 | 124906743 | 124908146 | known | GTAG | canonical |
| PB.1087.32 | chr3 | - | junction_2 | 124908349 | 124913110 | novel | GTAG | canonical |
| PB.1087.32 | chr3 | - | junction_3 | 124913241 | 124913561 | known | GTAG | canonical |
| PB.1087.32 | chr3 | - | junction_4 | 124913682 | 124916316 | known | GTAG | canonical |
| PB.1087.32 | chr3 | - | junction_5 | 124916481 | 124920233 | known | GTAG | canonical |
| PB.1087.32 | chr3 | - | junction_6 | 124920290 | 124922196 | known | GTAG | canonical |
| PB.1087.32 | chr3 | - | junction_7 | 124922304 | 124923526 | known | GTAG | canonical |
| PB.1087.32 | chr3 | - | junction_8 | 124923650 | 124927531 | known | GTAG | canonical |
| PB.1087.32 | chr3 | - | junction_9 | 124927994 | 124934660 | known | GTAG | canonical |
| PB.1087.27 | chr3 | - | junction_1 | 124906225 | 124908173 | novel | AGAC | non-canonical |
| PB.1087.27 | chr3 | - | junction_2 | 124908349 | 124910414 | known | GTAG | canonical |
| PB.1087.27 | chr3 | - | junction_3 | 124910500 | 124912103 | known | GTAG | canonical |
| PB.1087.27 | chr3 | - | junction_4 | 124912142 | 124913110 | known | GTAG | canonical |
| PB.1087.27 | chr3 | - | junction_5 | 124913241 | 124913561 | known | GTAG | canonical |
| PB.1087.27 | chr3 | - | junction_6 | 124913682 | 124916316 | known | GTAG | canonical |
| PB.1087.27 | chr3 | - | junction_7 | 124916481 | 124920233 | known | GTAG | canonical |
| PB.1087.27 | chr3 | - | junction_8 | 124920290 | 124922196 | known | GTAG | canonical |
| PB.1087.27 | chr3 | - | junction_9 | 124922304 | 124923526 | known | GTAG | canonical |
| PB.1087.27 | chr3 | - | junction_10 | 124923650 | 124927531 | known | GTAG | canonical |
| PB.1087.27 | chr3 | - | junction_11 | 124927994 | 124934660 | known | GTAG | canonical |
| PB.1087.31 | chr3 | - | junction_1 | 124906743 | 124908146 | known | GTAG | canonical |
| PB.1087.31 | chr3 | - | junction_2 | 124908349 | 124910414 | known | GTAG | canonical |
| PB.1087.31 | chr3 | - | junction_3 | 124910500 | 124912103 | known | GTAG | canonical |
| PB.1087.31 | chr3 | - | junction_4 | 124912142 | 124913110 | known | GTAG | canonical |
| PB.1087.31 | chr3 | - | junction_5 | 124913241 | 124913561 | known | GTAG | canonical |
| PB.1087.31 | chr3 | - | junction_6 | 124913682 | 124916316 | known | GTAG | canonical |
| PB.1087.31 | chr3 | - | junction_7 | 124916481 | 124920233 | known | GTAG | canonical |
| PB.1087.31 | chr3 | - | junction_8 | 124920290 | 124922196 | known | GTAG | canonical |
| PB.1087.31 | chr3 | - | junction_9 | 124922304 | 124923526 | known | GTAG | canonical |
| PB.1087.31 | chr3 | - | junction_10 | 124923650 | 124927531 | known | GTAG | canonical |
| PB.1087.31 | chr3 | - | junction_11 | 124927874 | 124927951 | novel | AAAG | non-canonical |
| PB.1087.31 | chr3 | - | junction_12 | 124927994 | 124934660 | known | GTAG | canonical |
| PB.1087.52 | chr3 | - | junction_1 | 124922304 | 124923526 | known | GTAG | canonical |
| PB.1087.52 | chr3 | - | junction_2 | 124923650 | 124927531 | known | GTAG | canonical |
| PB.1087.52 | chr3 | - | junction_3 | 124927994 | 124934660 | known | GTAG | canonical |
| PB.1087.56 | chr3 | - | junction_1 | 124922624 | 124922693 | novel | GTGT | non-canonical |

### EXAMPLE3: Aberrant mucin expression in association with tight junction dysfunction in the respiratory and intestinal epithelium during SARS-CoV-2 infection

### 1. BACKGROUND TO THE INVENTION

Severe acute respiratory syndrome coronavirus 2 (SARS-CoV-2), causing coronavirus disease 2019 (COVID-19), emerged in Wuhan, China, in December 2019. An initial cluster of infections was linked to the Huanan seafood market, potentially due to animal contact. SARS-CoV-2 is closely related to SARS-CoV, responsible for the SARS outbreak 18 years ago (Zhou et al., 2020), and has now spread rapidly worldwide. On March 11, 2020, the World Health Organization (WHO) declared COVID-19 a pandemic. Common symptoms reported in adults are fever, dry cough, fatigue and shortness of breath. While most COVID-19 patients (ca. 80%) remain asymptomatic or have mild to less severe respiratory complaints, some (ca. 15-20%) are hospitalised of which a minority develops a frequently lethal acute respiratory distress syndrome (ARDS). This results in mucus exudation, pulmonary oedema, hypoxia and lung failure in association with a cytokine storm characterized by amongst others Th17 immune profiles. Besides elderly or those with chronic underlying diseases, also young, healthy individuals die of COVID-19.

SARS-CoV-2 is a positive-sense single stranded RNA virus having 4 structural proteins, known as the S (spike), E (envelope), M (membrane) and N (nucleocapsid) proteins. The N protein holds the RNA genome, and the S, E and M proteins create the viral envelope. The S protein of coronaviruses regulates viral entry into target cells, i.e. ciliated epithelial cells. Entry depends on binding of the subunit S1 to a cellular receptor, which facilitates viral attachment to the surface of target cells. Entry also requires S protein priming by cellular proteases, which cleave the S protein at its S1/S2 site allowing fusion of viral and cellular membranes, a process driven by the S2 subunit. Similar to SARS-CoV, the angiotensin-converting enzyme 2 (ACE2) is the entry receptor for SARS-CoV-2 and the cellular serine protease TMPRSS2 is essential for priming the S protein. ACE2 and TMPRSS2 expression is not only limited to the respiratory tract and extrapulmonary spread of SARS-COV-2 should therefore not be neglected. Indeed, a subset (ca. 30-35%) of COVID-19-positive patients (both ambulatory and hospitalised) showed gastrointestinal symptoms, including diarrhoea, abdominal pain, loss of appetite and nausea, and associated with a more indolent form of COVID-19 compared to patients with respiratory symptoms. Live SARS-CoV-2 was even successfully isolated from the stool of patients. This indicates that the intestinal epithelium is also susceptible to infection and recent work even provided evidence for an additional serine protease TMPRSS4 in priming the SARS-CoV-2S protein.

Furthermore, it has been suggested that the modest ACE2 expression in the upper respiratory tract has limited SARS-CoV transmissibility in the past. This is in large contrast to the currently reported SARS-CoV-2 infected cases which clearly surpassed that of SARS-CoV. In light of this increased transmissibility, we can speculate that this new coronavirus utilizes additional cellular attachment-promoting co-factors to ensure robust infection of ACE2⁺ cells in the respiratory tract. This could comprise binding to cellular glycans, as shown for other coronaviruses. Interestingly, mucus hyperproduction in the bronchioles and alveoli from severely ill COVID-19 patients has been reported (Guan et al., 2020; own observations ICU UZA), complicating the ICU stay and recovery. Secreted and transmembrane mucins are O-linked glycans produced by goblet and ciliated cells, respectively, and are the major components of the mucus layer covering the epithelial cells. Both mucus and epithelium constitute the mucosal barrier. Besides having a protective function, transmembrane mucins also participate in intracellular signal transduction and thus play an important role in mucosal homeostasis by establishing a delicate balance with tight junctions to maintain barrier integrity. Transmembrane mucins, particularly MUC13, might thus act as additional host factors enabling the virus to spread faster and cause tissue damage. In this study, we therefore investigated the expression patterns of ACE2, TMPRSS2/TMPRSS4, mucins and junctional proteins during SARS-CoV-2 infection in the respiratory and intestinal epithelium. Furthermore, the interplay between MUC13 and ACE2 expression upon viral infection was also studied.

### 2. MATERIAL AND METHODS

### 2.1. Viruses and biosafety

The SARS-CoV-2 isolate 2019-nCoV/ltaly-lNMI1, available at the European Virus Archive-Global (EVAg) database, was used throughout the study. SARS-CoV-2 was subjected to passages in Vero E6 cells (green monkey kidney; ATCC CRL-1586), grown in Dulbecco's modified Eagle's minimal essential medium (DMEM; Gibco) supplemented with 10% heat-inactivated fetal calf serum (FCS), before usage in the cell culture experiments. The infectious viral titers in the cell-free supernatant were determined by a standard TCID₅₀ assay. All experiments entailing live SARS-CoV-2 were conducted in the biosafety level 3 facility at the Institute for Tropical Medicine, Antwerp, Belgium.

### 2.2. Cell culture and virus infection

LS513 (human colorectal carcinoma (ATCC CRL-2134TM)) and Caco-2 (human colorectal carcinoma ATCC HTB-37) cells were grown in Roswell Park Memorial Institute (RPMI)-1640 medium (Life Technologies) supplemented with 10% heat-inactivated FCS, 100 U ml⁻¹ penicillin, 100 µg ml⁻¹ streptomycin, and 2 mM L-glutamine. Calu3 (lung adenocarcinoma ATCC HBT-55) cells were grown in Minimal Essential Medium (MEM; Gibco) supplemented with 10% heat-inactivated FCS, 100 U ml⁻¹ penicillin, 100 µg ml⁻¹ streptomycin, 1X MEM Non-essential Amino Acids and 1mM sodium pyruvate. For viral infection, all cells were seeded in 6 well-plates: 1 x 10⁶ cells/ml (LS513); 5 x 10⁵ cells/ml (Caco-2 and Calu3). After reaching confluence, the cells were inoculated with SARS-CoV-2 at a multiplicity of infection (MOI) of 0.1 for 24h and 48h at 37°C (5% CO₂), Cells treated with the growth medium of the virus were included as controls. All experiments were performed containing 6 technical replicates for each time-point and cell line.

### 2.3. Small interfering RNA (siRNA) transfection assays

At the start of the transfection experiments, cells were seeded and grown in 6 well-plates (LS513: 1 x 10⁶ cells/ml; Caco-2 and Calu-3: 3 x 10⁵ cells/ml). After 24 hours, the cells were transfected with 75 pmol Silencer Select siRNA targeting MUC13 (s32232, ThermoFisher Scientific) or with 75pmol Silencer Select Negative Control siRNA (4390843, ThermoFisher Scientific) using Lipofectamine RNAiMAX transfection reagent (7.5 µl/well, Invitrogen). Forty-eight hours post-transfection, cells were extensively washed and infected with SARS-CoV-2 at a MOI of 0.1 for 48 hours. Cells treated with the growth medium of the virus were included as controls. All transfection experiments were performed containing 6 technical replicates per cell line.

### 2.4. RNA extraction and quantitative RT-PCR

Cells and supernatants were harvested at 24 hpi (hours post infection) and 48 hpi for quantitative RT-PCR analysis of host gene expression and virus replication, as previously described (Corman et al., 2020; Breugelmans et al., 2020). Briefly, total RNA from lysed cells and supernatants (100 µl) was extracted using the Nucleospin RNA plus kit (Macherey-Nagel) and QIAamp viral RNA kit (Qiagen), respectively, following the manufacturer's instructions. The concentration and quality of the RNA were evaluated using the Nanodrop ND-1000 UV-Vis Spectrophotometer (Thermo Fisher Scientific). For gene expression analysis, 1 µg RNA extracted from transfected and non-transfected cells was subsequently converted to cDNA by reverse transcription using the SensiFast^{™} cDNA synthesis kit (Bioline). Relative gene (i.e. ACE2, TMPRSS2, TMPRSS4, mucins and tight junctions) expression was then determined by SYBR Green RT-qPCR using the GoTaq qPCR master mix (Promega) on a QuantStudio 3 Real-Time PCR instrument (Thermo Fisher Scientific). Following quantitect primer assays (Qiagen) were used: Hs_GAPDH (QT00079247), Hs_ACTB (QT00095431), Hs_TMPRSS2 (QT00058156), Hs_TMPRSS4 (QT00033775), Hs_ACE2 (QT00034055), Hs_MUC1 (QT00015379), Hs_MUC2 (QT01004675), Hs_MUC4 (QT00045479), Hs_MUC5AC (QT00088991), Hs_MUC5B (QT01322818), Hs_MUC6 (QT00237839), Hs_MUC13 (QT00002478), Hs_CLDN1 (QT00225764), Hs_CLDN2 (QT00089481), Hs_CLDN3 (QT00201376), Hs_CLDN4 (QT00241073), Hs_CLDN7 (QT00236061), Hs_CLDN12 (QT01012186), Hs_CLDN15 (QT00202048), Hs_CLDN18 (QT00039550), Hs_CDH1 (QT00080143), Hs_OCLN (QT00081844), Hs_ZO-1 (QT00077308), Hs_ZO-2 (QT00010290). All RT-qPCR reactions were performed in duplicate and involved an initial DNA polymerase activation step for 2 min at 95°C, followed by 40 cycles of denaturation at 95°C for 15 sec and annealing/extension for 1 min at 60°C. Analysis and quality control were performed using qbase+ software (Biogazelle). Relative expression of the target genes was normalized to the expression of the housekeeping genes ACTB and GAPDH. To quantify viral RNA in the transfected and non-tranfected cells and supernatants, the iTaq Universal Probes One-Step kit (BioRad) was used on a LightCycler 480 Real-Time PCR System (Roche). A 25 µl reaction contained 1 µl RNA, 12.5 µl of 2 x reaction buffer provided with the kit, 0.625 µl of iScript reverse transcriptase from the kit, 0.4 µl forward primer (25 µM), 0.4 µl reverse primer (25 µM), 0.5 µl probe (10 µM) targeting the SARS-CoV-2 E gene and 9.575 µl H₂O. We incubated the reactions at 50°C for 10 min for reverse transcription, 95°C for 5 min for denaturation, followed by 50 cycles of 95°C for 10 s and 58°C for 30 s. Analysis was performed using qbase+ software to determine cycle tresholds (Ct).

### 2.5. Statistical analysis

Statistical analysis using the GraphPad Prism 8.00 software (license DFG170003) was performed to determine significant differences between SARS-CoV-2 infected and uninfected cells and between MUC13 siRNA and ctrl siRNA transfected cells infected or not with SARS-CoV-2. Data were analysed by the Analysis of Variance (ANOVA) test and are presented as means ± standard error of mean (SEM). Significance levels are indicated on the graphs and were corrected for multiple testing using the Tukey-Kramer's and Dunn's post-hoc multiple comparisons tests.

### 3. RESULTS AND DISCUSSION

All cell lines tested here were susceptible for SARS-CoV-2 infection as shown by virus replication over a period of 48h (data not shown). Virus production was significantly higher in the supernatant of Caco-2 and Calu3 cells compared to LS513 (p= 0.0004; **Figure 16**). This is in agreement with a recent study that described a robust replication of SARS-CoV-2 in both Caco-2 and Calu3 cells. Cell damage induced by SARS-CoV-2 was also assessed microscopically. No cytopathic effects, as typically described in Vero E6 cells, was noted in LS513 and Caco-2 cells. Interestingly, a substantial cell damage was noted in transfected Calu3 cells (30% viability at 48 hpi; p<0.001) but not in non-transfected cells. The induction of cell damage in Calu3 cells caused by corona viruses still remains controversial. A recent study described no cell death in SARS-CoV- and SARS-CoV-2-infected Calu3 cells, whereas earlier studies reported that SARS-CoV infection induced cytopathic effects in Calu3. A reason for these discrepancies is currently unknown, but it cannot be excluded that in our study transfection with siRNA made the cells more susceptible for viral cytopathic effects.

As SARS-CoV-2 uses the receptor ACE2 for entry and the serine proteases TMPRSS2 and TMPRSS4 for S protein priming, expression of these host factors was investigated. In our study, ACE2 mRNA expression was significantly reduced in Caco-2 cells at 24 hpi (p = 0.0001) and 48 hpi (p= 0.0008) and in Calu3 cells at 24 hpi only (p = 0.0004) **(****Figure 17****).** No changes in ACE2 expression were noted in LS513 which could explain the significant lower virus production compared to Caco-2 and Calu3 **(****Figure 16 & 17****).** ACE2 is an important component of the renin-angiotensin pathway and counterbalances the effects of AT1 activation by angiotensin II. In the lungs, ACE2 has an anti-inflammatory role protecting the respiratory tract from injury, whereas it maintains mucosal barrier homeostasis in the intestines by regulating expression of antimicrobial peptides (AMPs) and the ecology of the gut microbiome. Downregulation of this receptor upon SARS-CoV-2 infection could thus exaggerate acute lung and intestinal injury because of the imbalance in angiotensin II or AT1 signalling. On the contrary, expression of TMPRSS2 was significantly increased in all cell types at 48 hpi (TMPRSS2: p = 0.0433 (LS513), p = 0.0057 (Caco-2), p = 0.0001 (Calu3); **Figure 17**) compared to uninfected controls whereas upregulation of TMPRSS4 was remarkably only seen in Calu3 cells (p = 0.0152). The abundancy of TMPRSS2 and to a lesser extend TMPRSS4 is thus essential for promoting viral entry into host cells. In addition, TMPRSS2 is also an important mediator of mucosal barrier dysfunction and linked to aberrant mucin expression. We therefore also investigated the impact of SARS-CoV-2 infection on mucin and tight junction expression. In our study, significant changes in mucin expression were mainly seen at 48 hpi. More specifically, the transmembrane MUC1, MUC13 and MUC4 mucins were strongly upregulated in both intestinal and pulmonary SARS-CoV-2-infected epithelial cells (MUC1: p = 0.0022 (LS513); p = (Calu3); MUC4: p = 0.0022 (LS513); p = 0.0022 (Calu3); MUC13: p = 0.0022 (LS513); p = 0.0022 (Caco-2); p = 0.0022 (Calu3); **Figure 18**), whereas the secreted mucins (particularly MUC2 (p = 0.058 (LS513); p = (Caco, 24 hpi); p = (Caco-2; 48 hpi)), MUC5AC (p = 0.0012 (LS513)) and MUC6 (p = 0.0022 (LS513)), which are at the frontline of mucosal defence (Linden et al., 2007), were significantly downregulated with the exception of MUC2 (p = 0.0001) and MUC5AB (p = 0.0001) expression in Calu3 cells **(****Figure 19****).** As own data showed a functional link between MUC13 and ACE2, we further investigated whether ACE2 downregulation upon viral infection is mediated by MUC13 using siRNA transfection assays. Knockdown of MUC13 was successful in all three cell lines in which a reduction in MUC13 expression of approximately 70-80% was maintained during infection (Figure 5). In ctrl siRNA transfected Caco-2 and Calu-3 cells, MUC13 expression significantly increased upon SARS-CoV-2 infection whereas ACE2 expression significantly decreased **(****Figure 20****).** This is in agreement to what is seen in wildtype SARS-CoV-2-infected Caco-2 and Calu3 cells (**Figure 18**). Interestingly, knockdown of MUC13 decreased ACE2 expression in Caco-2 and Calu3 control cells (p = 0.0004 (Caco-2); p = 0,09 (Calu3)) and its expression further declined upon SARS-CoV-2 infection although not significant **(****Figure 20****).** This strengthens the evidence that ACE2 expression is mediated by MUC13. In addition, MUC13 expression was not altered in ctrl siRNA transfected LS513 cells upon infection **(****Figure 20****)** which is in contrast to what is seen in wildtype SARS-CoV-2-infected LS513 cells **(****Figure 18****).** ACE2 expression remained unchanged **(****Figure 20****)** and lower virus production in the supernatants was noted **(****Figure 16****).** This further highlights the importance of increased MUC13 expression mediating ACE2 signalling for optimal virus production.

Furthermore, inappropriate overexpression of MUC13 can also affect barrier integrity by disrupting cell polarity and cell-cell interactions resulting in tight junction dysfunction, as recently shown. In our study, a significant increase in gene expression of several junctional proteins was noted at 48 hpi **(****Figure 21****),** suggesting the ability of SARS-CoV-2 to alter epithelial barrier integrity, as described for SARS-CoV. Most alterations in expression were seen in LS513 and Calu3 cells, i.e.: CLDN1 (p = 0.0022 5LS513); p = 0.0001 (Calu3)), CLDN2 (p = 0.0007 (Caco-2)), CLDN3 (p = 0.075 (LS513); p = 0.0001 (Calu3)), CLDN4 (p = 0.01 (LS513); p = 0.0001 (Calu3)), CLDN7 (p = 0.0085 (LS513); p = 0.0001 (Calu3)), CLDN12 (p = 0.031 (Calu3)), CLDN15 (p = 0.0139 (Caco-2); P = 0.0004 (Calu3)), CDH1 (p = 0.003 (Caco-2); p = 0.0013 (Calu3)), OCLN (p = 0.0335 (LS513); p = 0.0004 (Caco-2); p = 0.0002 (Calu3)), ZO-1 (p = 0.034 (Caco-2); p = 0.0001 (Calu3)) and ZO-2 (p = 0.0005 (Caco-2)).

Taken together, the results from this study further underline the tropism of SARS-CoV-2 for both the respiratory and intestinal epithelium and demonstrate that this novel coronavirus strongly affects the mucosal barrier integrity upon infection by inducing aberrant mucin expression and tight junction dysfunction. Furthermore, a role for transmembrane mucins, particularly MUC13, in contributing to the infection of SARS-CoV-2 is also suggested.

### EXAMPLE 4: Mucin mRNA isoforms for diagnosis and monitoring coronaviral infections

### 1. BACKGROUND OF THE INVENTION

A novel coronavirus, SARS-CoV-2 causing coronavirus disease 2019 (COVID-19), emerged in Wuhan, China, in December 2019 and has since then disseminated globally. Common symptoms are fever, dry cough, fatigue, shortness of breath and changes in smell or taste whereas gastrointestinal symptoms, such diarrhoea, abdominal pain, loss of appetite and nausea can also occur.
Patients with COVID-19 exhibit a broad spectrum of disease severity with 80% showing mild, moderate or no symptoms; 15% showing severe symptoms; and 5% developing acute lung injury with the potential progression towards a lethal acute respiratory distress syndrome. Besides elderly or those with chronic underlying diseases, also young, healthy individuals and even children die of COVID-19 (Huang et al., 2020; Ruan, 2020). This underscores the urgent need to unravel molecular factors that shape the course of COVID-19 and identify "at risk" patients for progressing to severe disease.

Respiratory ciliated epithelial cells are the primary targets of SARS-CoV-2 and viral entry requires binding to the ACE2 receptor and subsequent priming by TMPRSS2. Interestingly, ACE2 expression increases with age and variation in ACE2 expression between children with high and low viral loads was recently described (Hoffmann et al., 2020). However, as other coronaviruses with markedly milder pathogenicity also use ACE2 for initial cellular entry (Hoffmann et al., 2020), we can then speculate that SARS-CoV-2 uses additional factors mediating infection of ACE2⁺ cells and subsequent tissue damage.
Secreted and transmembrane mucins (MUCs), produced by goblet and ciliated cells, respectively, are the gatekeepers of the mucus layer protecting the respiratory barrier function against inhaled injurious substances. Upon disease, however, aberrant mucin expression forms a dysfunctional mucus barrier and becomes pathologic (Breugelmans et al., 2020). Indeed, mucin hypersecretion is a major clinical feature seen in severely ill COVID-19 patients with mucus accumulating in the airways obstructing the respiratory tract and complicating breathing and recovery (Wenju et al., 2020). These observations prompted us to hypothesize that SARS-CoV-2 infection stimulates mucin overexpression, further promoting disease severity. Own recent unpublished data showed that the excessive mucus production seen in the lungs of COVID-19 patients is characterized by the presence of several mucins including not only MUC1 and MUC5AC as shown before (Wenju et al., 2020), but also MUC2, MUC4, MU5B, MUC13, MUC16 and MUC21 **(****Fig. 22A****),** which are triggered by SARS-CoV-2 **(****Fig. 19** **and** **22B****),** and we have also evidence for a functional link between increased MUC13 expression, ACE2 downregulation and lung barrier dysfunction (characterized by aberrant tight junction expression) upon viral infection **(****Fig. 22C****).**

Furthermore, mucins are highly polymorphic, and the presence of genetic differences can alter gene expression resulting in several mRNA isoforms via alternative splicing. While most mRNA isoforms encode similar biological functions, some alter protein function resulting in progression towards disease (Moehle et al., 2006). Such disease-associated mucin mRNA isoforms might thus contribute to COVID-19 severity and treatment to reduce mucin hyperproduction can be of utmost clinical importance (d'Alessandro et al., 2020).

In this study, we first analysed the mRNA expression levels of mucins in the blood of hospitalized COVID-19 patients with severe disease, ambulatory COVID-19 and non-COVID-19 patients with mild disease and healthy controls and correlated aberrantly expressed mucins with COVID-19 positivity and severity. Thereafter, we investigated the effect of treatment with FDA approved drugs for COVID-9 on mucin expression in pulmonary epithelial cells. Finally, we unravelled the mucin mRNA isoforms that were aberrantly expressed in COVID-19 patients and associated with COVID-19 positivity and severity.

### 2. MATERIAL AND METHODS

### 2.1. Patient cohorts, clinical characteristics and sample collection

Critical COVID-19 patients hospitalized at the tertiary intensive care unit (ICU) of the University hospital Antwerp, Belgium (Table 7; N=15) and ambulatory COVID-19 patients (Table 7; N=10) with mild symptoms (Table 8) recruited at general practitioner practices, were enrolled for this study. Ambulatory patients with mild common cold symptoms but negative for COVID-19 (Table 7; N=4) and healthy controls (Table 7; N=4) were included as control groups.

Regarding the hospitalized patients with severe COVID-19, the median duration from symptom onset until hospital admission was 6 days, with a total median hospital duration of 29 days of which ca. 18 days at the ICU. Most of these ICU patients required invasive ventilation with a median length of 13.8 days of which 50% also needed a replacement of the endotracheal tube due to mucus obstruction (Table 9). Other clinical characteristics, including co-morbidities, hospitalization, organ failure and mortality, are also shown in Table 9.

**Table 7: Demographics of the different patient groups**

| **Demographics** | **COVID-19 (severe, ICU)** | **COVID-19 (mild, ambulatory)** | **Non-COVID-19 (mild, ambulatory)** | **Non-COVID-19 (healthy)** |
|---|---|---|---|---|
| **Number** | 15 | 10 | 4 | 4 |
| **Age, years (median, range)** | 57.33 (27-82) | 30 (17-57) | 40 (32-48) | 36 (25-52) |
| **Sex (male, female)** | 9/15 (male) | 5/10 (male) | 3/4 (male) | 2/4 (male) |
| | 6/15 (female) | 5/10 (female) | 1/4 (female) | 2/4 (female) |
| **BMI, kg/m² (median, range)** | 29.02 (21.2-43) | - | - | - |

**Table 8: Overview of the symptoms among mild COVID-19 patients (N=10)**

| **Symptoms** | **Loss of smell and taste** | **Rhinitis** | **Fever** | **Myalgia** | **Malaise** | **Cough** | **Headache** | **Gastro-intestinal symptoms** |
|---|---|---|---|---|---|---|---|---|
| **Number of** | 9/10 | 6/10 | 3/10 | 2/10 | 2/10 | 1/10 | 3/10 | 1/10 |
| **patients (%)** | (90%) | (60%) | (30%) | (20%) | (20%) | (10%) | (30%) | (10%) |

**Table 9: clinical data of critically ill COVID-19 patients hospitalized at the ICU (N=15)**

| **Co-morbidities** | **Number of patients (%)** | **Hospitalization** | **Mean (range)** | **Organ failure** | **Number of patients (%)** |
|---|---|---|---|---|---|
| **Diabetes** | 5/15 (33%) | **Symptom onset until hospital admission (days)** | 6.08 (3-10) | **ARDS** | 3/15 (20%) |
| **Lung disease** | 5/15 (33%) | **SOFA/SOFAₘₐₓ scores*** | 8 (2-18)/12 (3-21) | **Renal failure** | 4/15 (27%) |
| **Heart failure** | 1/15(7%) | **Hospitalisation ICU (days)** | 17.93 (6-65) | **Need vasopressors** | 11/15 (73%) |
| **Hypertension** | 6/15(40%) | **Total hospitalisation (days)** | 29.47 (15-77) | **Bacterial coinfection** | 7/15(47%) |
| **Renal insufficiency** | 4/15(27%) | **Invasive ventilation (Y/N)** | 0.8 (0-1) | **Fungal coinfection** | 7/15(47%) |
| **Malignancy** | 6/15(40%) | **Duration invasive ventilation (days)** | 13.8 (0-65) | **Cardiac complications** | 3/15(20%) |
| **Lymphoproliferative disease** | 3/15(20%) | **PₐO₂/FᵢO₂ ratio** | 97.57(44-231) | **Neurological complications** | 4/15(27%) |
| **Auto-immune diseases** | 3/15(20%) | **Replacement endotracheal tubes (ETT) (Y/N)** | 0.5(0-1) | **mortality** | 4/15(27%) |

| | | | | | |
|---|---|---|---|---|---|
| *sequential organ failure assessment scores (SOFA) | | | | | |

From all patients, blood samples were collected in PAXgene RNA blood tubes (PreAnalytiX) for RNA extraction purposes and subsequent gene expression and iso-sequencing approaches (see further). This study was approved by the Ethical Committee of the UZA (20/14/176 and 20/43/555) and signed informed consent was obtained.

### 2.2.Screening the ability of different therapies for COVID-19 to reduce mucin hypersecretion

Calu3 (lung adenocarcinoma ATCC HBT-55) cells were grown in Minimal Essential Medium (MEM; Gibco) supplemented with 10% heat-inactivated FCS, 100 U ml⁻¹ penicillin, 100 µg ml⁻¹ streptomycin, 1X MEM Non-essential Amino Acids and 1mM sodium pyruvate. For viral infection, all cells were seeded in 6 well-plates at a concentration of 5 x 10⁵ cells/ml. After reaching confluence, the cells were inoculated with SARS-CoV-2 at MOI of 0.1 for 2h, thereafter washed and treated with a drug at different concentrations for 48h. These include the following drugs approved by the FDA to treat COVID-19: Remdesivir (antiviral; 3.7 µM); favipiravir (antiviral; 1mM), (Hydroxy)chloroquine (10µM); Dexamethasone (corticosteroid able to reduce mucin expression; 1-5-10 µM); Tocilizumab (anti-IL6; 10-100-1000 ng/ml); Anakinra (anti-IL1; 50-500 ng/ml, 10 µg/ml); and Baricitinib (JAK1/2 inhibitor; 0.3-1-5 µM). Untreated cells infected with SARS-CoV-2 were also included as control group. After the treatment, cells were lysed for RNA and RT-qPCR extraction purposes.

### 2.3. RNA isolation and quality control

Total RNA was extracted from the collected blood samples using the PAXgene RNA blood kit (PreAnalytiX) and from the lysed cells using the Nucleospin RNA plus kit, following the manufacturer's instructions. The concentration and purity of the RNA were evaluated using the NanoDrop^{®} ND-1000 UV-Vis Spectrophotometer (Thermo Fisher Scientific) and Qubit Fluorometer (Qubit Broad Range RNA kit, Thermo Fisher Scientific). Quality control of the RNA was performed by capillary electrophoresis using an Agilent 2100 Fragment Analyzer (Agilent).

### 2.4. Mucin mRNA expression by RT-qPCR

One µg RNA was converted to cDNA by reverse transcription using the SensiFast^{™} cDNA synthesis kit (Bioline). Relative mucin gene expression was then determined by SYBR Green RT-qPCR using the GoTaq qPCR master mix (Promega) on a QuantStudio 3 Real-Time PCR instrument (Thermo Fisher Scientific). Standard validated QuantiTect primers available from Qiagen were used for *GAPDH* (QT00079247), *ACTB* (QT00095431), *MUC4* (QT00045479), MUC5AC (QT00088991) and *MUC5B* (QT01322818), *MUC2* (QT01004675), *MUC13* (QT00002478), *MUC16* (QT01192996), *MUC20* (QT00012068), *MUC21* (QT01159060) and *MUC1* (QT00015379). All RT-qPCR reactions were performed in duplicate and involved an initial DNA polymerase activation step for 2 min at 95°C, followed by 40 cycles of denaturation at 95°C for 15 sec and annealing/extension for 1 min at 60°C. Analysis and quality control were performed using qbase+ software (Biogazelle). Relative expression of the target genes was normalized to the expression of the housekeeping genes *ACTB* and *GAPDH.*

### 2.5. cDNA library preparation and multiplexing

Initially, 1600 - 2000 ng of input RNA per sample was used. The reactions from each sample were first labeled with a barcoded oligo dT nucleotide for multiplexing purposes as shown in Table 10. Subsequently, first-strand cDNA synthesis was performed using the SMARTer PCR cDNA synthesis kit (Takara Bio) according to the manufacturer's instructions. The reactions were then diluted 1:10 in Elution Buffer (PacBio) and large-scale amplification was performed using 16 reactions per sample. Each reaction of 50 µL consisted of 10 µL of the diluted cDNA sample, 10 µL 5X PrimeSTAR GXL buffer (Takara Bio), 4 µL dNTP Mix (2.5 mM each), 1 µL 5' PCR Primer IIA (12 µM), 1 µL PrimeSTAR GXL DNA Polymerase (1.25 U/µL, Takara Bio) and 24 µL nuclease-free water. The samples were then incubated in a thermocyler using the following program: an initial denaturation step at 98°C for 30s, followed by 20 cycles of amplification at 98°C for 10s, 65°C for 15s and 68°C for 10 min, and a final extension step at 68°C for 5 min. From these PCR products, two fractions were purified using AMPure magnetic purification beads. After equimolar pooling of both fractions, two pools of 6 samples were generated by equimolar pooling of the samples based on the individual DNA concentration and fragment length which were evaluated using a Qubit fluorometer (Qubit dsDNA HS kit, ThermoFisher) and an Agilent 2100 Bioanalyzer. Hereafter, samples (pool 1 and pool 2) were ready for cDNA capture.

**Table 10. Barcoded primers used for multiplexing purposes.**

| Patient | Condition | SEQ ID N° | Barcode | Sequence |
|---|---|---|---|---|
| P01 | NON-COVID-19 (mild, ambulatory) | 67 | dT_BC1001_PB | AAGCAGTGGTATCAACGCAGAGTACCACATATCAGAGTGCGTTTTTTTTTTTTTTTTTTTTTTTTTTTTTTVN |
| P02 | COVID-19 (mild, ambulatory) | 68 | dT_BC1002_PB | AAGCAGTGGTATCAACGCAGAGTACACACACAGACTGTGAGTTTTTTTTTTTTTTTTTTTTTTTTTTTTTTTVN |
| P03 | COVID-19 (mild, ambulatory) | 69 | dT_BC1003_PB | AAGCAGTGGTATCAACGCAGAGTACACACATCTCGTGAGAGTTTTTTTTTTTTTTTTTTTTTTTTTTTTTTVN |
| P04 | COVID-19 (severe, ICU) | 70 | dT_BC1004_PB | AAGCAGTGGTATCAACGCAGAGTACCACGCACACACGCGTTTTTTTTTTTTTTTTTTTTTTTTTTTTTTTTTTTVN |
| P05 | COVID-19 (mild, ambulatory) | 71 | dT_BC1005_PB | AAGCAGTGGTATCAACGCAGAGTACCACTCGACTCTCGCGTTTTTTTTTTTTTTTTTTTTTTTTTTTTTTVN |
| P06 | COVID-19 (severe, ICU) | 72 | dT_BC1006_PB | AAGCAGTGGTATCAACGCAGAGTACCATATATATCAGCTGTTTTTTTTTTTTTTTTTTTTTTTTTTTTTTVN |
| P07 | NON-COVID-19 (mild, ambulatory) | 73 | dT_BC1007_PB | AAGCAGTGGTATCAACGCAGAGTACTCTGTATCTCTAGTGTTTTTTTTTTTTTTTTTTTTTTTTTTTTTTTTTVN |
| P08 | COVID-19 (mild, ambulatory) | 74 | dT_BC1008_PB | AAGCAGTGGTATCAACGCAGAGTACACAGTCGAGCGCTGCGTTTTTTTTTTTTTTTTTTTTTTTTTTTTTTTVN |
| P09 | COVID-19 (severe, ICU) | 75 | dT_BC1009_PB | AAGCAGTGGTATCAACGCAGAGTACACACACGCGAGACAGATTTTTTTTTTTTTTTTTTTTTTTTTTTTTTTVN |
| P10 | COVID-19 (severe, ICU) | 76 | dT_BC1010_PB | AAGCAGTGGTATCAACGCAGAGTACACGCGCTCAGAGTTTTTTTTTTTTTTTTTTTTTTTTTTTTTTVN |
| P11 | COVID-19 (mild, ambulatory) | 77 | dT_BC1011_PB | AAGCAGTGGTATCAACGCAGAGTACCTATACGTATATCTATTTTTTTTTTTTTTTTTTTTTTTTTTTTTTTTVN |
| P12 | COVID-19 (severe, ICU) | 78 | dT_BC1012_PB | AAGCAGTGGTATCAACGCAGAGTACACACTAGATCGCGTGTTTTTTTTTTTTTTTTTTTTTTTTTTTTTT |

### 2.6. cDNA capture using SeqCap EZ probes

Initially, 1 µL of SMARTer PCR oligo (1000 µM) and 1 µL PolyT blocker (1000 µM) were added to 1.5µg cDNA of pool 1 and pool 2 and subsequently dried for approximately 40 minutes in a DNA vacuum-concentrator. The cDNA was then hybridized with pre-designed SeqCap EZ probes targeting several mucin coding regions (Table 2 & 3) for 20 hours at 47°C. The captured cDNA was purified using Dynabeads M-270 (Thermo Fisher Scientific) according to the manufacturer's instructions and amplified by preparing a mixture containing 20 µl 10X LA PCR Buffer, 16 µl 2.5 mM dNTP's, 8.3 µL SMARTer PCR Oligos (12 µM each), 1.2 µl Takara LA Taq DNA polymerase and 50 µl cDNA supplemented with nuclease-free water to an end volume of 200 µl. For the actual PCR, the following program was ran on a thermocycler: an initial denaturation step at 95°C for 2 min, followed by 11 cycles of amplification at 95°C for 20s and 68°C for 10 min, and a final extension step at 72°C for 10 min. A final clean-up of the amplified captured cDNA was performed using AMPure purification beads. The DNA concentration and fragment length were evaluated using a Qubit fluorometer (Qubit dsDNA HS kit, ThermoFisher) and an Agilent 2100 Bioanalyzer, after which the samples were equimolary pooled. The resulting cDNA library was then ready for SMRTbell library construction.

### 2.7. SMRTbell library construction and sequencing on the PacBio Sequel system

Using the SMRTbell template prep kit (PacBio), 3 µg of captured cDNA was used for SMRTbell library construction. According to the manufacturer's instructions, the following steps were performed in chronological order: DNA damage repair, end repair, ligation of blunt adapters, Exo III and Exo VII treatment. One intermediate and two final purification steps were performed using AMPure purification beads. The DNA concentration and fragment length were evaluated using a Qubit fluorometer (Qubit dsDNA HS kit, ThermoFisher) and an Agilent 2100 Bioanalyzer for subsequent SMRTbell library construction. Following the instructions on SMRTlink, the Sequel Binding kit (PacBio) and Sequel Sequencing kit (PacBio) were used to dilute the DNA and internal control complexes, anneal the sequencing primer and bind the sequencing polymerase to the SMRTbell templates. Finally, 12 pM of the SMRTbell library was loaded on a 1M v3 SMRT cell.

### 2.8. Data analysis

Statistical analysis using the GraphPad Prism 8.00 software (license DFG170003) was performed to determine significant differences in mucin mRNA expression between COVID-19 patients with severe and mild disease, non-COVID-19 patients with mild disease and healthy controls. Data were analysed by the Analysis of Variance (ANOVA) test and are presented as means ± standard error of mean (SEM). Significance levels are indicated on the graphs and were corrected for multiple testing using the Tukey-Kramer's and Dunn's post-hoc multiple comparisons tests.

A discriminant function analysis was performed to determine COVID-19 severity and positivity based on a set of predictor variables [i.e. the mRNA expression of mucins]. The results are depicted as scatter plots showing the two main discriminant functions [i.e. function 1 (i.e. COVID-19 severity) and function 2 (i.e. COVID-19 positivity)] with the relevant main predictor variables summarized in a table. Furthermore, a multiple linear regression analysis was carried out to investigate associations [1] among mucin mRNA expression of the different patient groups (severe COVID-19, mild COVID-19 and mild non-COVID-19) and [2] between mucin expression and the clinical patient data. Correlation plots display results from Spearman correlation tests as well as a linear regression line with 95% confidence interval. A p-value below 0.05 was considered statistically significant. These analyses were performed using IBM SPSS Statistics 24 software.

Regarding analysis of isoform sequencing data, the raw subreads from single Zero Mode Waveguides (ZMWs) were initially aligned resulting in highly accurate polished circular consensus sequencing (ccs) reads (read accuracy set at 80% and minimum of 1 full pass for the ZMW) which were further processed using the command line interface. The lima tool v1.10.0 was used for demultiplexing and primer removal. Subsequently, the isoseq3 v3.2.2 package was used for further read processing to generate high quality mRNA transcripts. First, the refine tool was used for trimming of Poly(A) tails and identification and removal of concatemers. The data of the individual samples were then pooled together according to the condition (i.e. 2 samples from non-COVID patients with mild symptoms, 5 samples from COVID patients with mild symptoms and 5 samples from patients with severe symptoms) and analyzed in parallel. The isoseq3 cluster algorithm was used for transcript clustering. Minimap2 was used for the alignment of the processed reads to the human reference genome (GRCh38). After mapping, ToFU scripts from the cDNA_Cupcake GitHub repository were used to collapse redundant isoforms (minimal alignment coverage and minimal alignment identity set at 0.90) and identify associated count information. Finally, the SQANTI2 tool was used for extensive characterization of mucin mRNA isoforms. The eventual isoforms were then further inspected by visualization in the Integrative Genomics Viewer (IGV) version 2.8.0 and by the analysis of the classification and junction files in Excel.

### 3. RESULTS

### 3.1. Aberrant mucin mRNA expression associated with COVID-19 positivity and severity

Here, we first investigated mucin mRNA expression levels in the blood of the different patient groups by RT-qPCR. Compared to healthy controls, expression of *MUC13* and *MUC21* mRNA was significantly altered in COVID-19 patients with severe and mild disease, with a higher trend of expression seen in the COVID-19 patient group with mild symptoms. *MUC1* mRNA expression was significantly increased in critical severely ill COVID-19 patients compared to COVID-19 and non-COVID-19 patients with mild symptoms and healthy controls **(****Figure 23****).** On the contrary, mRNA levels of *MUC16* was significantly increased in the ambulatory patients with mild symptoms, irrespective of COVID-19 positivity, and remained low in the severe COVID-19 group **(****Figure 23****),** whereas expression of *MUC2* and *MUC5B* mRNA was significantly increased in all patient groups compared to healthy controls with a higher trend of expression seen in the COVID-19 mild patient group **(****Figure 23****).** MUC4 mRNA expression was only significantly increased in the mild COVID-19 patient group compared the healthy controls **(****Figure 23****).** MUC5AC mRNA expression was not significantly altered, although a decreasing trend in expression was seen in the severe COVID-19 patient group **(****Figure 23****).**

To elucidate which of the aberrantly expressed mucins can predict COVID-19 positivity and severity, the mucin mRNA expression data were used to perform a discriminant analysis. *MUC1* mRNA expression is the major determinant for identifying COVID-19 severity, followed by expression of *MUC13* and *MUC21* mRNA which are the best factors to discriminate between mild COVID-19 and mild non-COVID-19 (**Figure 24A****;** 84% of original grouped cases classified correctly). Interestingly, when *MUC16* mRNA expression was added to the analysis, correct classification of the original grouped cases even increased to 94.4% with *MUC1* and *MUC16* mRNA expression now being the best factors to discriminate for COVID-19 severity **(****Figure 24B****).** *MUC13* and *MUC21* mRNA expression (and to a lesser extend *MUC2, MUC5AC* and *MUC5B* mRNA expression) remained the best factors to discriminate between mild COVID-19 and mild non-COVID-19 **(****Figure 24B****).** Subsequently, a stepwise linear regression analysis on the mucin expression data of the different patient groups (severe COVID-19, mild COVID-19 and mild non-COVID-19), further confirmed *MUC16* (β-coefficient = -0.447; p = 0.025) and *MUC1* (β-coefficient = 0.657; p = 0.003) mRNA expression as major determinants for COVID-19 severity. To further investigate associations between *MUC16* and *MUC1* mRNA expression and the clinical data of the severe COVID-19 patient group, a backward linear regression analysis was performed. Interestingly, increased *MUC1* mRNA expression strongly associated with fungal co-infection (β-coefficient = 0.759; p = 0.001). On its turn, the presence of fungal co-infection associated with mortality (β-coefficient = 0.645; p = 0.009).
Furthermore, we also verified collinearity among the mucin mRNA expression data of the different patient groups (severe COVID-19, mild COVID-19 and mild non-COVID-19) and between mucin mRNA expression and the clinical patient data using Spearman correlation tests. Among the different patient groups, *MUC16 and MUC1 mRNA* expression strongly correlated with COVID-19 severity **(****Figure 25A****)** with a significant negative correlation seen between *MUC16* and *MUC1* mRNA expression **(****Figure 25B****).** While increased *MUC13* mRNA expression was associated with increased *MUC2* and *MUC21* mRNA expression **(****Figure 25C-****E),** *MUC21* mRNA expression also positively correlated with *MUC5B* mRNA expression **(****Figure 25F****)** and *MUC2* mRNA expression strongly correlated with *MUC4* mRNA expression **(****Figure 25G****).**
Finally, the mRNA expression levels of *MUC1* and *MUC13* also significantly correlated with fungal co-infection and the SOFA score, respectively **(****Figure 26****).**

In addition, also other correlations among the clinical variables were found within the severe COVID-19 patient group. These include associations between age and BMI (r = -0.677, p = 0.007), age and diabetes (r = -0.594, p = 0.020), diabetes and renal insufficiency (r = 0.533, p = 0.041), sex and lung disease (r = 0.661, p = 0.007), symptom onset between hospital admission and SOFA score (r = 0.598, p = 0.031), ICU/total hospitalization and duration ventilation (r = 0.985/0.889, p = 0.0001), replacement ETT and ICU hospitalization (r = 0.640, p = 0.010), replacement ETT and duration ventilation (r = 0.639, p = 0.010), replacement ETT and PₐO₂/FᵢO₂ ratio (r = -0.537, p = 0.048), replacement ETT and SOFAₘₐₓ (r = 0.569, p = 0.027), replacement ETT and fungal co-infection (r = 0.600, p = 0.018) and fungal co-infection and PₐO₂/FᵢO₂ ratio (r = -0.595, p = 0.025).

### 3.2. Remdesivir, favipiravir and baricitinib are able to reduce aberrant mucin expression induced by SARS-CoV-2 infection in pulmonary epithelial cells

Subsequently, we investigated the ability of potential COVID-19 treatments to reduce aberrant mucin expression triggered by SARS-CoV-2. *In vitro* stimulation of pulmonary epithelial Calu3 cells with several therapeutic drugs at certain doses, showed that remdesivir and baricitinib were able to significantly reduce *MUC1, MUC4, MUC5AC, MUC5B, MUC13* and *MUC21* mRNA expression **(****Figure 27****).** Also, favipiravir (another antiviral agent) was able to decrease *MUC4, MUC5AC, MUC5B, MUC13* and *MUC21* mRNA expression and to increase *MUC1* mRNA expression **(****Figure 27****).** *MUC1* and *MUC21* mRNA expression were also significantly increased upon dexamethasone treatment whereas toculizumab was able to significantly reduce *MUC1, MUC4* and *MUC21* mRNA expression **(****Figure 27****).**

### 3.3. Mucin mRNA isoforms associated with COVID-19 positivity and severity

### 3.3.1. General features of the sequencing run

Long-read RNA sequencing of all samples initially generated a total of 10.59 x 10⁹ bases after a movie time of 20 hours. Sequencing yield and read quality was high and comparable across all samples. The average read length was 2561 bp. Initially, 77547 ccs reads were generated from the alignment of subreads taken from a single ZMW. 28439 (37 %) reads were lost during primer removal and demultiplexing as a consequence of undesired barcoded primer combinations. After clustering, 24661 reads were remained corresponding to 4939 different transcripts. As visual analysis of targeted mucin regions in IGV showed dense coverage of the genomic regions of *MUC1, MUC2, MUC12, MUC13, MUC16 and MUC17,* further analysis was limited to these mucin glycoproteins.

### 3.3.2. MUC1 mRNA isoforms

Targeted PacBio isoform sequencing revealed the identification of novel *MUC1* mRNA isoforms **(****Figure 28** **& Table 11)** which had not been previously characterized. Interestingly, whereas no mRNA isoforms were identified using PacBio sequencing in the blood from non-COVID19 patients and COVID-19 patients with mild symptoms, 2 mono-exonic alternative transcripts were identified in the blood from COVID19 patients with severe symptoms that resulted from intron retention upstream of exon 11 (which is coding for the intracellular mucin domain (CT)). The results of these limited number of samples show that different alternative transcripts of *MUC1* can be identified in the blood of COVID19 patients which are associated with severe disease.

### 3.3.3. MUC2 mRNA isoforms

Targeted PacBio isoform sequencing revealed the identification of novel *MUC2* mRNA isoforms in the blood from COVID19 and non-COVID19 patients **(****Figure 29** **& Table 11).** In general, 3 alternative mRNA transcripts were found in mRNA isolated from blood which had not been previously characterized. Two alternative transcripts were found in non-COVID19 patients and one was found in COVID-19 patients with mild disease, which were all transcripts coding for the VNTR region of the *MUC2* gene. No alternative transcripts were identified in the blood of COVID-19 patients with severe disease. Nevertheless, targeted PacBio isoform sequencing suggests a distinct expression pattern of *MUC2* mRNA isoforms in the blood of COVID19 and non-COVID19 patients.

### 3.3.4. MUC13 mRNA isoforms

Five smaller alternative MUC13 mRNA transcripts were identified in the blood from COVID19 patients, of which four were found in patients with mild disease and one in patients with severe disease **(****Figure 30** **& Table 11).** No mRNA isoforms could be characterized in the blood from non-COVID19 patients with the targeted PacBio isoform sequencing approach **(****Figure 30** **& Table 11).** All mRNA isoforms found had not been identified previously and mainly resulted from intron retention. Of these expressed in the blood from patients with mild symptoms, two transcripts were coding for EGF-like domains (PB.3.2 and PB.3.4) and one transcript for the TM domain (PB.3.1).

### 3.3.5. MUC16 mRNA isoforms

Targeted PacBio isoform sequencing revealed the identification of many small novel *MUC16* mRNA isoforms in the blood from COVID19 and non-COVID19 **(****Figure 31** **& Table 11).** In general, 20 alternative mRNA transcripts (= isoforms) were found in mRNA isolated from blood which had not been previously characterized. All novel isoforms mapped to the extracellular domain of the MUC16 gene. One multi-exonic mRNA isoform was identified in non-COVID19 patients (PB.4.1), which was also found in COVID19 patients. In addition, several other small MUC16 mRNA isoforms were found, of which 14 were identified in patients with mild disease and five in patients with severe COVID-19. Hence, the expression of MUC16 mRNA isoforms was associated with disease severity in COVID19 patients. Most mRNA isoforms were mono-exonic and resulted from intron retention. A detailed overview of all alternative transcripts can be found in **Table 11.**

**Table 11: Detailed overview of characteristics of mucin mRNA isoforms in blood samples from COVID-19 patients and non-COVID19 controls.**

| **Ambulatory non-COVID19 patients with mild common cold symptoms** | | | | | | |
|---|---|---|---|---|---|---|
| Isoform ID | Chrom | Gene | Length (bp) | Exons | Transcript | Subcategory |
| PB.2.1 | chr11 | MUC2 | 870 | 1 | Novel | Mono-exon |
| PB.2.2 | chr11 | MUC2 | 1765 | 11 | Novel | Intron retention |
| PB.4.1 | chr19 | MUC16 | 2263 | 2 | Novel | Multi-exon |

| **ambulatory COVID-19 patients with mild symptoms** | | | | | | |
|---|---|---|---|---|---|---|
| Isoform ID | I Chrom | I Gene | Length (bp) | I Exons | Transcript | I Subcategory |
| PB.2.3 | chr11 | MUC2 | 271 | 1 | Novel | Mono-exon |
| PB.3.1 | chr3 | MUC13 | 2538 | 2 | Novel | Intron retention |
| PB.3.2 | chr3 | MUC13 | 1442 | 1 | Novel | Intron retention |
| PB.3.3 | chr3 | MUC13 | 712 | 1 | Novel | Intron retention |
| PB.3.4 | chr3 | MUC13 | 2538 | 2 | Novel | Intron retention |
| PB.4.1 | chr19 | MUC16 | 2263 | 2 | Novel | Multi-exon |
| PB.4.2 | chr19 | MUC16 | 2540 | 2 | Novel | Multi-exon |
| PB.4.3 | chr19 | MUC16 | 3017 | 1 | Novel | Intron retention |
| PB.4.4 | chr19 | MUC16 | 1807 | 1 | Novel | Intron retention |
| PB.4.5 | chr19 | MUC16 | 1808 | 1 | Novel | Intron retention |
| PB.4.6 | chr19 | MUC16 | 207 | 1 | Novel | Intron retention |
| PB.4.7 | chr19 | MUC16 | 2795 | 1 | Novel | Intron retention |
| PB.4.8 | chr19 | MUC16 | 984 | 1 | Novel | Intron retention |
| PB.4.9 | chr19 | MUC16 | 1438 | 1 | Novel | Intron retention |
| PB.4.10 | chr19 | MUC16 | 2239 | 1 | Novel | Intron retention |
| PB.4.11 | chr19 | MUC16 | 2364 | 1 | Novel | Intron retention |
| PB.4.12 | chr19 | MUC16 | 2309 | 1 | Novel | Intron retention |
| PB.4.13 | chr19 | MUC16 | 2241 | 1 | Novel | Intron retention |
| PB.4.14 | chr19 | MUC16 | 2223 | 1 | Novel | Intron retention |
| PB.4.15 | chr19 | MUC16 | 2200 | 1 | Novel | Intron retention |

| **COVID19 patients with severe symptoms and hospitalized at the ICU** | | | | | | |
|---|---|---|---|---|---|---|
| Isoform ID | Chrom | Gene | Length (bp) | Exons | Transcript | Subcategory |
| PB.1.1 | chr1 | MUC1 | 694 | 1 | Novel | Intron retention |
| PB.1.2 | chr1 | MUC1 | 1269 | 1 | Novel | Intron retention |
| PB.3.5 | chr3 | MUC13 | 494 | 1 | Novel | Intron retention |
| PB.4.1 | chr19 | MUC16 | 2266 | 2 | Novel | Multi-exon |
| PB.4.16 | chr19 | MUC16 | 2546 | 6 | Novel | Multi-exon |
| PB.4.17 | chr19 | MUC16 | 1488 | 1 | Novel | Intron retention |
| PB.4.18 | chr19 | MUC16 | 2224 | 1 | Novel | Intron retention |
| PB.4.19 | chr19 | MUC16 | 1457 | 1 | Novel | Intron retention |
| PB.4.20 | chr19 | MUC16 | 2155 | 1 | Novel | Intron retention |

### 4. CONCLUDING REMARKS

Based on the mucin mRNA expression data measured in the blood of COVID-19 patients with a varying degree of disease severity, non-COVID-19 patients with common cold like symptoms and healthy controls, a specific mucin signature for COVID-19 could be identified with a central role for **MUC1 and MUC16 mRNA expression in predicting disease severity** and **MUC13 and MUC21 mRNA expression in predicting COVID-19 positivity.** Furthermore, several COVID-19 treatments, such as baricitinib, favipiravir and remdesivir, which have shown promising results in clinical trials, were able to suppress mucin hypersecretion and more specifically the mucins defining the **mucin mRNA signature for COVID-19 disease severity and positivity, i.e. MUC13, MUC21, MUC16 and MUC1.** This highlights the potential of these mucins in disease surveillance as well.

Subsequently, based on the PacBio isoform sequencing data gathered from a limited number of blood samples, we were able to identify **unique and novel MUC1 mRNA isoforms associated with severe COVID-19** and **unique and novel MUC2 mRNA isoforms, MUC13 mRNA isoforms and MUC16 mRNA isoforms associated with mild COVID-19 and COVID-19 positivity.**

In conclusion, altered mRNA expression of MUC1, MUC2, MUC5AC, MUC5B, MUC13, MUC16 and MUC21 mucins as well as alternative mRNA isoforms of MUC1, MUC2, MUC13 and MUC16 could be associated with COVID-19 severity and positivity, highlighting their potential for COVID-19 diagnosis, prognosis, disease surveillance and treatment.

### REFERENCES

Breugelmans T, Van Spaendonk H, De Man JG, De Schepper HU, Jauregui-Amezaga A, Macken E, Linden SK, Pintelon I, Timmermans JP, De Winter BY, Smet A. In depth study of transmembrane mucins in association with intestinal barrier dysfunction during the course of T cell transfer and DSS-induced colitis. J Crohns Colitis 2020, jjaa015.
Corman VM, Landt O, Kaiser M, Molenkamp R, Meijer A, Chu DK, Bleicker T, Brünink S, Schneider J, Schmidt ML, Mulders DG, Haagmans BL, van der Veer B, van den Brink S, Wijsman L, Goderski G, Romette JL, Ellis J, Zambon M, Peiris M, Goossens H, Reusken C, Koopmans MP, Drosten C. Detection of 2019 novel coronavirus (2019-nCoV) by real-time RT-PCR. Euro Surveill 2020, 25(3):2000045.
d'Allessandro M, et al., 2020. Serum KL-6 concentrations as a novel biomarker of severe COVID-19. Journal of Medical Virology, 92:2216-2220.
Guan WJ, Chen RC, Zhong NS. Strategies for the prevention and management of coronavirus disease 2019. Eur Respir J. 2020, 55(4):2000597.
Gupta J, Nebreda A. Analysis of Intestinal Permeability in Mice. BIO-PROTOCOL 2014; 4(22).
Heylen M, Deleye S, De Man JG, et al. Colonoscopy and µPET/CT are Valid Techniques to Monitor Inflammation in the Adoptive Transfer Colitis Model in Mice. Inflamm Bowel Dis 2013; 19(5): 967-76.
Hoffmann et al., 2020. SARS-CoV-2 cell entry depends on ACE2 and TMPRSS2 and is blocked by a clinically proven protease inhibitor. Cell, 181:271-280.
Huang C, Yeming W, Li X, Ren L et al., 2020. Clinical features of patients infected with 2019 novel coronavirus in Wuhan, China. The Lancet, 395:497-506.
Linden S, Putton P, Karlsson N, et al. Mucins in the mucosal barrier to infection. Mucosal Immunol 2008; 1(3): 183-97.
Moehle C, Ackermann N, Langmann T, et al. Aberrant intestinal expression and allelic variants of mucin genes associated with inflammatory bowel disease. J Mol Med 2006; 84(12): 1055-66.
Moreels TG, Nieuwendijk RJ, De Man JG, et al. Concurrent infection with Schistosoma mansoni attenuates inflammation induced changes in colonic morphology, cytokine levels, and smooth muscle contractility of trinitrobenzene sulphonic acid induced colitis in rats. Gut 2004; 53(1): 99-107.
ObermairA, Schmid BC, Packer LM, Leodolter S, Birner P, Ward BG, Crandon AJ, McGuckin MA, Zeillinger R. Expression of MUC1 splice variants in benign and malignant ovarian tumours. Int J Cancer 2002; 100: 166-71.
Sheng YH, Lourie R, Linden SK, et al. The MUC13 cell-surface mucin protects against intestinal inflammation by inhibiting epithelial cell apoptosis. Gut 2011; 60(12): 1661-70.
Sheng Y, Triyana S, Wang R, et al. MUC1 and MUC13 differentially regulate epithelial inflammation in response to inflammatory and infectious stimuli. Mucosal Immunol 2012; 6(3): 557-68.
Vancamelbeke M, Vanuytsel T, Farré R, et al. Genetic and Transcriptomic Bases of Intestinal Epithelial Barrier Dysfunction in Inflammatory Bowel Disease. Inflamm Bowel Dis 2017; 23(10): 1718-29.
Wallace JL, Keenan CM, Gale D, Shoupe TS. Exacerbation of experimental colitis by nonsteroidal anti-inflammatory drugs is not related to elevated leukotriene B4synthesis. Gastroenterology 1992; 102(1): 18-27.
Wenju et al., 2020. Elevated MUC1 and MUC5AC mucin protein levels in airway mucus of critical ill COVID-19 patients. J Med Virol 93:582-584.
Zaretsky JZ, Barnea I, Aylon Y, Gorivodsky M, Wreschner DH, Keydar I. MUC1 gene overexpressed in breast cancer: Structure and transcriptional activity of the MUC1 promoter and role of estrogen receptor alpha (ERα) in regulation of the MUC1 gene expression. Mol Cancer 2006; 5: 57.
Zrihan-Licht S, Vos HL, Baruch A, Elroy-Stein O, Sagiv D, Keydar I, Hilkens J, Wreschner DH. Characterization and Molecular Cloning of a Novel MUC1 Protein, Devoid of Tandem Repeats, Expressed in Human Breast Cancer Tissue. Eur J Biochem 1994; 224: 787-95.

SEQUENCE LISTING

<110> Universiteit Antwerpen

<120> MUCIN ISOFORMS IN DISEASES CHARACTERIZED BY BARRIER DYSFUNCTION

<130> UNA-046

<160> 78

<170> BiSSAP 1.3.6

<210> 1
<211> 21
<212> DNA
<213> Artificial Sequence

<220>
<223> Cdh1 FW primer

<400> 1
cagttccgag gtctacacct t                                                21

<210> 2
<211> 22
<212> DNA
<213> Artificial Sequence

<220>
<223> Cdh1 REV primer

<400> 2
tgaatcggga gtcttccgaa aa                                               22

<210> 3
<211> 21
<212> DNA
<213> Artificial Sequence

<220>
<223> Cldn1 FW primer

<400> 3
tgccccagtg gaagatttac t                                                21

<210> 4
<211> 20
<212> DNA
<213> Artificial Sequence

<220>

<223> Cldn1 REV primer

<400> 4
ctttgcgaaa cgcaggacat                                                    20


<210> 5
<211> 21
<212> DNA
<213> Artificial Sequence


<220>
<223> Cldn2 FW primer

<400> 5
caactggtgg gctacatcct a                                                  21


<210> 6
<211> 19
<212> DNA
<213> Artificial Sequence


<220>
<223> Cldn2 REV primer

<400> 6
cccttggaaa agccaaccc                                                     19


<210> 7
<211> 21
<212> DNA
<213> Artificial Sequence


<220>
<223> Cldn3 FW primer

<400> 7
accaactgcg tacaagacga g                                                  21


<210> 8
<211> 20
<212> DNA
<213> Artificial Sequence


<220>
<223> Cldn3 REV primer

<400> 8
cgggcaccaa cgggttatag                                                    20

<210> 9
<211> 19
<212> DNA
<213> Artificial Sequence

<220>
<223> Cldn5 FW primer

<400> 9
gcaaggtgta tgaatctgt                                                    19

<210> 10
<211> 22
<212> DNA
<213> Artificial Sequence

<220>
<223> Cldn5 REV primer

<400> 10
gtcaaggtaa caaagagtgc ca                                                22

<210> 11
<211> 19
<212> DNA
<213> Artificial Sequence

<220>
<223> Cldn7 FW primer

<400> 11
ggcctgatag cgagcactg                                                    19

<210> 12
<211> 21
<212> DNA
<213> Artificial Sequence

<220>
<223> Cldn7 REV primer

<400> 12
tggcgacaaa catggctaag a                                                 21

<210> 13
<211> 21
<212> DNA
<213> Artificial Sequence

<220>
<223> Cldn15 FW primer

<400> 13
attgcaggga ccctccacat a                                                21


<210> 14
<211> 21
<212> DNA
<213> Artificial Sequence


<220>
<223> Cldn15 REV primer

<400> 14
gcccagttca tacttggttc c                                                21


<210> 15
<211> 21
<212> DNA
<213> Artificial Sequence


<220>
<223> Crb3 FW primer

<400> 15
caccggaccc tttcacaaat a                                                21


<210> 16
<211> 21
<212> DNA
<213> Artificial Sequence


<220>
<223> Crb3 REV primer

<400> 16
cccactgcta taaggaggac t                                                21


<210> 17
<211> 21
<212> DNA
<213> Artificial Sequence


<220>
<223> Dlg1 FW primer

<400> 17
agtgacgaag tcggagtgat t                                                21

<210> 18
<211> 22
<212> DNA
<213> Artificial Sequence

<220>
<223> Dlg1 REV primer

<400> 18
gtcagggatc tcccctttat ct                                                22

<210> 19
<211> 23
<212> DNA
<213> Artificial Sequence

<220>
<223> Jam1 FW primer

<400> 19
tctcttcacg tctatgatcc tgg                                               23

<210> 20
<211> 21
<212> DNA
<213> Artificial Sequence

<220>
<223> Jam1 REV primer

<400> 20
tttgatggac tcgttctggg g                                                 21

<210> 21
<211> 22
<212> DNA
<213> Artificial Sequence

<220>
<223> Jam2 FW primer

<400> 21
gtgcccactt ctgttatgac tg                                                22

<210> 22
<211> 21
<212> DNA
<213> Artificial Sequence

<220>
<223> Jam2 REV primer

<400> 22
ttccctagca aacttgtgcc a                                                21


<210> 23
<211> 19
<212> DNA
<213> Artificial Sequence


<220>
<223> Jam3 FW primer

<400> 23
ctgcgacttc gactgtacg                                                   19


<210> 24
<211> 22
<212> DNA
<213> Artificial Sequence


<220>
<223> Jam3 REV primer

<400> 24
ttcggttgct ggatttgaga tt                                               22


<210> 25
<211> 19
<212> DNA
<213> Artificial Sequence


<220>
<223> Llgl1 FW primer

<400> 25
gcttccccaa tcagcccag                                                   19


<210> 26
<211> 21
<212> DNA
<213> Artificial Sequence


<220>
<223> Llgl1 REV primer

<400> 26
gcgcagccat tatgatggat g                                                21

<210> 27
<211> 24
<212> DNA
<213> Artificial Sequence

<220>
<223> Muc1 FW primer

<400> 27
ggttgctttg gctatcgtct attt                                              24

<210> 28
<211> 21
<212> DNA
<213> Artificial Sequence

<220>
<223> Muc1 REV primer

<400> 28
aaagatgtcc agctgcccat a                                                 21

<210> 29
<211> 20
<212> DNA
<213> Artificial Sequence

<220>
<223> Muc2 FW primer

<400> 29
atgcccacct cctcaaagac                                                   20

<210> 30
<211> 23
<212> DNA
<213> Artificial Sequence

<220>
<223> Muc2 REV primer

<400> 30
gtagtttccg ttggaacagt gaa                                               23

<210> 31
<211> 22
<212> DNA
<213> Artificial Sequence

<220>
<223> Muc4 FW primer

<400> 31
acaggtgtaa ctagaagcct cg                                                22


<210> 32
<211> 21
<212> DNA
<213> Artificial Sequence


<220>
<223> Muc4 REV primer

<400> 32
caggggtgct atgcactact g                                                 21


<210> 33
<211> 21
<212> DNA
<213> Artificial Sequence


<220>
<223> Muc13 FW primer

<400> 33
gccagtcctc ccaccacggt a                                                 21


<210> 34
<211> 21
<212> DNA
<213> Artificial Sequence


<220>
<223> Muc13 REV primer

<400> 34
ctgggacctg tgcttccacc g                                                 21


<210> 35
<211> 21
<212> DNA
<213> Artificial Sequence


<220>
<223> Mylk FW primer

<400> 35

tgggggacgt gaaactgttt g                                                21

<210> 36
<211> 19
<212> DNA
<213> Artificial Sequence

<220>
<223> Mylk REV primer

<400> 36
ggggcagaat gaaagctgg                                                   19

<210> 37
<211> 22
<212> DNA
<213> Artificial Sequence

<220>
<223> Ocln FW primer

<400> 37
ggcggatata cagacccaag ag                                               22

<210> 38
<211> 24
<212> DNA
<213> Artificial Sequence

<220>
<223> Ocln REV primer

<400> 38
gataatcatg aaccccagga caat                                             24

<210> 39
<211> 20
<212> DNA
<213> Artificial Sequence

<220>
<223> Pals1 FW primer

<400> 39
tttgggcacc agaatgatgc                                                  20

<210> 40
<211> 23
<212> DNA

<213> Artificial Sequence

<220>
<223> Pals1 REV primer

<400> 40
aacaattcct tcttccgtgt caa                                               23

<210> 41
<211> 21
<212> DNA
<213> Artificial Sequence

<220>
<223> Par3 FW primer

<400> 41
ggagatggcc gcatgaaagt t                                                 21

<210> 42
<211> 21
<212> DNA
<213> Artificial Sequence

<220>
<223> Par3 REV primer

<400> 42
ctccaagcga tgcacctgta t                                                 21

<210> 43
<211> 20
<212> DNA
<213> Artificial Sequence

<220>
<223> Par6 FW primer

<400> 43
tcagaaacgg gcagaaggtg                                                   20

<210> 44
<211> 20
<212> DNA
<213> Artificial Sequence

<220>
<223> Par6 REV primer

<400> 44
ccaggcggga gatgaagata                                                    20

<210> 45
<211> 21
<212> DNA
<213> Artificial Sequence

<220>
<223> Patj FW primer

<400> 45
ttcgatgggc accactatat c                                                  21

<210> 46
<211> 21
<212> DNA
<213> Artificial Sequence

<220>
<223> Patj REV primer

<400> 46
ggtgggggca cttctttaag g                                                  21

<210> 47
<211> 21
<212> DNA
<213> Artificial Sequence

<220>
<223> aPkcλ FW primer

<400> 47
cactttgagc cttccatctc c                                                  21

<210> 48
<211> 19
<212> DNA
<213> Artificial Sequence

<220>
<223> aPkcλ REV primer

<400> 48
gtgaccagct tgtggcact                                                     19

<210> 49
<211> 21

<212> DNA
<213> Artificial Sequence

<220>
<223> aPkcς FW primer

<400> 49
gcgtggatgc catgacaaca t                                                21

<210> 50
<211> 21
<212> DNA
<213> Artificial Sequence

<220>
<223> aPkcς REV primer

<400> 50
ggctcttggg aaggcatgac a                                                21

<210> 51
<211> 21
<212> DNA
<213> Artificial Sequence

<220>
<223> Rpl4 FW primer

<400> 51
ccgtcccctc atatcggtgt a                                                21

<210> 52
<211> 23
<212> DNA
<213> Artificial Sequence

<220>
<223> Rpl4 REV primer

<400> 52
gcatagggct gtctgttgtt ttt                                              23

<210> 53
<211> 20
<212> DNA
<213> Artificial Sequence

<220>
<223> Scrib FW primer

<400> 53
cctgggcatc agtatcgcag                                                    20

<210> 54
<211> 20
<212> DNA
<213> Artificial Sequence

<220>
<223> Scrib REV primer

<400> 54
gccctcgtca tctcctttgt                                                    20

<210> 55
<211> 21
<212> DNA
<213> Artificial Sequence

<220>
<223> Tjp1 FW primer

<400> 55
gagcgggcta ccttactgaa c                                                  21

<210> 56
<211> 23
<212> DNA
<213> Artificial Sequence

<220>
<223> Tjp1 REV primer

<400> 56
gtcatctctt tccgaggcat tag                                                23

<210> 57
<211> 20
<212> DNA
<213> Artificial Sequence

<220>
<223> Tjp2 FW primer

<400> 57
atgggagcag tacaccgtga                                                    20

<210> 58

<211> 22
<212> DNA
<213> Artificial Sequence

<220>
<223> Tjp2 REV primer

<400> 58
tgaccaccct gtcattttct tg                                                22

<210> 59
<211> 21
<212> DNA
<213> Artificial Sequence

<220>
<223> Tjp3 FW primer

<400> 59
ctgtggagaa cgtcacatct g                                                 21

<210> 60
<211> 20
<212> DNA
<213> Artificial Sequence

<220>
<223> Tjp3 REV primer

<400> 60
cggggacgct tcactgtaac                                                   20

<210> 61
<211> 73
<212> DNA
<213> Artificial Sequence

<220>
<223> dt_BC1001_PB

<220>
<221> unsure
<222> 73
<223> /note="v is A or C or G"
      /note="n is any nucleotide"

<400> 61
aagcagtggt atcaacgcag agtaccacat atcagagtgc gttttttttt tttttttttt      60

tttttttttt tvn                                                          73

<210> 62
<211> 73
<212> DNA
<213> Artificial Sequence

<220>
<223> dt_BC1002_PB

<220>
<221> unsure
<222> 73
<223> /note="v is A or C or G"
      /note="n is any nucleotide"

<400> 62
aagcagtggt atcaacgcag agtacacaca cagactgtga gttttttttt tttttttttt      60

tttttttttt tvn                                                          73

<210> 63
<211> 73
<212> DNA
<213> Artificial Sequence

<220>
<223> dt_BC1003_PB

<220>
<221> unsure
<222> 73
<223> /note="v is A or C or G"
      /note="n is any nucleotide"

<400> 63
aagcagtggt atcaacgcag agtacacaca tctcgtgaga gttttttttt tttttttttt      60

tttttttttt tvn                                                          73

<210> 64
<211> 73
<212> DNA
<213> Artificial Sequence

<220>
<223> dt_BC1004_PB

<220>
<221> unsure
<222> 73
<223> /note="v is A or C or G"
      /note="n is any nucleotide"

<400> 64
aagcagtggt atcaacgcag agtaccacgc acacacgcgc gttttttttt tttttttttt     60

tttttttttt tvn                                                        73



<210> 65
<211> 73
<212> DNA
<213> Artificial Sequence



<220>
<223> dt_BC1005_PB

<220>
<221> unsure
<222> 73
<223> /note="v is A or C or G"
      /note="n is any nucleotide"

<400> 65
aagcagtggt atcaacgcag agtaccactc gactctcgcg tttttttttt tttttttttt     60

tttttttttt tvn                                                        73



<210> 66
<211> 73
<212> DNA
<213> Artificial Sequence



<220>
<223> dt_BC1006_PB

<220>
<221> unsure
<222> 73
<223> /note="v is A or C or G"
      /note="n is any nucleotide"

<400> 66
aagcagtggt atcaacgcag agtaccatat atatcagctg tttttttttt tttttttttt     60

tttttttttt tvn                                                        73



<210> 67
<211> 73
<212> DNA
<213> Artificial Sequence



<220>
<223> dT_BC1001_PB

<220>

<221> unsure
<222> 73
<223> /note="n is any nucleotide"
      /note="v is A or C or G"

<400> 67
aagcagtggt atcaacgcag agtaccacat atcagagtgc gttttttttt tttttttttt      60

tttttttttt tvn                                                          73


<210> 68
<211> 73
<212> DNA
<213> Artificial Sequence


<220>
<223> dT_BC1002_PB

<220>
<221> unsure
<222> 73
<223> /note="n is any nucleotide"
      /note="v is A or C or G"

<400> 68
aagcagtggt atcaacgcag agtacacaca cagactgtga gttttttttt tttttttttt      60

tttttttttt tvn                                                          73


<210> 69
<211> 73
<212> DNA
<213> Artificial Sequence


<220>
<223> dt_BC1003_PB

<220>
<221> unsure
<222> 73
<223> /note="n is any nucleotide"
      /note="v is A or C or G"

<400> 69
aagcagtggt atcaacgcag agtacacaca tctcgtgaga gttttttttt tttttttttt      60

tttttttttt tvn                                                          73


<210> 70
<211> 73
<212> DNA
<213> Artificial Sequence

<220>
<223> dT-BC1004_PB

<220>
<221> unsure
<222> 73
<223> /note="n is any nucleotide"
      /note="v is A or C or G"

<400> 70
aagcagtggt atcaacgcag agtaccacgc acacacgcgc gttttttttt tttttttttt      60

tttttttttt tvn                                                          73


<210> 71
<211> 73
<212> DNA
<213> Artificial Sequence


<220>
<223> dT_BC1005_PB

<220>
<221> unsure
<222> 73
<223> /note="n is any nucleotide"
      /note="v is A or G or C"

<400> 71
aagcagtggt atcaacgcag agtaccactc gactctcgcg tttttttttt tttttttttt      60

tttttttttt tvn                                                          73


<210> 72
<211> 73
<212> DNA
<213> Artificial Sequence


<220>
<223> dT_BC1006_PB

<220>
<221> unsure
<222> 73
<223> /note="n is any nucleotide"
      /note="v is A or G or C"

<400> 72
aagcagtggt atcaacgcag agtaccatat atatcagctg tttttttttt tttttttttt      60

tttttttttt tvn                                                          73

<210> 73
<211> 74
<212> DNA
<213> Artificial Sequence

<220>
<223> dT_BC1007_PB

<220>
<221> unsure
<222> 74
<223> /note="n is any nucleotide"
      /note="v is A or G or C"

<400> 73
aagcagtggt atcaacgcag agtactctgt atctctatgt gttttttttt tttttttttt     60

tttttttttt ttvn                                                       74

<210> 74
<211> 74
<212> DNA
<213> Artificial Sequence

<220>
<223> dT_BC1008_PB

<220>
<221> unsure
<222> 74
<223> /note="n is any nucleotide"
      /note="v is A or C or G"

<400> 74
aagcagtggt atcaacgcag agtacacagt cgagcgctgc gttttttttt tttttttttt     60

tttttttttt ttvn                                                       74

<210> 75
<211> 74
<212> DNA
<213> Artificial Sequence

<220>
<223> dT_BC1009_PB

<220>
<221> unsure
<222> 74
<223> /note="n is any nucleotide"
      /note="v is A or C or G"

<220>

<221> unsure
<222> 74
<223> /note="n is any nucleotide"
      /note="v is A or C or G"

<400> 75
aagcagtggt atcaacgcag agtacacaca cgcgagacag attttttttt tttttttttt     60

tttttttttt ttvn                                                       74


<210> 76
<211> 74
<212> DNA
<213> Artificial Sequence


<220>
<223> dT_BC1010_PB

<220>
<221> unsure
<222> 74
<223> /note="n is any nucleotide"
      /note="v is A or G or C"

<400> 76
aagcagtggt atcaacgcag agtacacgcg ctatctcaga gttttttttt tttttttttt     60

tttttttttt ttvn                                                       74


<210> 77
<211> 73
<212> DNA
<213> Artificial Sequence


<220>
<223> dT_BC1011_PB

<220>
<221> unsure
<222> 73
<223> /note="n is any nucleotide"
      /note="v is A or G or C"

<400> 77
aagcagtggt atcaacgcag agtacctata cgtatatcta tttttttttt tttttttttt     60

tttttttttt tvn                                                        73


<210> 78
<211> 73
<212> DNA
<213> Artificial Sequence

<220>
<223> dT_BC1012_PB

<220>
<221> unsure
<222> 73
<223> /note="n is any nucleotide"
      /note="v is A or G or C"

<400> 78
aagcagtggt atcaacgcag agtacacact agatcgcgtg tttttttttt tttttttttt      60

tttttttttt tvn                                                         73

## Claims

1. An in vitro method for diagnosing and/or determining the severity of a coronaviral infection or coronaviral infectious disease and/or associated co-infections, said method comprising:
a) providing a biological sample from a subject suspected of having a coronaviral infection or coronaviral infectious disease and/or associated co-infections, and
b) determining the presence and/or quantity of one or more mucins selected from the list comprising: MUC16, MUC21, MUC2, MUC4, MUC5AC, MUC5B, MUC13, optionally in combination with MUC1;
wherein the presence and/or quantity of the one or more mucins is indicative for the presence and/or severity of a coronaviral infection or coronaviral infectious disease.

2. The method according to claim 1; wherein said method comprises determining the presence and/or quantity of MUC13 and MUC21; and wherein high levels of MUC13 and MUC21 are indicative of a coronaviral infection or coronaviral infectious disease.

3. The method according to claim 1; wherein said method comprises determining the presence and/or quantity of MUC1 and MUC16; and wherein high levels of MUC1 and low levels of MUC16 are indicative of a more severe coronaviral infection or coronaviral infectious disease.

4. An in vitro method for diagnosing and/or determining the severity of a coronaviral infection or coronaviral infectious disease and/or associated co-infections, said method comprising:
a) providing a biological sample from a subject suspected of having a coronaviral infection or coronaviral infectious disease and/or associated co-infections, and
b) determining the presence and/or quantity of one or more of mucin isoforms; selected from the group comprising MUC16 mRNA isoforms, MUC 21 mRNA isoforms, MUC2 mRNA isoforms, MUC4 mRNA isoforms, MUC5AC mRNA isoforms, MUC5B mRNA isoforms, MUC13 mRNA isoforms, or MUC1 mRNA isoforms, ;
wherein the presence and/or quantity of the one or more mucin mRNA isoforms is indicative for the presence and/or severity of a coronaviral infection or coronaviral infectious disease.

5. The in vitro method of claim 4, wherein the one or more mucin mRNA isoforms are selected from the group comprising MUC1 mRNA isoforms, MUC13 mRNA isoforms, MUC16 mRNA isoforms, or MUC21 mRNA isoforms.

6. The in vitro method of claim 4 or 5, wherein at least 2; preferably at least 3 mucin mRNA isoforms are determined and/or quantified.

7. The in vitro method of any one of claims 4-6 wherein the presence and/or quantity of MUC1 mRNA isoforms, MUC13 mRNA isoforms, MUC16 mRNA isoforms and MUC 21 mRNA isoforms are determined and wherein the presence and/or quantity of said mucin mRNA isoforms is indicative for the presence and/or severity of a coronaviral infection or coronaviral infectious disease.

8. The in vitro method of any of one of claims 4-7, wherein the presence and/or quantity of MUC13 mRNA isoforms and/or MUC21 mRNA isoforms is determined and wherein the presence and/or quantity of MUC13 mRNA isoforms and/or MUC21 mRNA isoforms is indicative for the diagnosis of a coronaviral infection or coronaviral infectious disease.

9. The in vitro method of claim 1 or 2, wherein the presence and/or quantity of MUC1 mRNA isoforms and/or MUC16 mRNA isoforms is determined and wherein the presence and/or quantity of MUC1 mRNA isoforms and/or MUC16 mRNA isoforms is indicative for the severity of the coronaviral infection or coronaviral infectious disease.

10. The method according to anyone of claims 1 to 9, wherein said mucin mRNA isoform is a transmembrane mucin.

11. A combination of MUC1 mRNA isoforms, MUC13 mRNA isoforms, MUC16 mRNA isoforms and MUC21 mRNA isoforms; optionally in combination with one or more of MUC2 mRNA isoforms, MUC4 mRNA isoforms, MUC5AC mRNA isoforms or MUC5B mRNA isoforms for use in the diagnosis and/or monitoring of a coronaviral infection or coronaviral infectious disease.

12. A combination of MUC1 mRNA isoforms and MUC16 mRNA isoforms for use in determining the severity of a coronaviral infection or coronaviral infectious disease.

13. A combination of MUC13 mRNA isoforms and MUC21 mRNA isoforms for use in the diagnosis of a coronaviral infection or coronaviral disease.

14. The in vitro method of any one of claims 1 to 10, or the combination of mucin mRNA isoforms for use according to claim 11 to 13, wherein the coronaviral infection or coronaviral disease is SARS-CoV-2 infection or SARS-CoV-2 associated disease.

15. A diagnostic kit for performing the in vitro method according to any one of the claims 1 to 10, said kit comprising agents for detecting the presence of the one or more mucins and/or mucin mRNA isoforms as defined in anyone of claims 1 to 10.
